(19) Europäisches Patentamt European Patent Office Office européen des brevets

(11) **EP 3 765 489 B1**

(12) **EUROPEAN PATENT SPECIFICATION**

(45) Date of publication and mention of the grant of the patent:
**16.10.2024 Bulletin 2024/42**

(21) Application number: **19709056.6**

(22) Date of filing: **11.03.2019**

(51) International Patent Classification (IPC):
**C07K 14/705** $^{(2006.01)}$    **C07K 16/28** $^{(2006.01)}$
**A61P 35/00** $^{(2006.01)}$

(52) Cooperative Patent Classification (CPC):
**C07K 16/2887; A61P 35/00; C07K 14/70503;**
**C07K 16/2803;** A61K 2039/507; C07K 2317/515;
C07K 2317/52; C07K 2317/565; C07K 2317/70;
C07K 2319/33

(86) International application number:
**PCT/EP2019/055946**

(87) International publication number:
**WO 2019/175071 (19.09.2019 Gazette 2019/38)**

(54) **THERAPEUTIC COMBINATION  OF 4-1BB AGONISTS WITH ANTI-CD20 ANTIBODIES**

THERAPEUTISCHE KOMBINATION VON 4-1BB AGONISTEN UND ANTI-CD20 ANTIKÖRPER

COMBINATION THERAPEUTIQUE D'AGONISTES DE 4-1BB ET D'ANTICORPS CONTRE CD20

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **13.03.2018 EP 18161435**

(43) Date of publication of application:
**20.01.2021 Bulletin 2021/03**

(73) Proprietor: **F. Hoffmann-La Roche AG**
**4070 Basel (CH)**

(72) Inventors:
• **FERRARA KOLLER, Claudia**
**8952 Schlieren (CH)**
• **KLEIN, Christian**
**8952 Schlieren (CH)**
• **SAM, Johannes**
**8952 Schlieren (CH)**
• **UMANA, Pablo**
**8952 Schlieren (CH)**
• **XU, Wei**
**8952 Schlieren (CH)**

(74) Representative: **Klostermeyer-Rauber, Dörte**
**F. Hoffmann-La Roche AG**
**Corporate Law Patents (CLP)**
**Grenzacherstrasse 124**
**4070 Basel (CH)**

(56) References cited:
**WO-A1-2016/075278    WO-A1-2018/114748**

• **MARCUS ROBERT ET AL: "Obinutuzumab for the First-Line Treatment of Follicular Lymphoma", THE NEW ENGLAND JOURNAL OF MEDICINE, vol. 377, no. 14, 5 October 2017 (2017-10-05), US, pages 1331 - 1344, XP055884446, ISSN: 0028-4793, DOI: 10.1056/NEJMoa1614598**
• **KARMALI R. ET AL: "Rituximab: a benchmark in the development of chemotherapy-free treatment strategies for follicular lymphomas", ANNALS OF ONCOLOGY, vol. 29, no. 2, 1 February 2018 (2018-02-01), NL, pages 332 - 340, XP055884451, ISSN: 0923-7534, DOI: 10.1093/annonc/mdx768**
• **CORINA BUECHELE ET AL: "4-1BB ligand modulates direct and Rituximab-induced NK-cell reactivity in chronic lymphocytic leukemia", EUROPEAN JOURNAL OF IMMUNOLOGY, WILEY-VCH, HOBOKEN, USA, vol. 42, no. 3, 20 December 2011 (2011-12-20), pages 737 - 748, XP071226196, ISSN: 0014-2980, DOI: 10.1002/EJI.201141920**

EP 3 765 489 B1

EP 3 765 489 B1

EP 3 765 489 B1

- **LIU R. ET AL.: "Efficient inhibition of human B-cell lymphoma in SCID mice by synergistic antitumor effect of human 4-1BB ligand/anti-CD20 fusion protein and anti-CD3/anti-CD20 diabodies.", J. IMMUNOTHER., vol. 33, no. 5, June 2010 (2010-06-01), pages 500 - 509, XP002791738**
- **XU W. ET AL.: "Design of CD19-4-1BBL, a novel CD19-targeted 4-1BB ligand for combination therapy with CD20 T-cell bispecific antibodies and CD20 antibodies.", CANCER RES, vol. 78, no. 13 Supp, 957, July 2018 (2018-07-01), XP002791739**

Remarks:

The complete document including Reference Table(s) and the Sequence Listing(s) can be downloaded from the EPO website

- **LIU R. ET AL.: "Efficient inhibition of human B-cell lymphoma in SCID mice by synergistic antitumor effect of human 4-1BB ligand/anti-CD20 fusion protein and anti-CD3/anti-CD20 diabodies.", J. IMMUNOTHER., vol. 33, no. 5, June 2010**

**Description**

**FIELD**

The present disclosure relates to combination therapies employing a 4-1BB (CD137) agonist comprising at least one antigen binding domain capable of specific binding to a tumor-associated antigen, in particular a CD19-targeted 4-1BBL antigen binding molecule, and specific antibodies which bind human CD20, the use of these combination therapies for the treatment of cancer and methods of using the combination therapies.

**BACKGROUND**

B-cell proliferative disorders describe a heterogeneous group of malignancies that includes both leukemias and lymphomas. Lymphomas develop from lymphatic cells and include two main categories: Hodgkin lymphomas (HL) and the non-Hodgkin lymphomas (NHL). In the United States, lymphomas of B cell origin constitute approximately 80-85% of all non-Hodgkin lymphoma cases, and there is considerable heterogeneity within the B-cell subset, based upon genotypic and phenotypic expression patterns in the B-cell of origin. For example, B cell lymphoma subsets include the slow-growing indolent and incurable diseases, such as Follicular lymphoma (FL) or chronic lymphocytic leukemia (CLL), as well as the more aggressive subtypes, mantle cell lymphoma (MCL) and diffuse large B cell lymphoma (DLBCL). Despite the availability of various agents for the treatment of B-cell proliferative disorders, there is an ongoing need for development of safe and effective therapies to prolong remission and improve cure rates in patients.

A strategy currently being investigated is the engagement of T cells against malignant B cells. In order to effectively engage T cells against malignant B cells, two recent approaches have been developed. These two approaches are: 1) the administration of T cells engineered ex *vivo* to recognize tumour cells (also known as chimeric antigen receptor-modified T cell therapy or CAR-T cells) (Maude et al., N Engl J Med (2014) 371,1507-1517) and, 2) the administration of agents that activate endogenous T cells, such as bispecific antibodies (Oak and Bartlett, Expert Opin Investig Drugs (2015) 24, 715-724). An example of the first approach is reported in the study by Maude et al., in which 30 adult and pediatric patients were treated with autologous T cells transduced with a CD19-directed chimeric antigen receptor lentiviral vector (CTL019 CAR-T cells). The result was a sustained remission based upon a 6-month event-free survival rate of 67% and an overall survival rate of 78%. However, all patients had cytokine release syndrome (CRS) (associated with tumour burden), with 27% of patients having severe CRS. Central nervous system toxicities of unknown cause were also noted at high frequencies. The second approach, which involves activating endogenous T cells to recognize tumour targets, bypasses this hurdle of scalability, and can also provide competitive efficacy, safety data and potentially long term durations of response. In different $CD20^+$ hematologic malignancies, this approach is best exemplified by blinatumomab, a CD19-CD3 targeting T cell bispecific molecule (Bargou et al., Science (2008) 321, 974-977) that was recently approved for patients with minimal residual disease-positive acute lymphocytic leukemia (ALL). This compound, which is composed of two single chain Fv fragments (the so called BiTE® format), directs the lysis of $CD19^+$ cells by cytolytic T cells. The primary constraint of blinatumomab is its short half-life (approximately 2 hours), which necessitates continuous infusion via a pump over 4-8 weeks. Furthermore, severe CRS and CNS toxicity have also been frequently observed (Klinger et al., Blood. 2012;119(26):6226-6233). In patients receiving blinatumomab in all clinical trials, neurological toxicities have occurred in approximately 50% of patients, and the types of toxicities observed are well-defined in the package insert.

4-1BB (CD137), a member of the TNF receptor superfamily, was first identified as an inducible molecule expressed by activated by T cells (Kwon and Weissman, Proc Natl Acad Sci USA (1989) 86, 1963-1967). Subsequent studies demonstrated that many other immune cells also express 4-1BB, including NK cells, B cells, NKT cells, monocytes, neutrophils, mast cells, dendritic cells (DCs) and cells of non-hematopoietic origin such as endothelial and smooth muscle cells (Vinay and Kwon, Cell Mol Immunol. (2011) 8, 281-284). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., J Immunol (2002) 168, 3755-3762; Zhang et al., J Immunol (2010) 184, 787-795).

4-1BB ligand (4-1BBL or CD137L) was identified in 1993 (Goodwin et al., Eur J Immunol. (1993) 23, 2631-2641). It has been shown that expression of 4-1BBL was restricted on professional antigen presenting cells (APC) such as B-cells, DCs and macrophages. Inducible expression of 4-1BBL is characteristic for T-cells, including both $\alpha\beta$ and $\gamma\delta$ T-cell subsets, and endothelial cells (Shao and Schwarz, J Leukoc Biol (2011) 89, 21-29).

Co-stimulation through the 4-1BB receptor (for example by 4-1BBL ligation) activates multiple signaling cascades within the T cell (both $CD4^+$ and $CD8^+$ subsets), powerfully augmenting T cell activation (Bartkowiak and Curran, Front Oncol (2015) 5, 117). In combination with TCR triggering, agonistic 4-1BB-specific antibodies enhance proliferation of T-cells, stimulate lymphokine secretion and decrease sensitivity of T-lymphocytes to activationinduced cells death (Snell et al., Immunol Rev (2011) 244, 197-217). This mechanism was further advanced as the first proof of concept in cancer immunotherapy. In a preclinical model administration of an agonistic antibody against 4-1BB in tumor bearing mice led to potent anti-tumor effect (Melero et al., Nature Med (1997) 3, 682-685). Later, accumulating evidence indicated that 4-1BB usually exhibits its potency as an anti-tumor agent only when administered in combination with other immunomodulatory compounds, chemotherapeutic reagents, tumorspecific vaccination or radiotherapy (Bartkowiak and Curran, Front Oncol (2015) 5, 117).

Signaling of the TNFR-superfamily needs cross-linking of the trimerized ligands to engage with the receptors, so does the 4-1BB agonistic antibodies which require wild type Fc-binding (Li and Ravetch, Science (2011) 333, 1030-1034). However, systemic administration of 4-1BB-specific agonistic antibodies with the functionally active Fc domain resulted in influx of CD8$^+$ T-cells associated with liver toxicity (Dubrot et al., Cancer Immunol Immunother (2010) 59, 1223-1233) that is diminished or significantly ameliorated in the absence of functional Fc-receptors in mice. In the clinic, an Fc-competent 4-1BB agonistic Ab (BMS-663513) (NCT00612664) caused a grade 4 hepatitis leading to termination of the trial (Simeone and Ascierto, J Immunotoxicol (2012) 9, 241-247). Therefore, there is a need for effective and safer 4-1BB agonists.

Fusion proteins composed of one extracellular domain of a 4-1BB ligand and a single chain antibody fragment (Hornig et al., J Immunother (2012) 35, 418-429; Müller et al., J Immunother. (2008) 31, 714-722) or a single 4-1BB ligand fused to the C-terminus of a heavy chain (Zhang et al., Clin Cancer Res (2007) 13, 2758-2767) have been made. Liu et al., J. Immunother. 2010, 33, 500-509, disclose a 4-1BB ligand/anti-CD20 fusion protein and its combination with an anti-CD3/anti-CD20 diabody. WO 2010/010051 discloses the generation of fusion proteins that consist of three TNF ligand ectodomains linked to each other and fused to an antibody part. In the present invention, antigen binding molecules composed of a trimeric and thus biologically active 4-1BB ligand and an antigen binding domain specific for the tumor antigen CD19 and an Fc-region devoid of FcγR-binding, are shown that are particularly stable and robust (herein named as CD19-4-1BBL). These constructs are disclosed in WO 2016/075278 and replace unspecific FcγR-mediated crosslinking responsible for Fc-mediated toxicity, by CD19-targeted B cell specific crosslinking.

CD19 is an ideal target for immunotherapy of B-cell malignancies as it is expressed on the surface of B-cells and is almost specific to these cells. CD19 is more broadly expressed than CD20 on B cells during the B cell development, so typically a CD20 positive cell will also express CD19. During differentiation of B cells towards plasma cells (antibody-secreting cells), B cells down-regulate CD20 expression. Sometimes, B cell lymhomas also down-regulate CD20 expression, but remain positive for CD19. Therefore, targeting both CD19 and CD20 would cover broadly the diseased B cells in lymphomas, which might also deviate the selection pressure from CD20 to both CD19 and CD20. Though it is not known if CD19 contributes directly to B cell carcinogenesis, its expression is highly conserved on most B cell tumors such as acute lymphoblastic leukemias (ALL), chronic lymphocytic leukemias (CLL) and B cell lymphomas. In acute leukemias CD19 is steadily and continuously expressed on almost all subtypes while only a small number of leukemias express CD20.

The use of rituximab and obinutuzumab, which are anti-CD20 antibodies, in treating B-cell proliferative disorder has been described, for instance, in: Robert(2017), NEJM 377(14):1331-1344; and in Karmali (2018), Annals Onc. 29(2):332-340.

There is thus still a need for new compounds and combinations which have the potential to significantly contribute to the treatment of patients with B-cell proliferative disorders such as NHL and CLL. It has been found that an excellent anti-tumor effect is achieved when the CD19-targeted 4-1BBL antigen binding molecule is combined with specific antibodies which bind human CD20. Thus, we herein describe a novel combination therapy for cancer with B cell malignancy.

## SUMMARY OF THE INVENTION

The present invention relates to a 4-1BB (CD137) agonist comprising one antigen binding domain capable of specific binding to CD19, in particular an antigen binding molecule comprising three ectodomains of 4-1BBL, and its use in combination and concomitant with antibodies that bind human CD20, in particular rituximab or obinutuzumab, in a method for treating or delaying progression of cancer, more particularly for treating or delaying progression of B-cell proliferative disorders. It has been found that the combination therapy described herein is more effective in inhibiting tumor growth and eliminating tumor cells than treatment with anti-CD20 antibodies alone.

In one aspect, the invention provides a 4-1BB (CD137) agonist for use in a method for treating a B-cell proliferative disorder, wherein the 4-1BB agonist is used in combination with an anti-CD20 antibody selected from rituximab or obinutuzumab, wherein:

(a) said B-cell proliferative disorder is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL); and

(b) said 4-1BB agonist is an antigen binding molecule comprising

(i) one antigen binding domain capable of specific binding to CD19,
(ii) a first and a second polypeptide that are linked to each other by a disulfide bond, and
(iii) an IgG1 Fc domain comprising the amino acid substitutions L234A, L235A and P329G (EU numbering); and

(c) said first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other by a peptide linker and said second polypeptide comprises one ectodomain of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8. In a further aspect, the 4-1BB agonist comprising one antigen binding domain capable of specific binding to CD19 and the anti-CD20 antibody are administered together in a single composition or administered separately in two different compositions.

In one aspect, the ectodomains of 4-1BBL comprise an amino acid sequence of SEQ ID NO:1 or SEQ ID NO:5. More particularly, the ectodomains of 4-1BBL comprise an amino acid sequence of SEQ ID NO:5.

In one aspect, the antigen binding domain capable of specific binding to CD19 comprises (a) a heavy chain variable region ($V_H$CD19) comprising (i) CDR-H1 comprising the amino acid sequence of SEQ ID NO:9, (ii) CDR-H2 comprising the amino acid sequence of SEQ ID NO:10, and (iii) CDR-H3 comprising the amino acid sequence of SEQ ID NO:11, and a light chain variable region ($V_L$CD19) comprising (iv) CDR-L1 comprising the amino acid sequence of SEQ ID NO:12, (v) CDR-L2 comprising the amino acid sequence of SEQ ID NO:13, and (vi) CDR-L3 comprising the amino acid sequence of SEQ ID NO:14, or (b) a heavy chain variable region ($V_H$CD19) comprising (i) CDR-H1 comprising the amino acid sequence of SEQ ID NO: 15, (ii) CDR-H2 comprising the amino acid sequence of SEQ ID NO:16, and (iii) CDR-H3 comprising the amino acid sequence of SEQ ID NO:17, and a light chain variable region ($V_L$CD19) comprising (iv) CDR-L1 comprising the amino acid sequence of SEQ ID NO:18, (v) CDR-L2 comprising the amino acid sequence of SEQ ID NO:19, and (vi) CDR-L3 comprising the amino acid sequence of SEQ ID NO:20.

In a particular aspect, the antigen binding domain capable of specific binding to CD19 comprises a heavy chain variable region ($V_H$CD19) comprising an amino acid sequence of SEQ ID NO:21 and a light chain variable region ($V_L$CD19) comprising an amino acid sequence of SEQ ID NO:22 or the antigen binding domain capable of specific binding to CD19 comprises a heavy chain variable region ($V_H$CD19) comprising an amino acid sequence of SEQ ID NO:23 and a light chain variable region ($V_L$CD19) comprising an amino acid sequence of SEQ ID NO:24. In a particular aspect, the antigen binding domain capable of specific binding to CD19 comprises a heavy chain variable region ($V_H$CD19) comprising an amino acid sequence of SEQ ID NO:23 and a light chain variable region ($V_L$CD19) comprising an amino acid sequence of SEQ ID NO:24.

In a particular aspect, provided is the 4-1BB agonist for use in a method for treating a B-cell proliferative disorder selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL) in combination with an anti-CD20 antibody, wherein the 4-1BB agonist is a molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:41 and a second light chain comprising the amino acid sequence of SEQ ID NO:42.

In another aspect, provided is the 4-1BB agonist for use in a method for treating a B-cell proliferative disorder selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL) in combination with an anti-CD20 antibody as defined herein before, wherein the combination is administered concomitant at intervals from about about one week to three weeks.

In another aspect, provided is the 4-1BB agonist for use in a method for treating for treating a B-cell proliferative disorder selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL) in combination with an anti-CD20 antibody, wherein a pretreatment with an Type II anti-CD20 antibody, preferably obinutuzumab, is performed prior to the combination treatment, wherein the period of time between the pretreatment and the combination treatment is sufficient for the reduction of B-cells in the individual in response to the Type II anti-CD20 antibody, preferably obinutuzumab.

In another aspect, provided is a pharmaceutical composition which comprises the 4-1BB agonist as defined herein before and an anti-CD20 antibody selected from rituximab or obinutuzumab, wherein the 4-1BB agonist and the anti-CD20 antibody are administered together in a single composition. In particular, the pharmaceutical composition is for use in in the treatment of B-cell proliferative disorders, in particular a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).

## BRIEF DESCRIPTION OF THE DRAWINGS

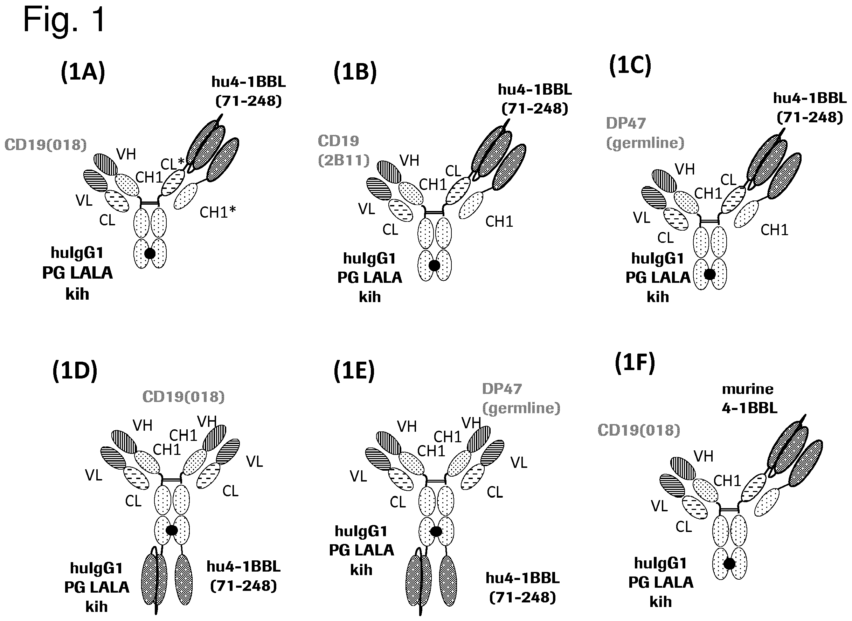

**Figure 1** shows particular CD19-4-1BBL antigen binding molecules as used in the Examples. These molecules are described in more detail in Examples 1 and 2, respectively. The thick black point stands for the knob-into-hole modification. Figure 1A shows a monovalent CD19 4-1BBL-trimer containing antigen binding molecule with amino acid modifications in the CH1 and CL domain adjacent to the 4-1BBL dimer and 4-1BBL monomer. As it comprised the CD19 binder 8B8-018, it was named mono CD19(018)-4-1BBL herein. The construct shown in Figure 1B differs from 1A in that it comprises the CD19 binder 8B8-2B11 and was thus termed mono CD19(2B11)-4-1BBL. Figure 1D shows the bivalent construct with binder CD19(018), termed bi CD19(018)-4-1BBL. Figures 1C and 1E show untargeted control molecules (the CD19 binder has been replaced by a non-binding DP47 Fab). Figure 1F shows a monovalent CD19 4-1BBL-trimer containing antigen binding molecule comprising a murine 4-1BBL dimer and a murine 4-1BBL monomer, termed herein hybrid CD19-4-1BBL or CD19-mu4-1BBL.

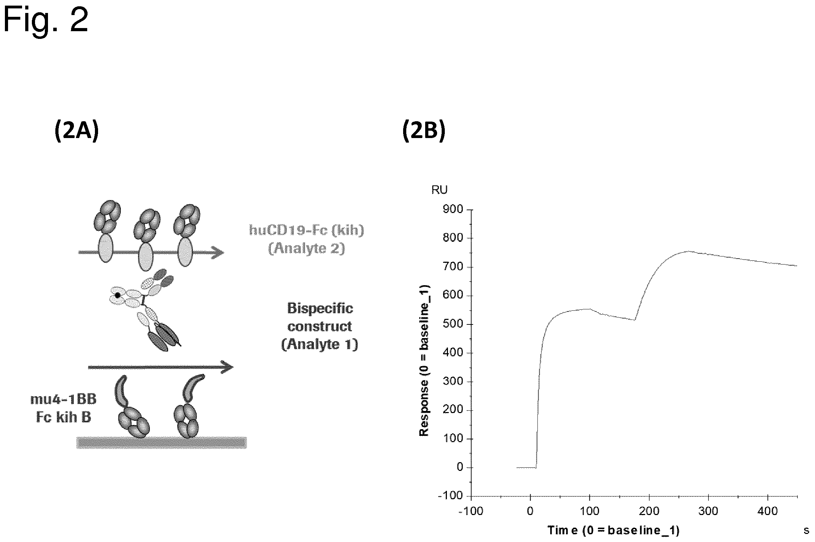

**Figure 2A** illustrates the setup of the assay measuring simultaneous binding of CD 19 targeted trimeric split 4-1BBL to murine 4-1BB and human CD19 (Example 2). The graph in **Figure 2B** shows simultaneous binding of the hybrid surrogate CD19-mu4-1BBL (Analyte 1) to immobilized murine 4-1BB and human CD19 (Analyte 2).

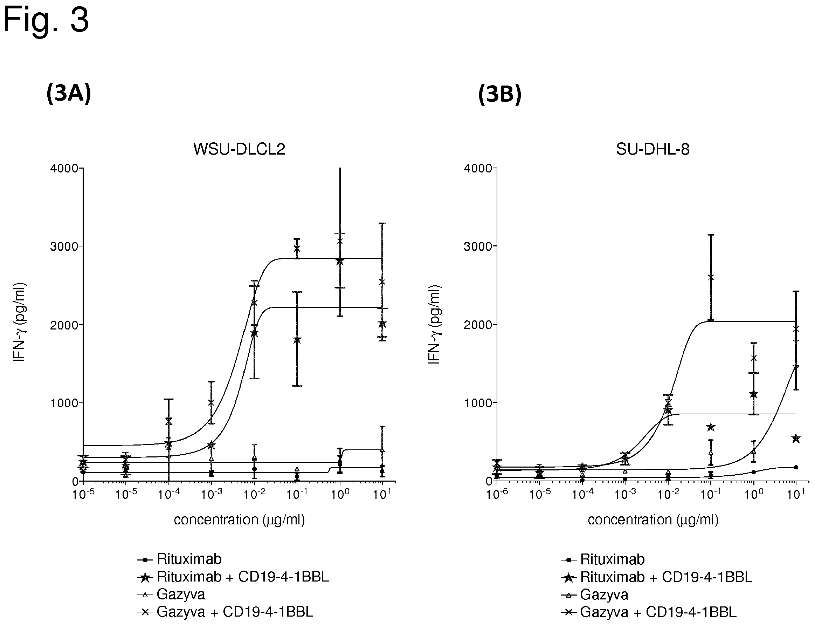

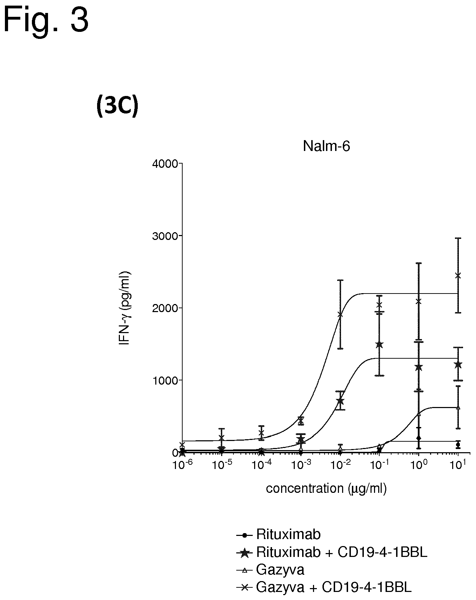

In **Figures 3A to 3C** it is shown that CD19-4-1BBL could boost activation of NK cells (IFNy release) that were activated by rituximab or obinutuzumab (Gazyva) when engaging with NHL cell lines, i.e with WSU-DLCL2 (CD19$^+$CD20$^{high}$) (**Fig. 3A**), SU-DHL-8 (CD19$^+$CD20$^{low}$) (**Fig. 3B**), or Naml-6 (CD19$^+$CD20$^{low}$) (**Fig. 3C**).

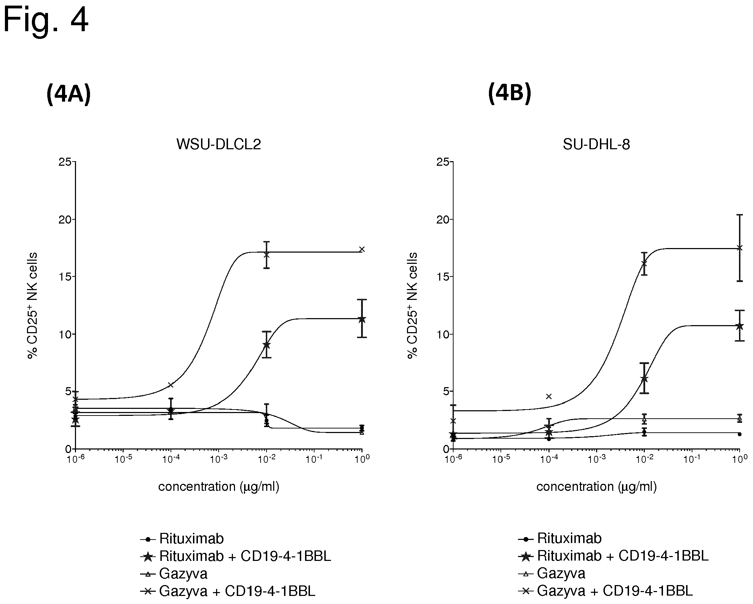

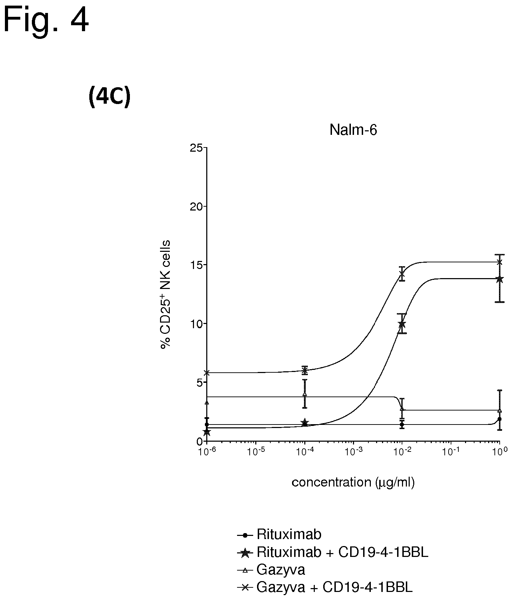

**Figures 4A to 4C** show that by combining CD19-4-1BBL with rituximab or obinutuzumab (Gazyva) activation of NK cells (CD25 upregulation) is boosted when engaging with NHL cell lines, i.e with WSU-DLCL2 (CD19$^+$CD20$^{high}$) (**Fig. 4A**), SU-DHL-8 (CD19$^+$CD20$^{low}$) (**Fig. 4B**), or Naml-6 (CD19$^+$CD20$^{low}$) (**Fig. 4C**).

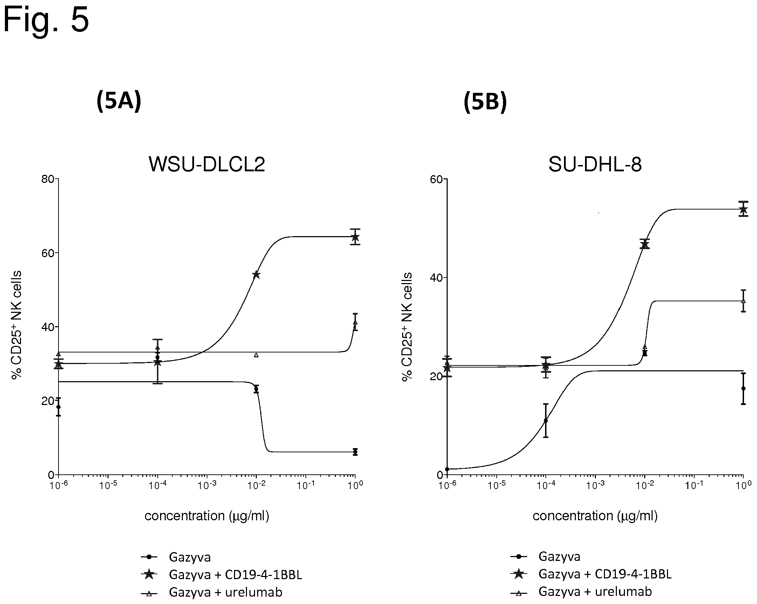

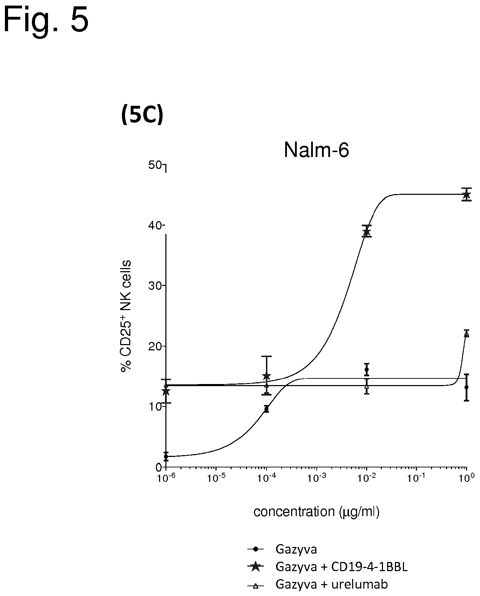

In **Figures 5A to 5C** it is shown that the combination of CD19-4-1BBL and obinutuzumab (Gazyva) boosts activation of NK cells (CD25 upregulation) when engaging with the NHL cell lines WSU-DLCL2 (**Fig. 5A**), SU-DHL-8 (**Fig. 5B**), or Naml-6 (**Fig. 5C**), whereas the combination of urelumab with obinutuzumab does not.

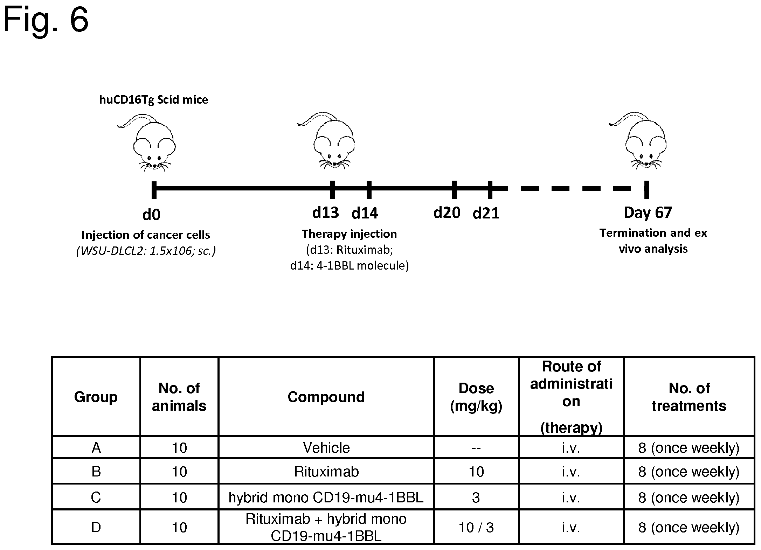

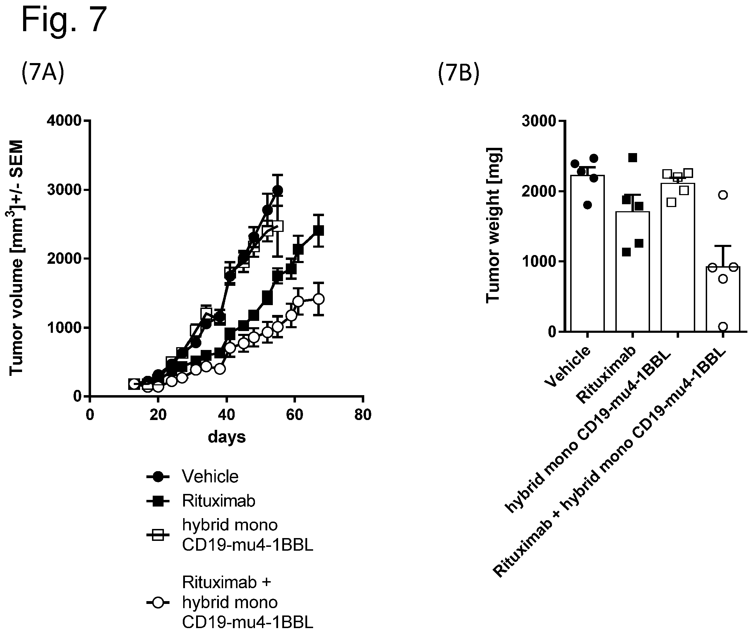

**Figure 6** shows the protocol of the *in vivo* efficacy study of the monovalent hybrid CD19-4-1BBL molecule (mono CD19-mu4-1BBL) in combination with rituximab in huCD16Tg Scid mice. In the table below the subgroups of mice receiving different combinations and doses are defined. The experiment is described in Example 4 and the results are shown in **Figures 7A** and **7B.** The combination of mono CD19-mu4-1BBL in combination with rituximab did induce stronger and faster tumor growth inhibition as compared to monotherapy with rituximab or monotherapy with mono CD19-mu4-1BBL in all doses tested. As shown in **Figure 7B,** the tumor weights at study termination confirmed these findings.

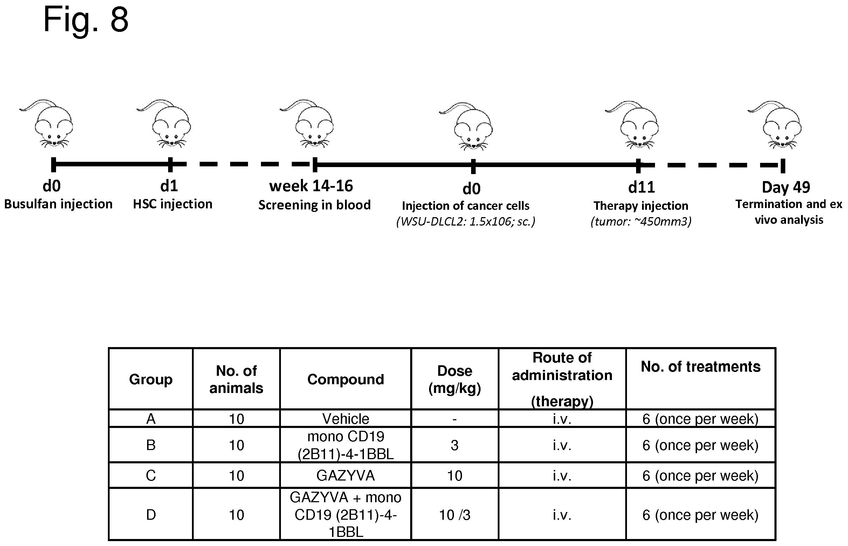

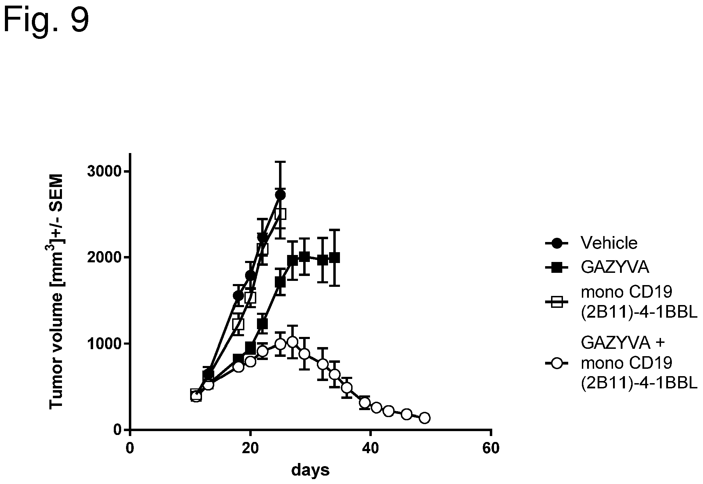

**Figure 8** shows the protocol of the *in vivo* efficacy study of the monovalent CD19 (2B11)-4-1BBL construct in combination with obinutuzumab (Gazyva) in WSU-DLCL2-bearing fully humanized NSG mice. In the table below the subgroups of mice receiving different combinations and doses are defined. The experiment is described in Example 5 and the results are shown in **Figure 9.** The combination of mono CD19 (2B11)-4-1BBL with Gazyva induced much stronger tumor growth inhibition as compared to monotherapy with Gazyva.

## DETAILED DESCRIPTION

The technical disclosure set out below may in some respects go beyond the scope of the claims. Elements of the disclosure which do not fall within the scope of the claims are provided for information.

**Definitions**

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term "**antigen binding molecule**" refers in its broadest sense to a molecule that specifically binds an antigenic determinant. Examples of antigen binding molecules are antibodies, antibody fragments and scaffold antigen binding proteins.

The term "**antibody**" herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term "**monoclonal antibody**" as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term "**monospecific**" antibody as used herein denotes an antibody that has one or more binding sites each of which bind to the same epitope of the same antigen. The term "**bispecific**" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "**valent**" as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule. "Monovalent" denotes the presence of only one binding domain for a particular target in the antigen binding molecule.

The terms "full length antibody", "intact antibody", and "whole antibody" are used herein interchangeably to refer to an antibody having a structure substantially similar to a native antibody structure. "**Native antibodies**" refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called $\alpha$ (IgA), $\delta$ (IgD), $\epsilon$ (IgE), $\gamma$ (IgG), or $\mu$ (IgM), some of which may be further divided into subtypes, e.g. $\gamma$1 (IgG1), y2 (IgG2), y3 (IgG3), y4 (IgG4), $\alpha$1 (IgA1) and $\alpha$2 (IgA2). The light chain of an antibody may be assigned to one of two types, called kappa ($\kappa$) and lambda ($\lambda$), based on the amino acid sequence of its constant domain.

An "**antibody fragment**" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain aspects, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term "**Fab fragment**" refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an $F(ab')_2$ fragment that has two antigen-combining sites (two Fab fragments) and a part of the Fc region.

The term "**cross-Fab fragment**" or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab $_{(VLVH)}$. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab $_{(CLCH1)}$.

A "single chain Fab fragment" or "**scFab**" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or "**x-scFab**" is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A "**single-chain variable fragment (scFv)**" is a fusion protein of the variable regions of the heavy ($V_H$) and light chains ($V_L$) of an antibody, connected with a short linker peptide of ten to about 25 amino acids. The linker is usually rich in glycine for flexibility, as well as serine or threonine for solubility, and can either connect the N-terminus of the $V_H$ with the C-terminus of the $V_L$, or *vice versa*. This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

"**Scaffold antigen binding proteins**" are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), $V_{NAR}$ fragments, a fibronectin (AdNectin), a C-type lectin domain (Tetranectin); a variable domain of a new antigen receptor beta-lactamase ($V_{NAR}$ fragments), a human gamma-crystallin or ubiquitin (Affilin molecules); a kunitz type domain of human protease inhibitors, microbodies such as the proteins from the knottin family, peptide aptamers and fibronectin (adnectin).

Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633.

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

A single-domain antibody is an antibody fragment consisting of a single monomeric variable antibody domain. The first single domains were derived from the variable domain of the antibody heavy chain from camelids (nanobodies or $V_H$H fragments). Furthermore, the term single-domain antibody includes an autonomous human heavy chain variable domain (aVH) or $V_{NAR}$ fragments derived from sharks.

An "**antigen binding molecule that binds to the same epitope**" as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term "**antigen binding domain**" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term "**antigenic determinant**" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moietyantigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

By "**specific binding**" is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, a molecule that binds to the antigen has a dissociation constant (Kd) of $\leq 1\ \mu M$, $\leq 100\ nM$, $\leq 10\ nM$, $\leq 1\ nM$, $\leq 0.1\ nM$, $\leq 0.01\ nM$, or $\leq 0.001\ nM$ (e.g. $10^{-8}$ M or less, e.g. from $10^{-8}$ M to $10^{-13}$ M, e.g. from $10^{-9}$ M to $10^{-13}$ M).

"**Affinity**" or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants ($k_{off}$ and $k_{on}$, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A "**B-cell antigen**" as used herein refers to an antigenic determinant presented on the surface of a B lymphocyte, particularly a malignant B lymphocyte (in that case the antigen also being referred to as "malignant B-cell antigen").

The term "**tumor-associated antigen**" means any antigen that is highly expressed by tumor cells or in the tumor stroma. A particular tumor-associated antigen is CD19.

The term "**CD19**" refers to B-lymphocyte antigen CD19, also known as B-lymphocyte surface antigen B4 or T-cell surface antigen Leu-12 and includes any native CD19 from any vertebrate source, including mammals such as primates (e.g. humans) non-human primates (e.g. cynomolgus monkeys) and rodents (e.g. mice and rats), unless otherwise indicated. The amino acid sequence of human CD19 is shown in Uniprot accession no. P15391 (version 160, SEQ ID NO:62). The term encompasses "full-length" unprocessed human CD19 as well as any form of human CD19 that results from processing in the cell as long as the antibody as reported herein binds thereto. CD19 is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias. The expression of CD19 on plasma cells further suggests it may be expressed on differentiated B cell tumors such as multiple myeloma. Therefore, the CD19 antigen is a target for immunotherapy in the treatment of non-Hodgkin's lymphoma, chronic lymphocytic leukemia and/or acute lymphoblastic leukemia.

"CD20" refers to B-lymphocyte antigen CD20, also known as B-lymphocyte surface antigen B1 or Leukocyte surface antigen Leu-16, and includes any native CD20 from any vertebrate source, including mammals such as primates (e.g. humans) non-human primates (e.g. cynomolgus monkeys) and rodents (e.g. mice and rats), unless otherwise indicated. The amino acid sequence of human CD20 is shown in Uniprot accession no. P11836 (version 149, SEQ ID NO:63). CD20 is a hydrophobic transmembrane protein with a molecular weight of approximately 35 kD expressed on pre-B and mature B lymphocytes. The corresponding human gene is membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 1 1q12, among a cluster of family members. Alternative splicing of this gene results in two transcript variants which encode the same protein. The term "CD20" encompasses "full-length," unprocessed CD20 as well as any form of CD20 that results from processing in the cell. The term also encompasses naturally occurring variants of CD20, e.g., splice variants or allelic variants.

The terms "anti-CD20 antibody" and "an antibody that binds to CD20" refer to an antibody that is capable of binding CD20 with sufficient affinity such that the antibody is useful as a diagnostic and/or therapeutic agent in targeting CD20. In one embodiment, the extent of binding of an anti-CD20 antibody to an unrelated, non-CD20 protein is less than about 10% of the binding of the antibody to CD20 as measured, e.g., by a radioimmunoassay (RIA). In certain embodiments, an antibody that binds to CD20 has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In certain embodiments, an anti-CD20 antibody binds to an epitope of CD20 that is conserved among CD20 from different species.

By "Type II anti-CD20 antibody" is meant an anti-CD20 antibody having binding properties and biological activities of Type II anti-CD20 antibodies as described in Cragg et al., Blood 103 (2004) 2738-2743; Cragg et al., Blood 101 (2003) 1045-1052, Klein et al., mAbs 5 (2013), 22-33, and summarized in Table 1 below.

**TABLE A. Properties of type I and type II anti-CD20 antibodies**

| type I anti-CD20 antibodies | type II anti-CD20 antibodies |
|---|---|
| Bind class I CD20 epitope | Bind class II CD20 epitope |
| Localize CD20 to lipid rafts | Do not localize CD20 to lipid rafts |
| High CDC * | Low CDC * |
| ADCC activity * | ADCC activity * |
| Full binding capacity to B cells | Approx. half binding capacity to B cells |
| Weak homotypic aggregation | Homotypic aggregation |
| Low cell death induction | Strong cell death induction |
| * if IgG$_1$ isotype | |

Examples of type II anti-CD20 antibodies include e.g. obinutuzumab (GA101), tositumumab (B1), humanized B-Ly1 antibody IgG1 (a chimeric humanized IgG1 antibody as disclosed in WO 2005/044859), 11B8 IgG1 (as disclosed in WO 2004/035607) and AT80 IgG1.

In one aspect, the Type II anti-CD20 antibody comprises the heavy chain variable region sequence (V$_H$CD20) comprising the HCDR1 of SEQ ID NO:73, the HCDR2 of SEQ ID NO:74, and the HCDR3 of SEQ ID NO:75 and the light chain variable region sequence (V$_L$CD20) comprising the LCDR1 of SEQ ID NO:76, the LCDR2 of SEQ ID NO:77, and the LCDR3 of SEQ ID NO:78. In another aspect, the Type II anti-CD20 antibody is engineered to have an increased proportion of non-fucosylated oligosaccharides in the Fc region as compared to a non-engineered antibody. In one aspect, at least about 40% of the N-linked oligosaccharides in the Fc region of the Type II anti-CD20 antibody are non-fucosylated.

In a particular aspect, the Type II anti-CD20 antibody is obinutuzumab (recommended INN, WHO Drug Information, Vol. 26, No. 4, 2012, p. 453). As used herein, obinutuzumab is synonymous for GA101. The tradename is GAZYVA® or GAZYVARO®. This replaces all previous versions (e.g. Vol. 25, No. 1, 2011, p.75-76), and is formerly known as afutuzumab (recommended INN, WHO Drug Information, Vol. 23, No. 2, 2009, p. 176; Vol. 22, No. 2, 2008, p. 124). In one embodiment, the Type II anti-CD20 antibody comprises the heavy chain variable region sequence of SEQ ID NO:79 and the light chain variable region sequence of SEQ ID NO: 80. In one aspect, the Type II anti-CD20 antibody is tositumomab.

By "Type I anti-CD20 antibody" is meant an anti-CD20 antibody selected from the group consisting of rituximab, ofatumumab, veltuzumab, ocaratuzumab, ocrelizumab, PRO131921, ublituximab, HI47 IgG3 (ECACC, hybridoma), 2C6 IgG1 (as disclosed in WO 2005/103081), 2F2 IgG1 (as disclosed in WO 2004/035607 and WO 2005/103081) and 2H7 IgG1 (as disclosed in WO 2004/056312). A particular Type I anti-CD20 antibody is rituximab.

"Rituximab" is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Andersen et. al.) issued on April 17, 1998, assigned to IDEC Pharmaceuticals Corporation. Rituximab is approved for the treatment of patients with relapsed or refracting lowgrade or follicular, CD20 positive, B cell non-Hodgkin's lymphoma. In vitro mechanism of action studies have shown that rituximab exhibits human complement--dependent cytotoxicity (CDC) (Reff, M.E., et. al., Blood 83 (1994) 435-445). Additionally, it exhibits significant activity in assays that measure antibody-dependent cellular cytotoxicity (ADCC). Rituximab is not afucosylated.

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO:64; variable region of the murine light chain (VL): SEQ ID NO:65 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

The term "**reduction**" (and grammatical variations thereof such as "reduce" or "reducing"), for example reduction of the number of B cells or cytokine release, refers to a decrease in the respective quantity, as measured by appropriate methods known in the art. For clarity the term includes also reduction to zero (or below the detection limit of the analytical method), i.e. complete abolishment or elimination. Conversely, "**increased**" refers to an increase in the respective quantity.

A "**T-cell antigen**" as used herein refers to an antigenic determinant presented on the surface of a T lymphocyte, particularly a cytotoxic T lymphocyte.

A "**T cell activating therapeutic agent**" as used herein refers to a therapeutic agent capable of inducing T cell activation in a subject, particularly a therapeutic agent designed for inducing T-cell activation in a subject. Examples of T cell activating therapeutic agents include bispecific antibodies that specifically bind an activating T cell antigen, such as CD3, and a target cell antigen, such as CD20 or CD19. Further examples include chimeric antigen receptors (CARs) which comprise a T cell activating domain and an antigen binding moiety that specifically binds to a target cell antigen, such as CD20 or CD19.

An "**activating T cell antigen**" as used herein refers to an antigenic determinant expressed by a T lymphocyte, particularly a cytotoxic T lymphocyte, which is capable of inducing or enhancing T cell activation upon interaction with an antigen binding molecule. Specifically, interaction of an antigen binding molecule with an activating T cell antigen may induce T cell activation by triggering the signaling cascade of the T cell receptor complex. An exemplary activating T cell antigen is CD3.

The term "**variable region**" or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "**hypervariable region**" or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementary determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table B as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE B. CDR Definitions[1]**

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$ CDR1 | 24-34 | 26-32 | 24-34 |
| $V_L$ CDR2 | 50-56 | 50-52 | 50-56 |
| $V_L$ CDR3 | 89-97 | 91-96 | 89-97 |

[1] Numbering of all CDR definitions in Table A is according to the numbering conventions set forth by Kabat et al. (see below).

[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or α-CDRs. Exemplary α-CDRs (a-CDR-L1, α-CDR-L2, α-CDR-L3, α-CDR-H1, α-CDR-H2, and α-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra*.

As used herein, the term "**affinity matured**" in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

"**Framework**" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "**acceptor human framework**" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "**chimeric**" antibody refers to an antibody in which a portion of the heavy and/or light chain is derived from a particular source or species, while the remainder of the heavy and/or light chain is derived from a different source or species.

The "**class**" of an antibody refers to the type of constant domain or constant region possessed by its heavy chain. There are five major classes of antibodies: IgA, IgD, IgE, IgG, and IgM, and several of these may be further divided into subclasses (isotypes), e.g. $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, y, and $\mu$ respectively..

A "**humanized**" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "**humanized form**" of an antibody, e.g., a non-human antibody, refers to an antibody that has undergone humanization. Other forms of "humanized antibodies" encompassed herein are those in which the constant region has been additionally modified or changed from that of the original antibody to generate the properties according to the disclosure, especially in regard to C1q binding and/or Fc receptor (FcR) binding.

A "**human**" antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or "**Fc region**" herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The "**knob-into-hole**" technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)).

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term "**effector functions**" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

An "**activating Fc receptor**" is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcαRI (CD89). A particular activating Fc receptor is human FcγRIIIa (see UniProt accession no. P08637, version 141).

As used herein, the term "**effector cells**" refers to a population of lymphocytes that display effector moiety receptors, e.g. cytokine receptors, and/or Fc receptors on their surface through which they bind an effector moiety, e.g. a cytokine, and/or an Fc region of an antibody and contribute to the destruction of target cells, e.g. tumor cells. Effector cells may for example mediate cytotoxic or phagocytic effects. Effector cells include, but are not limited to, effector T cells such as CD8$^+$cytotoxic T cells, CD4$^+$ helper T cells, γδ T cells, NK cells, lymphokineactivated killer (LAK) cells and macrophages/monocytes.

An "**ectodomain**" is the domain of a membrane protein that extends into the extracellular space (i.e. the space outside the target cell). Ectodomains are usually the parts of proteins that initiate contact with surfaces, which leads to signal transduction. The ectodomain of 4-1BBL as defined herein thus refers to the part of the 4-1BBL that extends into the extracellular space (the extracellular domain), but also includes shorter parts or fragments thereof that are responsible for the trimerization and for the binding to the corresponding receptor 4-1BB. The term "ectodomain of 4-1BBL or a fragment thereof' thus refers to the extracellular domain of 4-1BBL that forms the extracellular domain or to parts thereof that are still able to bind to the receptor (receptor binding domain).

"**4-1BBL**" or "**4-1BB ligand**" or "**CD137L**" is a costimulatory TNF ligand family member, which is able to costimulate proliferation and cytokine production of T-cells. Costimulatory TNF family ligands can costimulate TCR signals upon interaction with their corresponding TNF receptors and the interaction with their receptors leads to recruitment of TNFR-associated factors (TRAF), which initiate signalling cascades that result in T-cell activation. 4-1BBL is a type II transmembrane protein. Complete or full length 4-1BBL having the amino acid sequence of SEQ ID NO:66 has been described to form trimers on the surface of cells. The formation of trimers is enabled by specific motives of the ectodomain of 4-1BBL. Said motives are designated herein as "trimerization region". The amino acids 50-254 of the human 4-1BBL sequence (SEQ ID NO:67) form the extracellular domain of 4-1BBL, but even fragments thereof are able to form the trimers. In specific embodiments, the term "ectodomain of 4-1BBL or a fragment thereof' refers to a polypeptide having an amino acid sequence selected from SEQ ID NO:4 (amino acids 52-254 of human 4-1BBL), SEQ ID NO:1 (amino acids 71-254 of human 4-1BBL), SEQ ID NO:3 (amino acids 80-254 of human 4-1BBL), SEQ ID NO:2 (amino acids 85-254 of human 4-1BBL), SEQ ID NO:5 (amino acids 71-248 of human 4-1BBL), SEQ ID NO:6 (amino acids 85-248 of human 4-1BBL), SEQ ID NO:7 (amino acids 80-248 of human 4-1BBL) and SEQ ID NO:8 (amino acids 52-248 of human 4-1BBL), but also other fragments of the ectodomain capable of trimerization are included herein.

The term "**4-1BB**" or "**CD137**", as used herein, refers to any native 4-1BB from any vertebrate source, including mammals such as primates (e.g. humans) and rodents (e.g., mice and rats), unless otherwise indicated. The term encompasses "full-length," unprocessed 4-1BB as well as any form of 4-1BB that results from processing in the cell. The term also encompasses naturally occurring variants of 4-1BB, e.g., splice variants or allelic variants. The amino acid sequence of an exemplary human 4-1BB is shown in SEQ ID NO: 68 (Uniprot accession no. Q07011), the amino acid sequence of an exemplary murine 4-1BB is shown in SEQ ID NO: 69 (Uniprot accession no. P20334) and the amino acid sequence of an exemplary cynomolgous 4-1BB (from Macaca mulatta) is shown in SEQ ID NO:70 (Uniprot accession no. F6W5G6).

The terms "**anti-4-1BB antibody**", "anti-4-1BB", "4-1BB antibody and "an antibody that specifically binds to 4-1BB" refer to an antibody that is capable of binding 4-1BB with sufficient affinity such that the antibody is useful as a diagnostic and/or therapeutic agent in targeting 4-1BB. In one embodiment, the extent of binding of an anti-4-1BB antibody to an unrelated, non-4-1BB protein is less than about 10% of the binding of the antibody to 4-1BB as measured, e.g., by a radioimmunoassay (RIA) or flow cytometry (FACS). In certain embodiments, an antibody that binds to 4-1BB has a dissociation constant ($K_D$) of ≤ 1μM, ≤ 100 nM, ≤ 10 nM, ≤ 1 nM, ≤ 0.1 nM, ≤ 0.01 nM, or ≤ 0.001 nM (e.g. 10$^{-6}$ M

or less, e.g. from $10^{-68}$ M to $10^{-13}$ M, e.g., from $10^{-8}$ M to $10^{-10}$ M). For example, urelumab is an anti-4-1BB antibody derived from antibody 20H4.9.

The term "**peptide linker**" refers to a peptide comprising one or more amino acids, typically about 2 to 20 amino acids. Peptide linkers are known in the art or are described herein. Suitable, non-immunogenic linker peptides are, for example, $(G_4S)_n$, $(SG_4)_n$ or $G_4(SG_4)_n$ peptide linkers, wherein "n" is generally a number between 1 and 10, typically between 2 and 4, in particular 2, i.e. the peptides selected from the group consisting of GGGGS (SEQ ID NO: 81) GGGGSGGGGS (SEQ ID NO:82), SGGGGSGGGG (SEQ ID NO:83) and GGGGSGGGGSGGGG (SEQ ID NO:84), but also include the sequences GSPGSSSSGS (SEQ ID NO:85), $(G4S)_3$ (SEQ ID NO:86), $(G4S)_4$ (SEQ ID NO:87), GSGSGSGS (SEQ ID NO:88), GSGSGNGS (SEQ ID NO:89), GGSGSGSG (SEQ ID NO:90), GGSGSG (SEQ ID NO:91), GGSG (SEQ ID NO:92), GGSGNGSG (SEQ ID NO:93), GGNGSGSG (SEQ ID NO:94) and GGNGSG (SEQ ID NO:95). Peptide linkers of particular interest are (G4S) (SEQ ID NO:81) and $(G_4S)_2$ (SEQ ID NO:82).

The term "**amino acid**" as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

By "fused" or "connected" is meant that the components (e.g. a polypeptide and an ectodomain of 4-1BBL) are linked by peptide bonds, either directly or via one or more peptide linkers.

"**Percent (%) amino acid sequence identity**" with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the antigen binding molecules provided herein are contemplated. For example, it may be desirable to improve the binding affinity and/or other biological properties of the antigen binding molecules. Amino acid sequence variants of the antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table C under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE C**

| Original Residue | Exemplary Substitutions | Preferred Substitutions |
| --- | --- | --- |
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (1) | Leu; Val; Met; Ala; Phe Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.
The term "**amino acid sequence variants**" includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more CDR residues are mutated and the variant antigen binding molecules displayed on phage and screened for a particular biological activity (e.g. binding affinity). In certain embodiments, substitutions, insertions, or deletions may occur within one or more CDRs so long as such alterations do not substantially reduce the ability of the antigen binding molecule to bind antigen. For example, conservative alterations (e.g., conservative substitutions as provided herein) that do not substantially reduce binding affinity may be made in CDRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include antigen binding molecules with an N-terminal methionyl residue. Other insertional variants of the molecule include the fusion to the N- or C-terminus to a polypeptide which increases the serum half-life of the antigen binding molecules.

In certain embodiments, the antigen binding molecules provided herein are altered to increase or decrease the extent to which the antibody is glycosylated. Glycosylation variants of the molecules may be conveniently obtained by altering the amino acid sequence such that one or more glycosylation sites is created or removed. Where the antigen binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in the antigen binding molecules may be made in order to create variants with certain improved properties. In one aspect, variants of antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of the antigen binding molecules include those with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

Engineering", particularly with the prefix "glyco-", as well as the term "glycosylation engineering" includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one aspect, the glycosylation engineering is an alteration in glycosyltransferase activity. In a particular aspect, the engineering results in altered glucosaminyltransferase activity and/or fucosyltransferase activity. Glycosylation engineering can be used to obtain a "host cell having increased GnTIII activity" (e.g. a host cell that has been manipulated to express increased levels of one or more polypeptides having $\beta(1,4)$-N-acetylglucosaminyltransferase III (GnTIII) activity), a "host cell having increased ManII activity" (e.g. a host cell that has been manipulated to express increased levels of one or more polypeptides having $\alpha$-mannosidase II (ManII) activity), or a "host cell having decreased $\alpha(1,6)$ fucosyltransferase activity" (e.g. a host cell that has been manipulated to express decreased levels of $\alpha(1,6)$ fucosyltransferase).

As used herein, the term "polypeptide having GnTIII activity" refers to polypeptides that are able to catalyze the addition of a N-acetylglucosamine (GlcNAc) residue in $\beta$-1,4 linkage to the $\beta$-linked mannoside of the trimannosyl core of N-linked oligosaccharides. This includes fusion polypeptides exhibiting enzymatic activity similar to, but not necessarily identical to, an activity of $\beta(1,4)$-N-acetylglucosaminyltransferase III, also known as $\beta$-1,4-mannosyl-glycoprotein 4-beta-N-acetylglucosaminyl-transferase (EC 2.4.1.144), according to the Nomenclature Committee of the International Union of Biochemistry and Molecular Biology (NC-IUBMB), as measured in a particular biological assay, with or without dose dependency. In the case where dose dependency does exist, it needs not be identical to that of GnTIII, but rather substantially similar to the dose-dependency in a given activity as compared to the GnTIII (i.e. the candidate polypeptide will exhibit greater activity or not more than about 25-fold less and, preferably, not more than about ten-fold less activity, and most preferably, not more than about three-fold less activity relative to the GnTIII).

Antibody-dependent cell-mediated cytotoxicity (ADCC) is an immune mechanism leading to the lysis of antibody-coated target cells by immune effector cells. The target cells are cells to which antibodies or fragments thereof comprising an Fc region specifically bind, generally via the protein part that is N-terminal to the Fc region. As used herein, the term **"increased/reduced** ADCC" is defined as either an increase/reduction in the number of target cells that are lysed in a given time, at a given concentration of antibody in the medium surrounding the target cells, by the mechanism of ADCC defined above, and/or a reduction/increase in the concentration of antibody, in the medium surrounding the target cells, required to achieve the lysis of a given number of target cells in a given time, by the mechanism of ADCC. The increase/reduction in ADCC is relative to the ADCC mediated by the same antibody produced by the same type of host cells, using the same standard production, purification, formulation and storage methods (which are known to those skilled in the art), but that has not been engineered. For example, the increase in ADCC mediated by an antibody produced by host cells engineered to have an altered pattern of glycosylation (e.g. to express the glycosyltransferase, GnTIII, or other glycosyltransferases) by the methods described herein, is relative to the ADCC mediated by the same antibody produced by the same type of non-engineered host cells.

In certain aspects, contemplated is an antibody variant that possesses some but not all effector functions, which make it a desirable candidate for applications in which the half life of the antibody *in vivo* is important yet certain effector functions (such as complement-dependent cytotoxicity (CDC) and antibody-dependent cell-mediated cytotoxicity (ADCC)) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006); WO 2013/120929 A1).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581). Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).) In certain embodiments, an antibody variant comprises an Fc region with one or more amino acid substitutions which improve ADCC, e.g., substitutions at positions 298, 333, and/or 334 of the Fc region (EU numbering of residues).

In certain aspects, an antibody variant comprises an Fc region with one or more amino acid substitutions which diminish FcγR binding, e.g., substitutions at positions 234 and 235 of the Fc region (EU numbering of residues). In one aspect, the substitutions are L234A and L235A (LALA). In certain aspects, the antibody variant further comprises D265A and/or P329G in an Fc region derived from a human IgG1 Fc region. In one aspect, the substitutions are L234A, L235A and P329G (LALA-PG) in an Fc region derived from a human IgG1 Fc region. (See, e.g., WO 2012/130831). In another aspect, the substitutions are L234A, L235A and D265A (LALA-DA) in an Fc region derived from a human IgG1 Fc region.

In some embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934 (Hinton et al.). Those antibodies comprise an Fc region with one or more substitutions therein which improve binding of the Fc region to FcRn. Such Fc variants include those with substitutions at one or more of Fc region residues: 238, 252, 254, 256, 265, 272, 286, 303, 305, 307, 311, 312, 317, 340, 356, 360, 362, 376, 378, 380, 382, 413, 424 or 434, e.g., substitution of Fc region residue 434 (See, e.g., US Patent No. 7,371,826; Dall'Acqua, W.F., et al. J. Biol. Chem. 281 (2006) 23514-23524).

In certain aspects, an antibody variant comprises an Fc region with one or more amino acid substitutions, which reduce FcRn binding, e.g., substitutions at positions 253, and/or 310, and/or 435 of the Fc-region (EU numbering of residues). In certain aspects, the antibody variant comprises an Fc region with the amino acid substitutions at positions 253, 310 and 435. In one aspect, the substitutions are I253A, H310A and H435A in an Fc region derived from a human IgG1 Fc-region. See e.g., Grevys, A., et al., J. Immunol. 194 (2015) 5497-5508.

In another aspect, an antibody variant comprises an Fc region with one or more amino acid substitutions, which reduce FcRn binding, e.g., substitutions at positions 310, and/or 433, and/or 436 of the Fc region (EU numbering of residues). In certain aspects, the antibody variant comprises an Fc region with the amino acid substitutions at positions 310, 433 and 436. In one aspect, the substitutions are H310A, H433A and Y436A in an Fc region derived from a human IgG1 Fc-region. (See, e.g., WO 2014/177460 A1).

In certain aspects, an antibody variant comprises an Fc region with one or more amino acid substitutions which increase FcRn binding, e.g., substitutions at positions 252, and/or 254, and/or 256 of the Fc region (EU numbering of residues). In certain aspects, the antibody variant comprises an Fc region with amino acid substitutions at positions 252, 254, and 256. In one embodiment the substitutions are M252Y, S254T and T256E in an Fc region derived from a human IgG1 Fc-region (see also Duncan & Winter, Nature 322:738-40 (1988); U.S. Patent No. 5,648,260; U.S. Patent No. 5,624,821; and WO 94/29351 concerning other examples of Fc region variants.

In certain aspects, it may be desirable to create **cysteine engineered variants** of the antigen binding molecules, e.g., "thioMAbs," in which one or more residues of the molecule are substituted with cysteine residues. In particularaspects, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain aspects, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the antigen binding molecules provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the bispecific antibody derivative will be used in a therapy under defined conditions, etc. In another aspect, conjugates of an antibody and non-proteinaceous moiety that may be selectively heated by exposure to radiation are provided. In one aspect, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present disclosure. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts, as well as positive and negative strand forms, and double-stranded forms. Isolated polynucleotides or nucleic acids further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence can be determined conventionally using known computer programs, such as the ones discussed above for polypeptides (e.g. ALIGN-2).

The term "**expression cassette**" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette comprises polynucleotide sequences that encode bispecific antigen binding molecules or fragments thereof.

The term "**vector**" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector comprises an expression cassette that comprises polynucleotide sequences that encode bispecific antigen binding molecules or fragments thereof.

The terms "**host cell**", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "**effective amount**" of an agent refers to the amount that is necessary to result in a physiological change in the cell or tissue to which it is administered.

A "**therapeutically effective amount**" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "**individual**" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "**pharmaceutical composition**" refers to a preparation which is in such form as to permit the biological activity of an active ingredient contained therein to be effective, and which contains no additional components which are unacceptably toxic to a subject to which the formulation would be administered.

A "**pharmaceutically acceptable excipient**" refers to an ingredient in a pharmaceutical composition, other than an active ingredient, which is nontoxic to a subject. A pharmaceutically acceptable excipient includes, but is not limited to, a buffer, a stabilizer, or a preservative.

The term "**package insert**" is used to refer to instructions customarily included in commercial packages of therapeutic products, that contain information about the indications, usage, dosage, administration, combination therapy, contraindications and/or warnings concerning the use of such therapeutic products.

As used herein, "**treatment**" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the disclosure are used to delay development of a disease or to slow the progression of a disease.

The term "**cancer**" as used herein refers to proliferative diseases, such as lymphomas or lymphocytic leukemias, or melanoma.

By "**B cell proliferative disorder**" is meant a disease wherein the number of B cells in a patient is increased as compared to the number of B cells in a healthy subject, and particularly wherein the increase in the number of B cells is the cause or hallmark of the disease. A "CD20-positive B cell proliferative disorder" is a B cell proliferative disorder wherein B-cells, particularly malignant B-cells (in addition to normal B-cells), express CD20. Exemplary B cell proliferation disorders include Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), as well as some types of Multiple myeloma (MM) and Hodgkin lymphoma (HL).

**Exemplary 4-1BB agonists**

In particular, the 4-1BB agonists comprising one antigen binding domain capable of specific binding to CD19 as used in combination with the anti-CD20 antibody are molecules comprising 4-1BBL. In particular, the 4-1BB agonist used in the invention comprises three ectodomains of 4-1BBL or fragments thereof.

In a particular aspect, the 4-1BB agonist is a molecule comprising three ectodomains of 4-1BBL or fragments thereof and wherein the ectodomains of 4-1BBL comprise an amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO: 1 or SEQ ID NO:5.

It has been shown herein, that the 4-1BB agonist is especially useful if it comprises an antigen binding domain that is specific for a tumor target, in particular for a target on B cells. Thus, in another aspect, the 4-1BB agonist is an antigen binding molecule comprising three ectodomains of 4-1BBL or fragments thereof and at least one antigen binding domain capable of specific binding to CD19.

It has been further shown herein, that a 4-1BB agonist comprising at least one antigen binding domain capable of specific binding to CD19 was not internalized by CD19 into B cells and thus did not loss its ability to interact with the tumor microenvironment. In a further aspect, provided is a 4-1BB agonist that will not be internalized in B cells, thereby maintaining its activity.

In another aspect, the 4-1BB agonist is an antigen binding molecule comprising three ectodomains of 4-1BBL or fragments thereof and one moiety capable of specific binding to CD19, wherein the antigen binding domain capable of specific binding to CD19 is cyno-cross-reactive, i.e. the antigen binding domain capable of specific binding to CD19 specifically binds to human and to cynomolgus CD19.

In a further aspect, the 4-1BB agonist is an antigen binding molecule comprising three ectodomains of 4-1BBL or fragments thereof and at least one moiety capable of specific binding to CD19, wherein the antigen binding domain capable of specific binding to CD19 comprises (a) a heavy chain variable region ($V_H$CD19) comprising (i) CDR-H1 comprising the amino acid sequence of SEQ ID NO:9, (ii) CDR-H2 comprising the amino acid sequence of SEQ ID NO:10, and (iii) CDR-H3 comprising the amino acid sequence of SEQ ID NO:11, and a light chain variable region ($V_L$CD19) comprising (iv) CDR-L1 comprising the amino acid sequence of SEQ ID NO:12, (v) CDR-L2 comprising the amino acid sequence of SEQ ID NO:13, and (vi) CDR-L3 comprising the amino acid sequence of SEQ ID NO:14, or (b) a VH domain comprising (i) CDR-H1 comprising the amino acid sequence of SEQ ID NO:15, (ii) CDR-H2 comprising the amino acid sequence of SEQ ID NO:16, and (iii) CDR-H3 comprising the amino acid sequence of SEQ ID NO:17, and a VL domain comprising (iv) CDR-L1 comprising the amino acid sequence of SEQ ID NO:18, (v) CDR-L2 comprising the amino acid sequence of SEQ ID NO:19, and (vi) CDR-L3 comprising the amino acid sequence of SEQ ID NO:20.

In a particular aspect, the antigen binding domain capable of specific binding to CD19 comprises a VH domain comprising (i) CDR-H1 comprising the amino acid sequence of SEQ ID NO:15, (ii) CDR-H2 comprising the amino acid sequence of SEQ ID NO:16, and (iii) CDR-H3 comprising the amino acid sequence of SEQ ID NO:17, and a VL domain comprising (iv) CDR-L1 comprising the amino acid sequence of SEQ ID NO:18, (v) CDR-L2 comprising the amino acid sequence of SEQ ID NO:19, and (vi) CDR-L3 comprising the amino acid sequence of SEQ ID NO:20.

In a further aspect, the 4-1BB agonist is an antigen binding molecule comprising three ectodomains of 4-1BBL or fragments thereof and one antigen binding domain capable of specific binding to CD 19, wherein the antigen binding domain capable of specific binding to CD19 comprises a heavy chain variable region ($V_H$CD19) comprising an amino acid sequence of SEQ ID NO:21 and a light chain variable region ($V_L$CD19) comprising an amino acid sequence of SEQ ID NO:22 or wherein the antigen binding domain capable of specific binding to CD 19 comprises a heavy chain variable region ($V_H$CD19) comprising an amino acid sequence of SEQ ID NO:23 and a light chain variable region ($V_L$CD19) comprising an amino acid sequence of SEQ ID NO:24. More particularly, the antigen binding domain capable of specific binding to CD19 comprises a heavy chain variable region ($V_H$CD19) comprising an amino acid sequence of SEQ ID NO:23 and a light chain variable region ($V_L$CD19) comprising an amino acid sequence of SEQ ID NO:24.

In another aspect, the 4-1BB agonist is an antigen binding molecule further comprising a Fc domain composed of a first and a second subunit capable of stable association. In one aspect, the 4-1BB agonist is an antigen binding molecule comprising an IgG Fc domain, specifically an IgG1 Fc domain or an IgG4 Fc domain. Particularly, the 4-1BB agonist is an antigen binding molecule comprising a Fc domain that comprises one or more amino acid substitution that reduces binding to an Fc receptor and/or effector function. In a particular aspect, the 4-1BB agonist is an antigen binding molecule comprising an IgG1 Fc domain comprising the amino acid substitutions L234A, L235A and P329G.

In one aspect, the 4-1BB agonist is an antigen binding molecule comprising

(a) one antigen binding domain capable of specific binding to CD 19,

(b) a first and a second polypeptide that are linked to each other by a disulfide bond,

wherein the first polypeptide comprises two ectodomains of 4-1BBL or fragments thereof that are connected to each other by a peptide linker and in that the second polypeptide comprises one ectodomain of 4-1BBL or a fragment thereof.

In a particular aspect of the disclosure, the 4-1BB agonist is an antigen binding molecule comprising

(a) one Fab domain capable of specific binding to CD19, and

(b) a first and a second polypeptide that are linked to each other by a disulfide bond,

wherein the antigen binding molecule is characterized in that

(i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of 4-1BBL or fragments thereof that are connected to each other and to the CH1 or CL domain by a peptide linker and wherein the second polypeptide comprises one ectodomain of 4-1BBL or a fragment therof connected via a peptide linker to the CL or CH1 domain of said polypeptide, or

(ii) the first polypeptide contains a CH3 domain and the second polypeptide contains a CH3 domain, respectively, and wherein the first polypeptide comprises two ectodomains of 4-1BBL or fragments thereof that are connected to each other and to the C-terminus of the CH3 domain by a peptide linker and wherein the second polypeptide comprises one ectodomain of 4-1BBL or a fragment thereof connected via a peptide linker to the C-terminus of the CH3 domain of said polypeptide, or

(iii) the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of 4-1BBL or fragments thereof that are connected to each other and to VH or VL by a peptide linker and wherein the second polypeptide comprises one ectodomain of 4-1BBL or a fragment thereof connected via a peptide linker to VL or VH of said polypeptide.

In another aspect, the 4-1BB agonist is an antigen binding molecule comprising

(a) at least one Fab domain capable of specific binding to CD19 comprising a heavy chain variable region ($V_H$CD19) comprising the amino acid sequence of SEQ ID NO:21 and a light chain variable region ($V_L$CD19) comprising the amino acid sequence of SEQ ID NO:22 or a heavy chain variable region ($V_H$CD19) comprising the amino acid sequence of SEQ ID NO:23 and a light chain variable region ($V_L$CD19) comprising the amino acid sequence of SEQ ID NO:24, and

(b) a first and a second polypeptide that are linked to each other by a disulfide bond,

wherein the antigen binding molecule is characterized in that the first polypeptide comprises the amino acid sequence selected from the group consisting of SEQ ID NO:25, SEQ ID NO:26, SEQ ID NO:27, SEQ ID NO:28, SEQ ID NO:29, SEQ ID NO:30, SEQ ID NO:31 and SEQ ID NO:32 and in that the second polypeptide comprises the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

In a particular aspect of the disclosure, the 4-1BB agonist is an antigen binding molecule selected from the group consisting of

a) a molecule comprising a first heavy chain comprising the amino acid sequence of SEQ ID NO:33, a first light chain comprising the amino acid sequence of SEQ ID NO:34, a second heavy chain comprising the amino acid sequence of SEQ ID NO:35 and a second light chain comprising the amino acid sequence of SEQ ID NO:36;

b) a molecule comprising a first heavy chain comprising the amino acid sequence of SEQ ID NO:33, a first light chain comprising the amino acid sequence of SEQ ID NO:34, a second heavy chain comprising the amino acid sequence of SEQ ID NO:37 and a second light chain comprising the amino acid sequence of SEQ ID NO:38;

c) a molecule comprising two light chains comprising the amino acid sequence of SEQ ID NO:34, a first heavy chain comprising the amino acid sequence of SEQ ID NO:39 and a second heavy chain comprising the amino acid sequence of SEQ ID NO:40;

d) a molecule comprising a first heavy chain comprising the amino acid sequence of SEQ ID NO:33, a first light chain comprising the amino acid sequence of SEQ ID NO:34, a second heavy chain comprising the amino acid sequence of SEQ ID NO:41 and a second light chain comprising the amino acid sequence of SEQ ID NO:42;

e) a molecule comprising a first heavy chain comprising the amino acid sequence of SEQ ID NO:33, a first light chain comprising the amino acid sequence of SEQ ID NO:34, a second heavy chain comprising the amino acid sequence of SEQ ID NO:43 and a second light chain comprising the amino acid sequence of SEQ ID NO:44;

f) a molecule comprising two light chains comprising the amino acid sequence of SEQ ID NO:34, a first heavy chain comprising the amino acid sequence of SEQ ID NO:45 and a second heavy chain comprising the amino acid sequence of SEQ ID NO:46;

g) a molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:35 and a second light chain comprising the amino acid sequence of SEQ ID NO:36;

h) a molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:37 and a second light chain comprising the amino acid sequence of SEQ ID NO:38;

i) a molecule comprising two light chains comprising the amino acid sequence of SEQ ID NO:48, a first heavy chain comprising the amino acid sequence of SEQ ID NO:49 and a second heavy chain comprising the amino acid sequence of SEQ ID NO:50;

j) a molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:41 and a second light chain comprising the amino acid sequence of SEQ ID NO:42;

k) a molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:43 and a second light chain comprising the amino acid sequence of SEQ ID NO:44; and

l) a molecule comprising two light chains comprising the amino acid sequence of SEQ ID NO:48, a first heavy chain comprising the amino acid sequence of SEQ ID NO:51 and a second heavy chain comprising the amino acid sequence of SEQ ID NO:52.

In another aspect of the disclosure, the 4-1BB agonist is an antigen binding molecule further comprising a Fc domain composed of a first and a second subunit capable of stable association. In one aspect, the 4-1BB agonist is an antigen binding molecule comprising an IgG Fc domain, specifically an IgG1 Fc domain or an IgG4 Fc domain. Particularly, the 4-1BB agonist is an antigen binding molecule comprising a Fc domain that comprises one or more amino acid substitution that reduces binding to an Fc receptor and/or effector function. In a particular aspect, the 4-1BB agonist is an antigen binding molecule comprising an IgG1 Fc domain comprising the amino acid substitutions L234A, L235A and P329G.

**Fc domain modifications reducing Fc receptor binding and/or effector function**

The Fc domain of the antigen binding molecules consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

The Fc domain confers favorable pharmacokinetic properties to the antigen binding molecules, including a long serum half-life which contributes to good accumulation in the target tissue and a favorable tissue-blood distribution ratio. At the same time it may, however, lead to undesirable targeting of the bispecific antibodies to cells expressing Fc receptors rather than to the preferred antigen-bearing cells. Accordingly, in particular aspects, the Fc domain of the antigen binding molecules exhibits reduced binding affinity to an Fc receptor and/or reduced effector function, as compared to a native IgG1 Fc domain. In one aspect, the Fc does not substantially bind to an Fc receptor and/or does not induce effector function. In a particular aspect the Fc receptor is an Fcγ receptor. In one aspect, the Fc receptor is a human Fc receptor. In a specific aspect, the Fc receptor is an activating human Fcγ receptor, more specifically human FcγRIIIa, FcγRI or FcγRIIa, most specifically human FcγRIIIa. In one aspect, the Fc domain does not induce effector function. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

In certain aspects, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In a particular aspect, the Fc domain comprises one or more amino acid substitution that reduces binding to an Fc receptor, in particular towards Fcγ receptor.

In one aspect, the Fc domain of the antibody comprises one or more amino acid mutation that reduces the binding affinity of the Fc domain to an Fc receptor and/or effector function. Typically, the same one or more amino acid mutation is present in each of the two subunits of the Fc domain. In particular, the Fc domain comprises an amino acid substitution at a position of E233, L234, L235, N297, P331 and P329 (EU numbering). In particular, the Fc domain comprises amino acid substitutions at positions 234 and 235 (EU numbering) and/or 329 (EU numbering) of the IgG heavy chains. More particularly, provided is an antibody which comprises an Fc domain with the amino acid substitutions L234A, L235A and P329G ("P329G LALA", EU numbering) in the IgG heavy chains. The amino acid substitutions L234A and L235A refer to the so-called LALA mutation. The "P329G LALA" combination of amino acid substitutions almost completely abolishes Fcγ receptor binding of a human IgG1 Fc domain and is described in International Patent Appl. Publ. No. WO 2012/130831 A1 which also describes methods of preparing such mutant Fc domains and methods for determining its properties such as Fc receptor binding or effector functions.

Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In another aspect, the Fc domain is an IgG4 Fc domain. IgG4 antibodies exhibit reduced binding affinity to Fc receptors and reduced effector functions as compared to IgG1 antibodies. In a more specific aspect, the Fc domain is an IgG4 Fc domain comprising an amino acid substitution at position S228 (Kabat numbering), particularly the amino acid substitution S228P. In a more specific aspect, the Fc domain is an IgG4 Fc domain comprising amino acid substitutions L235E and S228P and P329G (EU numbering). Such IgG4 Fc domain mutants and their Fcγ receptor binding properties are also described in WO 2012/130831.

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. Alternatively, binding affinity of Fc domains or cell activating antibodies comprising an Fc domain for Fc receptors may be evaluated using cell lines known to express particular Fc receptors, such as human NK cells expressing FcγIIIa receptor.

Effector function of an Fc domain, or antibodies comprising an Fc domain, can be measured by methods known in the art. A suitable assay for measuring ADCC is described herein. Other examples of in vitro assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in an animal model such as that disclosed in Clynes et al., Proc Natl Acad Sci USA 95, 652-656 (1998).

In some aspects, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the bispecific antigen binding molecule is able to bind C1q and hence has CDC activity (see e.g., C1q and C3c binding ELISA in WO 2006/029879 and WO 2005/100402). To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

**Fc domain modifications promoting heterodimerization**

The bispecific antigen binding molecules comprise different antigen-binding sites, fused to one or the other of the two subunits of the Fc domain, thus the two subunits of the Fc domain may be comprised in two non-identical polypeptide chains. Recombinant co-expression of these polypeptides and subsequent dimerization leads to several possible combinations of the two polypeptides. To improve the yield and purity of the bispecific antibodies in recombinant production, it will thus be advantageous to introduce in the Fc domain of the bispecific antigen binding molecules a modification promoting the association of the desired polypeptides.

Accordingly, in particular aspects the invention relates to the bispecific antigen binding molecule comprising (a) one moiety capable of specific binding to CD19, (b) a first and a second polypeptide that are linked to each other by a disulfide bond, wherein the antigen binding molecule is characterized in that the first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other by a peptide linker and in that the second polypeptide comprises only one ectodomain of said 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8, and (c) a Fc domain composed of a first and a second subunit capable of stable association, wherein the Fc domain comprises a modification promoting the association of the first and second subunit of the Fc domain. The site of most extensive protein-protein interaction between the two subunits of a human IgG Fc domain is in the CH3 domain of the Fc domain. Thus, in one aspect said modification is in the CH3 domain of the Fc domain.

In a specific aspect said modification is a so-called "knob-into-hole" modification, comprising a "knob" modification in one of the two subunits of the Fc domain and a "hole" modification in the other one of the two subunits of the Fc domain. Thus, the invention relates to an antigen binding molecule comprising (a) one moiety capable of specific binding to a CD19, (b) a first and a second polypeptide that are linked to each other by a disulfide bond, wherein the antigen binding molecule is characterized in that the first polypeptide comprises two ectodomains of 4-1BBL 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8 that are connected to each other by a peptide linker and in that the second polypeptide comprises only one ectodomain of said 4-1BBL 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8, and (c) a Fc domain composed of a first and a second subunit capable of stable association, wherein the first subunit of the Fc domain comprises knobs and the second subunit of the Fc domain comprises holes according to the knobs into holes method. In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in one aspect, in the CH3 domain of the first subunit of the Fc domain of the bispecific antigen binding molecules an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable. The protuberance and cavity can be made by altering the nucleic acid encoding the polypeptides, e.g. by site-specific mutagenesis, or by peptide synthesis. In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one aspect, in the second subunit of the Fc domain additionally the threonine residue at position 366 is replaced with a serine residue (T366S) and the leucine residue at position 368 is replaced with an alanine residue (L368A).

In yet a further aspect, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in the formation of a disulfide bridge between the two subunits of the Fc domain that further stabilizes the dimer (Carter (2001), J Immunol Methods 248, 7-15). In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

The C-terminus of the heavy chain of the bispecific antibody as reported herein can be a complete C-terminus ending with the amino acid residues PGK. The C-terminus of the heavy chain can be a shortened C-terminus in which one or two of the C terminal amino acid residues have been removed. In one preferred aspect, the C-terminus of the heavy chain is a shortened C-terminus ending PG. In one aspect of all aspects as reported herein, a bispecific antibody comprising a heavy chain including a C-terminal CH3 domain as specified herein, comprises the C-terminal glycine-lysine dipeptide (G446 and K447, numbering according to Kabat EU index). In one aspect of all aspects as reported herein, a bispecific antibody comprising a heavy chain including a C-terminal CH3 domain, as specified herein, comprises a C-terminal glycine residue (G446, numbering according to Kabat EU index).

**Modifications in the Fab domains**

Multispecific antibodies with a domain replacement/exchange in one binding arm (CrossMabVH-VL or Cross-MabCH-CL) are described in detail in WO2009/080252 and Schaefer, W. et al, PNAS, 108 (2011) 11187-1191. They clearly reduce the byproducts caused by the mismatch of a light chain against a first antigen with the wrong heavy chain against the second antigen (compared to approaches without such domain exchange).

In one aspect, the invention relates to a bispecific antigen binding molecule comprising (a) a first Fab fragment capable of specific binding to CD 19, (b) a first and a second polypeptide that are linked to each other by a disulfide bond, wherein the antigen binding molecule is characterized in that the first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other by a peptide linker and in that the second polypeptide comprises only one ectodomain of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, and wherein each of them is linked to a CH1 or CL domain, and (c) a Fc domain composed of a first and a second subunit capable of stable association, wherein the constant domains CL and CH1 adjacent to 4-1BBL are replaced by each other so that the CH1 domain is part of the light chain and the CL domain is part of the heavy chain.

In another aspect, and to further improve correct pairing, the bispecific antigen binding molecule comprising (a) a first Fab fragment capable of specific binding to CD19, (b) a first and a second polypeptide that are linked to each other by a disulfide bond, wherein the antigen binding molecule is characterized in that the first polypeptide comprises two ectodomains of 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8that are connected to each other by a peptide linker and in that the second polypeptide comprises only one ectodomain of 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8, and wherein each of them is linked to a CH1 or CL domain, and (c) a Fc domain composed of a first and a second subunit capable of stable association, can contain different charged amino acid substitutions (so-called "charged residues"). These modifications are introduced in the crossed or non-crossed CH1 and CL domains. In a particular aspect, the disclosure relates to a bispecific antigen binding molecule, wherein in one of CL domains the amino acid at position 123 (EU numbering) has been replaced by arginine (R) and the amino acid at position 124 (EU numbering) has been substituted by lysine (K) and wherein in one of the CH1 domains the amino acids at position 147 (EU numbering) and at position 213 (EU numbering) have been substituted by glutamic acid (E).

More particularly, thedisclosure relates to a bispecific antigen binding molecule comprising a Fab, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

**Polynucleotides**

Further provided are isolated polynucleotides encoding a bispecific antibody as described herein or a fragment thereof.

The isolated polynucleotides encoding the antibodies of the disclosure may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes the entire antibody as described herein. In other embodiments, the isolated polynucleotide encodes a polypeptide comprised in the antibody as described herein.

In certain embodiments the polynucleotide or nucleic acid is DNA. In other embodiments, a polynucleotide is RNA, for example, in the form of messenger RNA (mRNA). RNA of the present invention may be single stranded or double stranded.

**Recombinant Methods**

Bispecific antibodies as used in the disclosure may be obtained, for example, by solid-state peptide synthesis (e.g. Merrifield solid phase synthesis) or recombinant production. For recombinant production one or more polynucleotide encoding the antibody or polypeptide fragments thereof, e.g., as described above, is isolated and inserted into one or more vectors for further cloning and/or expression in a host cell. Such polynucleotide may be readily isolated and sequenced using conventional procedures. In one aspect, a vector, preferably an expression vector, comprising one or more of the polynucleotides is provided. Methods which are well known to those skilled in the art can be used to construct expression vectors containing the coding sequence of the antibody (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the antibody or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector as disclosed herein may encode one or more polypeptides, which are post- or co-translationally separated into the final proteins via proteolytic cleavage. In addition, a vector, polynucleotide, or nucleic acid may encode heterologous coding regions, either fused or unfused to a polynucleotide encoding the antibody or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit $\alpha$-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions may be associated with additional coding regions which encode secretory or signal peptides, which direct the secretion of a polypeptide encoded by a polynucleotide as disclosed herein. For example, if secretion of the antibody or polypeptide fragments thereof is desired, DNA encoding a signal sequence may be placed upstream of the nucleic acid an antibody as disclosed herein or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding an antibody or polypeptide fragments thereof.

In a further aspect of the disclosure, a host cell comprising one or more polynucleotides is provided. In certain embodiments a host cell comprising one or more vectors is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) an antibody. As used herein, the term "host cell" refers to any kind of cellular system which can be engineered to generate the fusion proteins or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In certain aspects the moieties capable of specific binding to a target cell antigen (e.g. Fab fragments) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used. Non-limiting immunoglobulins useful can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins according to the disclosure are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the antibodies are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules disclosed herein to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain embodiments, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch. 14 (Cold Spring Harbor Laboratory, Cold Spring Harbor, NY).

Antibodies prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the antigen binding molecule binds. For example, for affinity chromatography purification of fusion proteins disclosed herein, a matrix with protein A or protein G may be used. Sequential Protein A or G affinity chromatography and size exclusion chromatography can be used to isolate an antigen binding molecule essentially as described in the Examples. The purity of the antigen binding molecule or fragments thereof can be determined by any of a variety of well-known analytical methods including gel electrophoresis, high pressure liquid chromatography, and the like. For example, the 4-1BBL-containing antigen binding molecules expressed as described in the Examples were shown to be intact and properly assembled as demonstrated by reducing and non-reducing SDS-PAGE.

**Assays**

The antigen binding molecules provided herein may be identified, screened for, or characterized for their physical/chemical properties and/or biological activities by various assays known in the art.

**1. Affinity assays**

The affinity of the bispecific antigen binding molecules provided herein for the corresponding receptor can be determined by surface plasmon resonance (SPR), using standard instrumentation such as a BIAcore instrument (GE Healthcare), and receptors or target proteins such as may be obtained by recombinant expression. The affinity of the bispecific antigen binding molecule for the target cell antigen can also be determined by surface plasmon resonance (SPR), using standard instrumentation such as a BIAcore instrument (GE Healthcare), and receptors or target proteins such as may be obtained by recombinant expression. For the CD19-4-1BBL antigen binding molecules the methods have been described in more detail in International Patent Appl. Publ. No. WO 2016/075278 A1. According to one aspect, $K_D$ is measured by surface plasmon resonance using a BIACORE® T100 machine (GE Healthcare) at 25 °C.

**2. Binding assays and other assays**

In one aspect, the CD19-4-1BBL antigen binding molecules as reported herein are tested for its antigen binding activity as described in more detail in Example 2.

**3. Activity assays**

In one aspect, assays are provided for identifying the biological activity of CD19-4-1BBL antigen binding molecules. Biological activity may include, e.g., inhibition of B-cell proliferation or killing of B-cells. Antibodies having such biological activity in vivo and/or in vitro are also provided. For the CD19-4-1BBL antigen binding molecules certain methods have been described in more detail in International Patent Appl. Publ. No. WO 2016/075278 A1.

In certain aspects, an antibody as reported herein is tested for such biological activity.

**Pharmaceutical Compositions, Formulations and Routes of Administation**

In a further aspect, provided are pharmaceutical compositions comprising the 4-1BB agonists comprising one antigen binding domain capable of specific binding to CD19, and the anti-CD20 antibodies provided herein, e.g., for use in any of the below therapeutic methods. In one aspect, the pharmaceutical composition comprises a 4-1BB agonist comprising one antigen binding domain capable of specific binding to CD19 provided herein and at least one pharmaceutically acceptable excipient, and an anti-CD20 antibody. In another aspect, a pharmaceutical composition comprises a 4-1BB agonist comprising one antigen binding domain capable of specific binding to CD19 and at least one pharmaceutically acceptable excipient, an anti-CD20 antibody and at least one additional therapeutic agent, e.g., as described below.

Pharmaceutical compositions of the present disclosure comprise a therapeutically effective amount of one or more antigen binding molecules described herein dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one antibody and optionally an additional active ingredient will be known to those of skill in the art in light of the present disclosure, as exemplified by Remington's Pharmaceutical Sciences, 18th Ed. Mack Printing Company, 1990. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the antigen binding molecules disclosed herein may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins disclosed herein in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the antibodies may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the antigen binding molecules of the disclosure may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The 4-1BB agonist comprising one antigen binding domain capable of specific binding to CD19 provided herein may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

In one aspect, there is provided a pharmaceutical composition comprising 4-1BB agonist comprising one antigen binding domain capable of specific binding to CD19 and a pharmaceutically acceptable excipient, and comprising an anti-CD20 antibody as described herein. In a particular aspect, the pharmaceutical composition is for use in the treatment of B-cell proliferative disorders, in particular a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).

The formulations to be used for in vivo administration are generally sterile. Sterility may be readily accomplished, e.g., by filtration through sterile filtration membranes.

**Administration of the 4-1BB agonist comprising one antigen binding domain capable of specific binding to CD19 provided herein and the anti-CD20 antibody**

Both the 4-1BB agonist comprising at least one antigen binding domain capable of specific binding to CD19 and the anti-CD20 antibody (both called substance herein) can be administered by any suitable means, including parenteral, intrapulmonary, and intranasal, and, if desired for local treatment, intralesional administration. The methods are particularly useful, however, in relation to therapeutic agents administered by parenteral, particularly intravenous, infusion.

Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. In one embodiment, the therapeutic agent is administered parenterally, particularly intravenously. In a particular aspect, the substance is administered by intravenous infusion. In another aspect, the substance is administered subcutaneously.

Both the 4-1BB agonist comprising one antigen binding domain capable of specific binding to CD 19 and the anti-CD20 antibody would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. Both the 4-1BB agonist comprising one antigen binding domain capable of specific binding to CD 19 and the anti-CD20 antibody need not be, but are optionally formulated with one or more agents currently used to prevent or treat the disorder in question. The effective amount of such other agents depends on the amount of therapeutic agent present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of 4-1BB agonist comprising one antigen binding domain capable of specific binding to CD19 provided herein and the anti-CD20 antibody (when used in their combination or with one or more other additional therapeutic agents) will depend on the type of disease to be treated, the type of 4-1BB agent, the severity and course of the disease, whether both agents are administered for preventive or therapeutic purposes, previous therapy, the patient's clinical history and response to the therapeutic agent, and the discretion of the attending physician. Each substance is suitably administered to the patient at one time or over a series of treatments. Depending on the type and severity of the disease, about 1 μg/kg to 15 mg/kg (e.g. 0.1 mg/kg - 10 mg/kg) of the substance can be an initial candidate dosage for administration to the subject, whether, for example, by separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of each substance would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the subject. Such doses may be administered intermittently, e.g. every week, every two weeks, or every three weeks (e.g. such that the subject receives from about two to about twenty, or e.g. about six doses of the therapeutic agent). An initial higher loading dose, followed by one or more lower doses, or an initial lower dose, followed by one or more higher doses may be administered. An exemplary dosing regimen comprises administering an initial dose of about 10 mg, followed by a bi-weekly dose of about 20 mg of the therapeutic agent. However, other dosage regimens may be useful. The progress of this therapy is easily monitored by conventional techniques and assays.

In one aspect, the administration of both the 4-1BB agonist comprising one antigen binding domain capable of specific binding to CD19 and the anti-CD20 antibody is a single administration. In certain aspects, the administration of the substance is two or more administrations. In one such aspect, the substances are administered every week, every two weeks, or every three weeks, particularly every two weeks. In one aspect, the substance is administered in a therapeutically effective amount. In one aspect the substance is administered at a dose of about 50 μg/kg, about 100 μg/kg, about 200 μg/kg, about 300 μg/kg, about 400 μg/kg, about 500 μg/kg, about 600 μg/kg, about 700 μg/kg, about 800 μg/kg, about 900 μg/kg or about 1000 μg/kg. In one embodiment, the 4-1BB agonist comprising one antigen binding domain capable of specific binding to CD19 is administered at a dose which is higher than the dose of the 4-1BB agonist comprising one antigen binding domain capable of specific binding to CD19 in a corresponding treatment regimen without the administration of the anti-CD20 antibody. In one aspect, the administration of the 4-1BB agonist comprising one antigen binding domain capable of specific binding to CD19 provided herein comprises an initial administration of a first dose of the anti-CD20 antibody, and one or more subsequent administrations of a second dose of the anti-CD20 antibody, wherein the second dose is higher than the first dose. In one aspect, the administration of the 4-1BB agonist comprising one antigen binding domain capable of specific binding to CD 19 comprises an initial administration of a first dose of the anti-CD20 antibody, and one or more subsequent administrations of a second dose of the anti-CD20 antibody, wherein the first dose is not lower than the second dose.

In one aspect, the administration of the 4-1BB agonist comprising one antigen binding domain capable of specific binding to CD19 in the treatment regimen according to the disclosure is the first administration of the 4-1BB agonist comprising one antigen binding domain capable of specific binding to CD19 to the subject (at least within the same course of treatment). In one aspect, no administration of the anti-CD20 antibody is made to the subject prior to the administration of the 4-1BB agonist comprising one antigen binding domain capable of specific binding to CD 19. In another aspect, the anti-CD20 antibody is administered prior to the administration of the 4-1BB agonist comprising one antigen binding domain capable of specific binding to CD 19.

In another aspect, the 4-1BB agonist comprising one antigen binding domain capable of specific binding to CD19, is for use in combination with an anti-CD20 antibody, wherein a pretreatment with an Type II anti-CD20 antibody, preferably obinutuzumab, is performed prior to the combination treatment, wherein the period of time between the pretreatment and the combination treatment is sufficient for the reduction of B-cells in the individual in response to the Type II anti-CD20 antibody, preferably obinutuzumab.

Activation of T cells can lead to severe cytokine release syndrome (CRS). In a phase 1 study conducted by TeGenero (Suntharalingam et al., N Engl J Med (2006) 355,1018-1028), all 6 healthy volunteers experienced near fatal, severe cytokine release syndrome (CRS) rapidly post-infusion of an inappropriately-dosed, T-cell stimulating super-agonist anti-CD28 monoclonal antibody. The cytokine release associated with administration of a T-cell activating therapeutic agent, such as the 4-1BB agonist comprising at least one antigen binding domain capable of specific binding to a tumor-associated antigen, in particular CD19, to a subject can be significantly reduced by pre-treatment of said subject with a Type II anti-CD20 antibody, such as obinutuzumab. the use of GAZYVA® pre-treatment (Gpt) should aid in the rapid depletion of B cells, both in the peripheral blood and in secondary lymphoid organs, such that the risk of highly relevant adverse events (AEs) from strong systemic T cell activation by T-cell activating therapeutic agents (e.g. CRS) is reduced, while supporting exposure levels of T-cell activating therapeutic agents that are high enough from the start of dosing to mediate tumour cell elimination. To date, the safety profile of obinutuzumab (including cytokine release) has been assessed and managed in hundreds of patients in ongoing obinutuzumab clinical trials. Finally, in addition to supporting the safety profile of 4-1BB agonist comprising one antigen binding domain capable of specific binding to CD19 provided herein, Gpt should also help prevent the formation of anti-drug antibodies (ADAs) to these unique molecules.

The combination of the 4-1BB agonist comprising one antigen binding domain capable of specific binding to CD19, and the anti-CD20 antibody can be used in combination with one or more further agents in a therapy. For instance, at least one additional therapeutic agent may be co-administered. In certain aspects, an additional therapeutic agent is an immunotherapeutic agent.

Such combination therapies noted above encompass combined administration (where two or more therapeutic agents are included in the same or separate formulations), and separate administration, in which case, administration of the therapeutic agent can occur prior to, simultaneously, and/or following, administration of an additional therapeutic agent or agents. In one embodiment, administration of the therapeutic agent and administration of an additional therapeutic agent occur within about one month, or within about one, two or three weeks, or within about one, two, three, four, five, or six days, of each other.

## Therapeutic compositions

CD20 and CD19 are expressed on most B-cells (pan-B-cell marker) with the exception of stem cells and plasma cells, and are frequently expressed on most human B-cell malignancies (tumor associated antigen), such as lymphoma and leukemias except for multiple myeloma, e.g. in non-Hodgkin lymphoma and acute lymphoblastic leukemia.

Bispecific molecules recognizing two cell surface proteins on different cell populations hold the promise to redirect cytotoxic immune cells for destruction of pathogenic target cells.

In further aspects, a composition for use in cancer immunotherapy is provided comprising a 4-1BB agonist comprising one antigen binding domain capable of specific binding to CD19 as provided herein and an anti-CD20 antibody. In certain aspects, a composition comprising a 4-1BB agonist comprising one antigen binding domain capable of specific binding to CD19 as provided herein and an anti-CD20 antibody for use in a method of cancer immunotherapy is provided.

In a further aspect, herein is provided the use of a composition comprising a 4-1BB agonist comprising one antigen binding domain capable of specific binding to CD19 provided herein and an anti-CD20 antibody in the manufacture or preparation of a medicament. In one aspect, the medicament is for treatment of a B-cell proliferative disorder. In a further aspect, the medicament is for use in a method of treating a B-cell proliferative disorder comprising administering to an individual having a B-cell proliferative disorder an effective amount of the medicament. In one such aspect, the method further comprises administering to the individual an effective amount of at least one additional therapeutic agent. In a further aspect, the medicament is for depleting B-cells. B-cell proliferative disorders are selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL). In one particular aspect, the B-cell cancer is non-Hodgkin lymphoma or acute lymphoblastic leukemia.

The combination therapies noted above encompass combined administration (where two or more therapeutic agents are included in the same or separate formulations), and separate administration, in which case, administration of the antibody as reported herein can occur prior to, simultaneously, and/or following, administration of the additional therapeutic agent or agents. In one embodiment, administration of the anti-human CD20 antibody and administration of an additional therapeutic agent occur within about one month, or within about one, two or three weeks, or within about one, two, three, four, five, or six days, of each other.

Both the 4-1BB agonist comprising one antigen binding domain capable of specific binding to CD 19 and the anti-CD20 antibody as reported herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Both the 4-1BB agonist comprising one antigen binding domain capable of specific binding to CD 19 and the anti-CD20 antibody as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibodies need not be, but are optionally formulated with one or more agents currently used to prevent or treat the disorder in question. The effective amount of such other agents depends on the amount of antibodies present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

**Other agents and treatments**

The antigen binding molecules may be administered in combination with one or more other agents in therapy. For instance, a fusion protein as described herein may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is another anti-cancer agent.

Such other agents are suitably present in combination in amounts that are effective for the purpose intended. The effective amount of such other agents depends on the amount of fusion protein used, the type of disorder or treatment, and other factors discussed above. The antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the antigen binding molecules disclosed hereincan occur prior to, simultaneously, and/or following, administration of the additional therapeutic agent and/or adjuvant.

**Articles of Manufacture (Kits)**

In another aspect, a kit containing materials useful for the treatment, prevention and/or diagnosis of the disorders described above is provided. The kit comprises at least one container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least two active agents in the kit are an anti-CD20/anti-CD3 bispecific antibody and a 4-1BB agonist comprising one antigen binding domain capable of specific binding to CD19.

In a particular aspect, provided is a kit for treating or delaying progression of cancer in a subject, comprising a package comprising (A) a first composition comprising as active ingredient a 4-1BB agonist comprising one antigen binding domain capable of specific binding to CD19 and a pharmaceutically acceptable excipient; (B) a second composition comprising as active ingredient an anti-CD20 antibody and a pharmaceutically acceptable excipient, and (C) instructions for using the compositions in a combination therapy.

The label or package insert indicates how the composition is used for treating the condition of choice and provides the instructions for using the compositions in a combination therapy. Moreover, the kit may comprise (a) a first container with a composition contained therein, wherein the composition comprises a 4-1BB agonist comprising one antigen binding domain capable of specific binding to CD19; and (b) a second container with a composition contained therein, wherein the composition comprises an anti-CD20 antibody. In addition, the kit may comprise one or more further containers comprising further active ingredients that can be used in combination. The article of manufacture may further comprise a package insert indicating that the compositions can be used to treat a particular condition.

Alternatively, or additionally, the kit may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table D (Sequences):**

| SEQ ID NO: | Name | Sequence |
|---|---|---|
| 1 | Human (hu) 4-1BBL (71-254) | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSE |

(continued)

| SEQ ID NO: | Name | Sequence |
|---|---|---|
| 2 | hu 4-1BBL (85-254) | LDLRQGMFAQLVAQNVLLIDGPLSWYSDPGLAGVSL TGGLSYKEDTKELVVAKAGVYYVFFQLELRRVVAG EGSGSVSLALHLQPLRSAAGAAALALTVDLPPASSEA RNSAFGFQGRLLHLSAGQRLGVHLHTEARARHAWQ LTQGATVLGLFRVTPEIPAGLPSPRSE |
| 3 | hu 4-1BBL (80-254) | DPAGLLDLRQGMFAQLVAQNVLLIDGPLSWYSDPGL AGVSLTGGLSYKEDTKELVVAKAGVYYVFFQLELRR VVAGEGSGSVSLALHLQPLRSAAGAAALALTVDLPP ASSEARNSAFGFQGRLLHLSAGQRLGVHLHTEARAR HAWQLTQGATVLGLFRVTPEIPAGLPSPRSE |
| 4 | hu 4-1BBL (52-254) | PWAVSGARASPGSAASPRLREGPELSPDDPAGLLDLR QGMFAQLVAQNVLLIDGPLSWYSDPGLAGVSLTGGL SYKEDTKELVVAKAGVYYVFFQLELRRVVAGEGSG SVSLALHLQPLRSAAGAAALALTVDLPPASSEARNSA FGFQGRLLHLSAGQRLGVHLHTEARARHAWQLTQG ATVLGLFRVTPEIPAGLPSPRSE |
| 5 | Human (hu) 4-1BBL (71-248) | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGL |
| 6 | hu 4-1BBL (85-248) | LDLRQGMFAQLVAQNVLLIDGPLSWYSDPGLAGVSL TGGLSYKEDTKELVVAKAGVYYVFFQLELRRVVAG EGSGSVSLALHLQPLRSAAGAAALALTVDLPPASSEA RNSAFGFQGRLLHLSAGQRLGVHLHTEARARHAWQ LTQGATVLGLFRVTPEIPAGL |
| 7 | hu 4-1BBL (80-248) | DPAGLLDLRQGMFAQLVAQNVLLIDGPLSWYSDPGL AGVSLTGGLSYKEDTKELVVAKAGVYYVFFQLELRR VVAGEGSGSVSLALHLQPLRSAAGAAALALTVDLPP ASSEARNSAFGFQGRLLHLSAGQRLGVHLHTEARAR HAWQLTQGATVLGLFRVTPEIPAGL |
| 8 | hu 4-1BBL (52-248) | PWAVSGARASPGSAASPRLREGPELSPDDPAGLLDLR QGMFAQLVAQNVLLIDGPLSWYSDPGLAGVSLTGGL SYKEDTKELVVAKAGVYYVFFQLELRRVVAGEGSG SVSLALHLQPLRSAAGAAALALTVDLPPASSEARNSA FGFQGRLLHLSAGQRLGVHLHTEARARHAWQLTQG ATVLGLFRVTPEIPAGL |
| 9 | CD19 (8B8-018) CDR-H1 | DYIMH |
| 10 | CD19 (8B8-018) CDR-H2 | YINPYNDGSKYTEKFQG |
| 11 | CD19 (8B8-018) CDR-H3 | GTYYYGSALFDY |
| 12 | CD19 (8B8-018) CDR-L1 | KSSQSLENPNGNTYLN |
| 13 | CD19 (8B8-018) CDR-L2 | RVSKRFS |
| 14 | CD19 (8B8-018) CDR-L3 | LQLTHVPYT |

(continued)

| SEQ ID NO: | Name | Sequence |
|---|---|---|
| 15 | CD19 (8B8-2B11) CDR-H1 | DYIMH |
| 16 | CD19 (8B8-2B11) CDR-H2 | YINPYNDGSKYTEKFQG |
| 17 | CD19 (8B8-2B11) CDR-H3 | GTYYYGPQLFDY |
| 18 | CD19 (8B8-2B11) CDR-L1 | KSSQSLETSTGTTYLN |
| 19 | CD19 (8B8-2B11) CDR-L2 | RVSKRFS |
| 20 | CD19 (8B8-2B11) CDR-L3 | LQLLEDPYT |
| 21 | CD19 (8B8-018) VH | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSS |
| 22 | CD19 (8B8-018) VL | DIVMTQTPLSLSVTPGQPASISCKSSQSLENPNGNTYL NWYLQKPGQSPQLLIYRVSKRFSGVPDRFSGSGSGTD FTLKISRVEAEDVGVYYCLQLTHVPYTFGQGTKLEIK |
| 23 | CD19 (8B8-2B11) VH | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSS |
| 24 | CD19 (8B8-2B11) VL | DIVMTQTPLSLSVTPGQPASISCKSSQSLETSTGTTYL NWYLQKPGQSPQLLIYRVSKRFSGVPDRFSGSGSGTD FTLKISRVEAEDVGVYYCLQLLEDPYTFGQGTKLEIK |
| 25 | dimeric hu 4-1BBL (71-254) connected by (G4S)<sub>2</sub> linker | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSREGPELSPDDPAGLLDLRQGM FAQLVAQNVLLIDGPLSWYSDPGLAGVSLTGGLSYK EDTKELVVAKAGVYYVFFQLELRRVVAGEGSGSVSL ALHLQPLRSAAGAAALALTVDLPPASSEARNSAFGF QGRLLHLSAGQRLGVHLHTEARARHAWQLTQGATV LGLFRVTPEIPAGLPSPRSE |
| 26 | dimeric hu 4-1BBL (85-254) connected by (G4S)<sub>2</sub> linker | LDLRQGMFAQLVAQNVLLIDGPLSWYSDPGLAGVSL TGGLSYKEDTKELVVAKAGVYYVFFQLELRRVVAG EGSGSVSLALHLQPLRSAAGAAALALTVDLPPASSEA RNSAFGFQGRLLHLSAGQRLGVHLHTEARARHAWQ LTQGATVLGLFRVTPEIPAGLPSPRSEGGGGSGGGGS LDLRQGMFAQLVAQNVLLIDGPLSWYSDPGLAGVSL TGGLSYKEDTKELVVAKAGVYYVFFQLELRRVVAG EGSGSVSLALHLQPLRSAAGAAALALTVDLPPASSEA RNSAFGFQGRLLHLSAGQRLGVHLHTEARARHAWQ LTQGATVLGLFRVTPEIPAGLPSPRSE |

(continued)

| SEQ ID NO: | Name | Sequence |
|---|---|---|
| 27 | dimeric hu 4-1BBL (80-254) connected by (G4S)₂ linker | DPAGLLDLRQGMFAQLVAQNVLLIDGPLSWYSDPGL AGVSLTGGLSYKEDTKELVVAKAGVYYVFFQLELRR VVAGEGSGSVSLALHLQPLRSAAGAAALALTVDLPP ASSEARNSAFGFQGRLLHLSAGQRLGVHLHTEARAR HAWQLTQGATVLGLFRVTPEIPAGLPSPRSEGGGGSG GGGSDPAGLLDLRQGMFAQLVAQNVLLIDGPLSWY SDPGLAGVSLTGGLSYKEDTKELVVAKAGVYYVFFQ LELRRVVAGEGSGSVSLALHLQPLRSAAGAAALALT VDLPPASSEARNSAFGFQGRLLHLSAGQRLGVHLHT EARARHAWQLTQGATVLGLFRVTPEIPAGLPSPRSE |
| 28 | dimeric hu 4-1BBL (52-254) connected by (G4S)₂ linker | PWAVSGARASPGSAASPRLREGPELSPDDPAGLLDLR QGMFAQLVAQNVLLIDGPLSWYSDPGLAGVSLTGGL SYKEDTKELVVAKAGVYYVFFQLELRRVVAGEGSG SVSLALHLQPLRSAAGAAALALTVDLPPASSEARNSA FGFQGRLLHLSAGQRLGVHLHTEARARHAWQLTQG ATVLGLFRVTPEIPAGLPSPRSEGGGGSGGGGSPWAV SGARASPGSAASPRLREGPELSPDDPAGLLDLRQGMF AQLVAQNVLLIDGPLSWYSDPGLAGVSLTGGLSYKE DTKELVVAKAGVYYVFFQLELRRVVAGEGSGSVSL ALHLQPLRSAAGAAALALTVDLPPASSEARNSAFGF QGRLLHLSAGQRLGVHLHTEARARHAWQLTQGATV LGLFRVTPEIPAGLPSPRSE |
| 29 | dimeric hu 4-1BBL (71-248) connected by (G4S)2 linker | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLG GGGSGGGGSREGPELSPDDPAGLLDLRQGMFAQLVA QNVLLIDGPLSWYSDPGLAGVSLTGGLSYKEDTKEL VVAKAGVYYVFFQLELRRVVAGEGSGSVSLALHLQP LRSAAGAAALALTVDLPPASSEARNSAFGFQGRLLH LSAGQRLGVHLHTEARARHAWQLTQGATVLGLFRV TPEIPAGL |
| 30 | dimeric hu 4-1BBL (85-248) connected by (G4S)2 linker | LDLRQGMFAQLVAQNVLLIDGPLSWYSDPGLAGVSL TGGLSYKEDTKELVVAKAGVYYVFFQLELRRVVAG EGSGSVSLALHLQPLRSAAGAAALALTVDLPPASSEA RNSAFGFQGRLLHLSAGQRLGVHLHTEARARHAWQ LTQGATVLGLFRVTPEIPAGLGGGGSGGGGSLDLRQ GMFAQLVAQNVLLIDGPLSWYSDPGLAGVSLTGGLS YKEDTKELVVAKAGVYYVFFQLELRRVVAGEGSGS VSLALHLQPLRSAAGAAALALTVDLPPASSEARNSAF GFQGRLLHLSAGQRLGVHLHTEARARHAWQLTQGA TVLGLFRVTPEIPAGL |

(continued)

| SEQ ID NO: | Name | Sequence |
|---|---|---|
| 31 | dimeric hu 4-1BBL (80-248) connected by (G4S)2 linker | DPAGLLDLRQGMFAQLVAQNVLLIDGPLSWYSDPGL AGVSLTGGLSYKEDTKELVVAKAGVYYVFFQLELRR VVAGEGSGSVSLALHLQPLRSAAGAAALALTVDLPP ASSEARNSAFGFQGRLLHLSAGQRLGVHLHTEARAR HAWQLTQGATVLGLFRVTPEIPAGLGGGGSGGGGSD PAGLLDLRQGMFAQLVAQNVLLIDGPLSWYSDPGLA GVSLTGGLSYKEDTKELVVAKAGVYYVFFQLELRRV VAGEGSGSVSLALHLQPLRSAAGAAALALTVDLPPA SSEARNSAFGFQGRLLHLSAGQRLGVHLHTEARARH AWQLTQGATVLGLFRVTPEIPAGL |
| 32 | dimeric hu 4-1BBL (52-248) connected by (G4S)2 linker | PWAVSGARASPGSAASPRLREGPELSPDDPAGLLDLR QGMFAQLVAQNVLLIDGPLSWYSDPGLAGVSLTGGL SYKEDTKELVVAKAGVYYVFFQLELRRVVAGEGSG SVSLALHLQPLRSAAGAAALALTVDLPPASSEARNSA FGFQGRLLHLSAGQRLGVHLHTEARARHAWQLTQG ATVLGLFRVTPEIPAGLGGGGSGGGGSPWAVSGARA SPGSAASPRLREGPELSPDDPAGLLDLRQGMFAQLV AQNVLLIDGPLSWYSDPGLAGVSLTGGLSYKEDTKE LVVAKAGVYYVFFQLELRRVVAGEGSGSVSLALHL QPLRSAAGAAALALTVDLPPASSEARNSAFGFQGRL LHLSAGQRLGVHLHTEARARHAWQLTQGATVLGLF RVTPEIPAGL |
| 33 | anti-CD19(8B8-018) Fc hole chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGK |
| 34 | anti-CD19(8B8-018) light chain | DIVMTQTPLSLSVTPGQPASISCKSSQSLENPNGNTYL NWYLQKPGQSPQLLIYRVSKRFSGVPDRFSGSGSGTD FTLKISRVEAEDVGVYYCLQLTHVPYTFGQGTKLEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC |

(continued)

| SEQ ID NO: | Name | Sequence |
|---|---|---|
| 35 | dimeric hu 4-1BBL (71-254)-CL* Fc knob chain | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSREGPELSPDDPAGLLDLRQGM FAQLVAQNVLLIDGPLSWYSDPGLAGVSLTGGLSYK EDTKELVVAKAGVYYVFFQLELRRVVAGEGSGSVSL ALHLQPLRSAAGAAALALTVDLPPASSEARNSAFGF QGRLLHLSAGQRLGVHLHTEARARHAWQLTQGATV LGLFRVTPEIPAGLPSPRSEGGGGSGGGGSRTVAAPS VFIFPPSDRKLKSGTASVVCLLNNFYPREAKVQWKV DNALQSGNSQESVTEQDSKDSTYSLSSTLTLSKADYE KHKVYACEVTHQGLSSPVTKSFNRGECDKTHTCPPC PAPEAAGGPSVFLFPPKPKDTLMISRTPEVTCVVVDV SHEDPEVKFNWYVDGVEVHNAKTKPREEQYNSTYR VVSVLTVLHQDWLNGKEYKCKVSNKALGAPIEKTIS KAKGQPREPQVYTLPPCRDELTKNQVSLWCLVKGFY PSDIAVEWESNGQPENNYKTTPPVLDSDGSFFLYSKL TVDKSRWQQGNVFSCSVMHEALHNHYTQKSLSLSP GK |
| 36 | monomeric hu 4-1BBL (71-254)-CH1* | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSASTKGPSVFPLAPSSKSTSGGT AALGCLVEDYFPEPVTVSWNSGALTSGVHTFPAVLQ SSGLYSLSSVVTVPSSSLGTQTYICNVNHKPSNTKVD EKVEPKSC |

(continued)

| SEQ ID NO: | Name | Sequence |
|---|---|---|
| 37 | dimeric hu 4-1BBL (71-254)-CL Fc knob chain | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSREGPELSPDDPAGLLDLRQGM FAQLVAQNVLLIDGPLSWYSDPGLAGVSLTGGLSYK EDTKELVVAKAGVYYVFFQLELRRVVAGEGSGSVSL ALHLQPLRSAAGAAALALTVDLPPASSEARNSAFGF QGRLLHLSAGQRLGVHLHTEARARHAWQLTQGATV LGLFRVTPEIPAGLPSPRSEGGGGSGGGGSRTVAAPS VFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGECDKTHTCPPC PAPEAAGGPSVFLFPPKPKDTLMISRTPEVTCVVVDV SHEDPEVKFNWYVDGVEVHNAKTKPREEQYNSTYR VVSVLTVLHQDWLNGKEYKCKVSNKALGAPIEKTIS KAKGQPREPQVYTLPPCRDELTKNQVSLWCLVKGFY PSDIAVEWESNGQPENNYKTTPPVLDSDGSFFLYSKL TVDKSRWQQGNVFSCSVMHEALHNHYTQKSLSLSP GK |
| 38 | monomeric hu 4-1BBL (71-254)-CH1 | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSC |

(continued)

| SEQ ID NO: | Name | Sequence |
|---|---|---|
| 39 | anti-CD19(8B8-018) Fc hole dimeric ligand chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSEGGG GSGGGGSREGPELSPDDPAGLLDLRQGMFAQLVAQN VLLIDGPLSWYSDPGLAGVSLTGGLSYKEDTKELVV AKAGVYYVFFQLELRRVVAGEGSGSVSLALHLQPLR SAAGAAALALTVDLPPASSEARNSAFGFQGRLLHLS AGQRLGVHLHTEARARHAWQLTQGATVLGLFRVTP EIPAGLPSPRSE |
| 40 | anti-CD19(8B8-018) Fc knob monomeric ligand | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSE |

(continued)

| SEQ ID NO: | Name | Sequence |
|---|---|---|
| 41 | dimeric hu 4-1BBL (71-248)-CL* Fc knob chain | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLG GGGSGGGGSREGPELSPDDPAGLLDLRQGMFAQLVA QNVLLIDGPLSWYSDPGLAGVSLTGGLSYKEDTKEL VVAKAGVYYVFFQLELRRVVAGEGSGSVSLALHLQP LRSAAGAAALALTVDLPPASSEARNSAFGFQGRLLH LSAGQRLGVHLHTEARARHAWQLTQGATVLGLFRV TPEIPAGLGGGGSGGGGSRTVAAPSVFIFPPSDRKLKS GTASVVCLLNNFYPREAKVQWKVDNALQSGNSQES VTEQDSKDSTYSLSSTLTLSKADYEKHKVYACEVTH QGLSSPVTKSFNRGECDKTHTCPPCPAPEAAGGPSVF LFPPKPKDTLMISRTPEVTCVVVDVSHEDPEVKFNW YVDGVEVHNAKTKPREEQYNSTYRVVSVLTVLHQD WLNGKEYKCKVSNKALGAPIEKTISKAKGQPREPQV YTLPPCRDELTKNQVSLWCLVKGFYPSDIAVEWESN GQPENNYKTTPPVLDSDGSFFLYSKLTVDKSRWQQG NVFSCSVMHEALHNHYTQKSLSLSPGK |
| 42 | monomeric hu 4-1BBL (71-248)-CH1 * | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLG GGGSGGGGSASTKGPSVFPLAPSSKSTSGGTAALGCL VEDYFPEPVTVSWNSGALTSGVHTFPAVLQSSGLYSL SSVVTVPSSSLGTQTYICNVNHKPSNTKVDEKVEPKS C |
| 43 | Dimeric hu 4-1BBL (71-248) - CL Fc knob chain | REGPELSPDDPAGLLDLRQGMFAQLVAQNVLLIDGP LSWYSDPGLAGVSLTGGLSYKEDTKELVVAKAGVY |

(continued)

| SEQ ID NO: | Name | Sequence |
|---|---|---|
| | | YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLG GGGSGGGGSREGPELSPDDPAGLLDLRQGMFAQLVA QNVLLIDGPLSWYSDPGLAGVSLTGGLSYKEDTKEL VVAKAGVYYVFFQLELRRVVAGEGSGSVSLALHLQP LRSAAGAAALALTVDLPPASSEARNSAFGFQGRLLH LSAGQRLGVHLHTEARARHAWQLTQGATVLGLFRV TPEIPAGLGGGGSGGGGSRTVAAPSVFIFPPSDEQLKS GTASVVCLLNNFYPREAKVQWKVDNALQSGNSQES VTEQDSKDSTYSLSSTLTLSKADYEKHKVYACEVTH QGLSSPVTKSFNRGECDKTHTCPPCPAPEAAGGPSVF LFPPKPKDTLMISRTPEVTCVVVDVSHEDPEVKFNW YVDGVEVHNAKTKPREEQYNSTYRVVSVLTVLHQD WLNGKEYKCKVSNKALGAPIEKTISKAKGQPREPQV YTLPPCRDELTKNQVSLWCLVKGFYPSDIAVEWESN GQPENNYKTTPPVLDSDGSFFLYSKLTVDKSRWQQG NVFSCSVMHEALHNHYTQKSLSLSPGK |
| 44 | Monomeric hu 4-1BBL (71-248) - CH1 | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLG GGGSGGGGSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKKVEPK SC |

(continued)

| SEQ ID NO: | Name | Sequence |
|---|---|---|
| 45 | anti-CD19(8B8-018) Fc hole dimeric ligand (71-248) chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLGGGGSGGG GSREGPELSPDDPAGLLDLRQGMFAQLVAQNVLLID GPLSWYSDPGLAGVSLTGGLSYKEDTKELVVAKAG VYYVFFQLELRRVVAGEGSGSVSLALHLQPLRSAAG AAALALTVDLPPASSEARNSAFGFQGRLLHLSAGQR LGVHLHTEARARHAWQLTQGATVLGLFRVTPEIPAG L |
| 46 | anti-CD19(8B8-018) Fc knob monomeric (71-248) ligand | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGL |

(continued)

| SEQ ID NO: | Name | Sequence |
|---|---|---|
| 47 | anti-CD19(8B8-2B11) Fc hole chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGK |
| 48 | CD19 (8B8-2B11) light chain | DIVMTQTPLSLSVTPGQPASISCKSSQSLETSTGTTYL NWYLQKPGQSPQLLIYRVSKRFSGVPDRFSGSGSGTD FTLKISRVEAEDVGVYYCLQLLEDPYTFGQGTKLEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC |
| 49 | anti-CD19(8B8-2B11) Fc hole dimeric ligand (71-254) chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSEGGG GSGGGGSREGPELSPDDPAGLLDLRQGMFAQLVAQN VLLIDGPLSWYSDPGLAGVSLTGGLSYKEDTKELVV AKAGVYYVFFQLELRRVVAGEGSGSVSLALHLQPLR SAAGAAALALTVDLPPASSEARNSAFGFQGRLLHLS AGQRLGVHLHTEARARHAWQLTQGATVLGLFRVTP EIPAGLPSPRSE |

(continued)

| SEQ ID NO: | Name | Sequence |
|---|---|---|
| 50 | anti-CD19(8B8-2B11) Fc knob monomeric (71-254) ligand | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSE |
| 51 | anti-CD19(8B8-2B11) Fc hole dimeric ligand (71-248) chain | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY KTTPPVLDSDGSFFLVSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLGGGGSGGG GSREGPELSPDDPAGLLDLRQGMFAQLVAQNVLLID GPLSWYSDPGLAGVSLTGGLSYKEDTKELVVAKAG VYYVFFQLELRRVVAGEGSGSVSLALHLQPLRSAAG AAALALTVDLPPASSEARNSAFGFQGRLLHLSAGQR LGVHLHTEARARHAWQLTQGATVLGLFRVTPEIPAG L |

(continued)

| SEQ ID NO: | Name | Sequence |
|---|---|---|
| 52 | anti-CD19(8B8-2B11) Fc knob monomeric (71-248) ligand | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGL |
| 53 | trimeric hu 4-1BBL (71-254) Fc knob chain | REGPELSPDDPAGLLDLRQGMFAQLVAQNVLLIDGP LSWYSDPGLAGVSLTGGLSYKEDTKELVVAKAGVY YVFFQLELRRVVAGEGSGSVSLALHLQPLRSAAGAA ALALTVDLPPASSEARNSAFGFQGRLLHLSAGQRLG VHLHTEARARHAWQLTQGATVLGLFRVTPEIPAGLP SPRSEGGGGSGGGGSREGPELSPDDPAGLLDLRQGM FAQLVAQNVLLIDGPLSWYSDPGLAGVSLTGGLSYK EDTKELVVAKAGVYYVFFQLELRRVVAGEGSGSVSL ALHLQPLRSAAGAAALALTVDLPPASSEARNSAFGF QGRLLHLSAGQRLGVHLHTEARARHAWQLTQGATV LGLFRVTPEIPAGLPSPRSEGGGGSGGGGSREGPELSP DDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSEGSPG SSSSGSDKTHTCPPCPAPEAAGGPSVFLFPPKPKDTL MISRTPEVTCVVVDVSHEDPEVKFNWYVDGVEVHN AKTKPREEQYNSTYRVVSVLTVLHQDWLNGKEYKC KVSNKALGAPIEKTISKAKGQPREPQVYTLPPCRDEL TKNQVSLWCLVKGFYPSDIAVEWESNGQPENNYKTT PPVLDSDGSFFLYSKLTVDKSRWQQGNVFSCSVMHE ALHNHYTQKSLSLSPGK |

(continued)

| SEQ ID NO: | Name | Sequence |
|---|---|---|
| 54 | anti- CD19(8B8-018) Fc knob chain fused to trimeric hu 4-1BBL (71-254) | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSEGGG GSGGGGSREGPELSPDDPAGLLDLRQGMFAQLVAQN VLLIDGPLSWYSDPGLAGVSLTGGLSYKEDTKELVV AKAGVYYVFFQLELRRVVAGEGSGSVSLALHLQPLR SAAGAAALALTVDLPPASSEARNSAFGFQGRLLHLS AGQRLGVHLHTEARARHAWQLTQGATVLGLFRVTP EIPAGLPSPRSEGGGGSGGGGSREGPELSPDDPAGLL DLRQGMFAQLVAQNVLLIDGPLSWYSDPGLAGVSLT GGLSYKEDTKELVVAKAGVYYVFFQLELRRVVAGE GSGSVSLALHLQPLRSAAGAAALALTVDLPPASSEAR NSAFGFQGRLLHLSAGQRLGVHLHTEARARHAWQL TQGATVLGLFRVTPEIPAGLPSPRSE |

(continued)

| SEQ ID NO: | Name | Sequence |
|---|---|---|
| 55 | anti- CD19(8B8-2B11) Fc knob chain fused to trimeric hu 4-1BBL (71-254) | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVYTLPPCR DELTKNQVSLWCLVKGFYPSDIAVEWESNGQPENNY KTTPPVLDSDGSFFLYSKLTVDKSRWQQGNVFSCSV MHEALHNHYTQKSLSLSPGGGGGSGGGGSREGPELS PDDPAGLLDLRQGMFAQLVAQNVLLIDGPLSWYSDP GLAGVSLTGGLSYKEDTKELVVAKAGVYYVFFQLEL RRVVAGEGSGSVSLALHLQPLRSAAGAAALALTVDL PPASSEARNSAFGFQGRLLHLSAGQRLGVHLHTEAR ARHAWQLTQGATVLGLFRVTPEIPAGLPSPRSEGGG GSGGGGSREGPELSPDDPAGLLDLRQGMFAQLVAQN VLLIDGPLSWYSDPGLAGVSLTGGLSYKEDTKELVV AKAGVYYVFFQLELRRVVAGEGSGSVSLALHLQPLR SAAGAAALALTVDLPPASSEARNSAFGFQGRLLHLS AGQRLGVHLHTEARARHAWQLTQGATVLGLFRVTP EIPAGLPSPRSEGGGGSGGGGSREGPELSPDDPAGLL DLRQGMFAQLVAQNVLLIDGPLSWYSDPGLAGVSLT GGLSYKEDTKELVVAKAGVYYVFFQLELRRVVAGE GSGSVSLALHLQPLRSAAGAAALALTVDLPPASSEAR NSAFGFQGRLLHLSAGQRLGVHLHTEARARHAWQL TQGATVLGLFRVTPEIPAGLPSPRSE |
| 56 | DP47 Fc hole chain | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCAKGSGFDYWG QGTLVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKKVEPK SCDKTHTCPPCPAPEAAGGPSVFLFPPKPKDTLMISRT PEVTCVVVDVSHEDPEVKFNWYVDGVEVHNAKTKP REEQYNSTYRVVSVLTVLHQDWLNGKEYKCKVSNK ALGAPIEKTISKAKGQPREPQVCTLPPSRDELTKNQV SLSCAVKGFYPSDIAVEWESNGQPENNYKTTPPVLDS DGSFFLVSKLTVDKSRWQQGNVFSCSVMHEALHNH YTQKSLSLSPGK |

(continued)

| SEQ ID NO: | Name | Sequence |
|---|---|---|
| 57 | DP47 light chain | EIVLTQSPGTLSLSPGERATLSCRASQSVSSSYLAWY QQKPGQAPRLLIYGASSRATGIPDRFSGSGSGTDFTLT ISRLEPEDFAVYYCQQYGSSPLTFGQGTKVEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC |
| 58 | DP47 Fc hole chain fused to dimeric hu 4-1BBL (71-254) | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSEGGGGSGGGG SREGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAGA AALALTVDLPPASSEARNSAFGFQGRLLHLSAGQRL GVHLHTEARARHAWQLTQGATVLGLFRVTPEIPAGL PSPRSE |
| 59 | DP47 Fc knob chain fused to monomeric hu 4-1BBL (71-254) | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVYTLPPCRDELTKN QVSLWCLVKGFYPSDIAVEWESNGQPENNYKTTPPV LDSDGSFFLYSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS |

(continued)

| SEQ ID NO: | Name | Sequence |
|---|---|---|
|  |  | LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSE |
| 60 | di-mu4-1BBL-CL Fc knob chain | see Table 5 |
| 61 | mono-mu4-1BBL-CH1 chain | see Table 5 |
| 62 | CD19 | UniProt accession No. P15391 |
| 63 | CD20 | UniProt accession No. P11836 |
| 64 | murine anti-CD20 B-Ly1 VH | GPELVKPGASVKISCKASGYAFSYSWMNWVKLRPG QGLEWIGRIFPGDGDTDYNGKFKGKATLTADKSSNT AYMQLTSLTSVDSAVYLCARNVFDGYWLVYWGQG TLVTVSA |
| 65 | murine anti-CD20 B-Lyl VL | NPVTLGTSASISCRSSKSLLHSNGITYLYWYLQKPGQ SPQLLIYQMSNLVSGVPDRFSSSGSGTDFTLRISRVEA EDVGVYYCAQNLELPYTFGGGTKLEIKR |
| 66 | full length 4-1BBL | UniProt No. P41273 |
| 67 | 4-1BBL (50-254) | ACPWAVSGARASPGSAASPRLREGPELSPDDPAGLL DLRQGMFAQLVAQNVLLIDGPLSWYSDPGLAGVSLT GGLSYKEDTKELVVAKAGVYYVFFQLELRRVVAGE GSGSVSLALHLQPLRSAAGAAALALTVDLPPASSEAR NSAFGFQGRLLHLSAGQRLGVHLHTEARARHAWQL TQGATVLGLFRVTPEIPAGLPSPRSE |
| 68 | human 4-1BB | UniProt accession No. Q07011 |
| 69 | murine 4-1BB | UniProt accession No. P20334 |
| 70 | cynomolgus 4-1BB | Uniprot accession No. F6W5G6 |
| 71 | 4-1BB (20H4.9) VH | QVQLQQWGAGLLKPSETLSLTCAVYGGSFSGYYWS WIRQSPEKGLEWIGEINHGGYVTYNPSLESRVTISVD TSKNQFSLKLSSVTAADTAVYYCARDYGPGNYDWY FDLWGRGTLVTVSS |
| 72 | 4-1BB (20H4.9) VL | EIVLTQSPATLSLSPGERATLSCRASQSVSSYLAWYQ QKPGQAPRLLIYDASNRATGIPARFSGSGSGTDFTLTI SSLEPEDFAVYYCQQRSNWPPALTFGGGTKVEIK |
| 73 | CD20-HCDR1 | YSWIN |
| 74 | CD20-HCDR2 | RIFPGDGDTDYNGKFK |
| 75 | CD20-HCDR3 | NVFDGYWLVY |
| 76 | CD20-LCDR1 | RS SKSLLHSNGITYLY |
| 77 | CD20-LCDR2 | QMSNLVS |
| 78 | CD20-LCDR3 | AQNLELPYT |

(continued)

| SEQ ID NO: | Name | Sequence |
|---|---|---|
| 79 | CD20 VH | QVQLVQSGAEVKKPGSSVKVSCKASGYAFSYSWIN WVRQAPGQGLEWMGRIFPGDGDTDYNGKFKGRVTI TADKSTSTAYMELSSLRSEDTAVYYCARNVFDGYW LVYWGQGTLVTVSS |
| 80 | CD20 VL | DIVMTQTPLSLPVTPGEPASISCRSSKSLLHSNGITYLY WYLQKPGQSPQLLIYQMSNLVSGVPDRFSGSGSGTD FTLKISRVEAEDVGVYYCAQNLELPYTFGGGTKVEIK RTV |
| 81 | G4S peptide linker | GGGGS |
| 82 | (G4S)2 | GGGGSGGGGS |
| 83 | (SG4)2 | SGGGGSGGGG |
| 84 | peptide linker | GGGGSGGGGSGGGG |
| 85 | peptide linker | GSPGSSSSGS |
| 86 | (G4S)$_3$ peptide linker | GGGGSGGGGSGGGGS$_3$ |
| 87 | (G4S)$_4$ peptide linker | GGGGSGGGGSGGGGSGGGGS |
| 88 | peptide linker | GSGSGSGS |
| 89 | peptide linker | GSGSGNGS |
| 90 | peptide linker | GGSGSGSG |
| 91 | peptide linker | GGSGSG |
| 92 | peptide linker | GGSG |
| 93 | peptide linker | GGSGNGSG |
| 94 | peptide linker | GGNGSGSG |
| 95 | peptide linker | GGNGSG |
| 96 | FAP (28H1) CDR-H1 | SHAMS |
| 97 | FAP (28H1) CDR-H2 | AIWASGEQYYADSVKG |
| 98 | FAP (28H1) CDR-H3 | GWLGNFDY |
| 99 | FAP (28H1) CDR-L1 | RASQSVSRSYLA |
| 100 | FAP (28H1) CDR-L2 | GASTRAT |
| 101 | FAP (28H1) CDR-L3 | QQGQVIPPT |
| 102 | FAP(4B9) CDR-H1 | SYAMS |
| 103 | FAP(4B9) CDR-H2 | AIIGSGASTYYADSVKG |
| 104 | FAP(4B9) CDR-H3 | GWFGGFNY |
| 105 | FAP(4B9) CDR-L1 | RASQSVTSSYLA |
| 106 | FAP(4B9) CDR-L2 | VGSRRAT |
| 107 | FAP(4B9) CDR-L3 | QQGIMLPPT |

(continued)

| SEQ ID NO: | Name | Sequence |
|---|---|---|
| 108 | FAP(28H1) VH | EVQLLESGGGLVQPGGSLRLSCAASGFTFSSHAMSW VRQAPGKGLEWVSAIWASGEQYYADSVKGRFTISRD NSKNTLYLQMNSLRAEDTAVYYCAKGWLGNFDYW GQGTLVTVSS |
| 109 | FAP(28H1) VL | EIVLTQSPGTLSLSPGERATLSCRASQSVSRSYLAWY QQKPGQAPRLLIIGASTRATGIPDRFSGSGSGTDFTLTI SRLEPEDFAVYYCQQGQVIPPTFGQGTKVEIK |
| 110 | FAP(4B9) VH | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSS |
| 111 | FAP(4B9) VL | EIVLTQSPGTLSLSPGERATLSCRASQSVTSSYLAWY QQKPGQAPRLLINVGSRRATGIPDRFSGSGSGTDFTL TISRLEPEDFAVYYCQQGIMLPPTFGQGTKVEIK |
| 112 | anti-FAP (4B9) Fc hole chain (Construct 2.4) | EVQLLESGGGLVQPGGSLRLSCAASGFTFSSYAMSW<br><br>VRQAPGKGLEWVSAIIGSGASTYYADSVKGRFTISRD NSKNTLYLQMNSLRAEDTAVYYCAKGWFGGFNYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGK |
| 113 | anti-FAP (4B9) light chain (Construct 2.4) | EIVLTQSPGTLSLSPGERATLSCRASQSVTSSYLAWY QQKPGQAPRLLINVGSRRATGIPDRFSGSGSGTDFTL TISRLEPEDFAVYYCQQGIMLPPTFGQGTKVEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |

(continued)

| SEQ ID NO: | Name | Sequence |
|---|---|---|
| 114 | anti-FAP(28H1) Fc hole chain | EVQLLESGGGLVQPGGSLRLSCAASGFTFSSHAMSW VRQAPGKGLEWVSAIWASGEQYYADSVKGRFTISRD NSKNTLYLQMNSLRAEDTAVYYCAKGWLGNFDYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGK |
| 115 | anti-FAP (28H1) light chain | EIVLTQSPGTLSLSPGERATLSCRASQSVSRSYLAWY QQKPGQAPRLLIIGASTRATGIPDRFSGSGSGTDFTLTI SRLEPEDFAVYYCQQGQVIPPTFGQGTKVEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| 116 | anti-FAP (4B9) Fc hole chain fused to dimeric hu 4-1BBL (71-254) | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSEGGGGSGGGG SREGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAGA AALALTVDLPPASSEARNSAFGFQGRLLHLSAGQRL GVHLHTEARARHAWQLTQGATVLGLFRVTPEIPAGL PSPRSE |

(continued)

| SEQ ID NO: | Name | Sequence |
|---|---|---|
| 117 | anti-FAP (4B9) Fc knob chain fused to monomeric hu 4-1BBL (71-254) | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVYTLPPCRDELTKN QVSLWCLVKGFYPSDIAVEWESNGQPENNYKTTPPV LDSDGSFFLYSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSE |
| 118 | anti-FAP (28H1) Fc hole chain fused to dimeric hu 4-1BBL (71-254) | EVQLLESGGGLVQPGGSLRLSCAASGFTFSSHAMSW VRQAPGKGLEWVSAIWASGEQYYADSVKGRFTISRD NSKNTLYLQMNSLRAEDTAVYYCAKGWLGNFDYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSEGGGGSGGGG SREGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAGA AALALTVDLPPASSEARNSAFGFQGRLLHLSAGQRL GVHLHTEARARHAWQLTQGATVLGLFRVTPEIPAGL PSPRSE |
| 119 | anti-FAP (28H1) Fc knob chain fused to monomeric hu 4-1BBL (71-254) | EVQLLESGGGLVQPGGSLRLSCAASGFTFSSHAMSW |

(continued)

| SEQ ID NO: | Name | Sequence |
|---|---|---|
| | | VRQAPGKGLEWVSAIWASGEQYYADSVKGRFTISRD NSKNTLYLQMNSLRAEDTAVYYCAKGWLGNFDYW GQGTLVTVSSASTKGPSVFPLAPSSKSTSGGTAALGC LVKDYFPEPVTVSWNSGALTSGVHTFPAVLQSSGLY SLSSVVTVPSSSLGTQTYICNVNHKPSNTKVDKKVEP KSCDKTHTCPPCPAPEAAGGPSVFLFPPKPKDTLMIS RTPEVTCVVVDVSHEDPEVKFNWYVDGVEVHNAKT KPREEQYNSTYRVVSVLTVLHQDWLNGKEYKCKVS NKALGAPIEKTISKAKGQPREPQVYTLPPCRDELTKN QVSLWCLVKGFYPSDIAVEWESNGQPENNYKTTPPV LDSDGSFFLYSKLTVDKSRWQQGNVFSCSVMHEALH NHYTQKSLSLSPGGGGGSGGGGSREGPELSPDDPAG LLDLRQGMFAQLVAQNVLLIDGPLSWYSDPGLAGVS LTGGLSYKEDTKELVVAKAGVYYVFFQLELRRVVA GEGSGSVSLALHLQPLRSAAGAAALALTVDLPPASSE ARNSAFGFQGRLLHLSAGQRLGVHLHTEARARHAW QLTQGATVLGLFRVTPEIPAGLPSPRSE |
| 120 | Human (hu) FAP | UniProt no. Q12884 |
| 121 | hu FAP ectodomain+poly-lys-tag+his$_6$-tag | RPSRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPVVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEEYPGRRNIYRISIGS YPPSKKCVTCHLRKERCQYYTASFSDYAKYYALVCY GPGIPISTLHDGRTDQEIKILEENKELENALKNIQLPKE EIKKLEVDEITLWYKMILPPQFDRSKKYPLLIQVYGG PCSQSVRSVFAVNWISYLASKEGMVIALVDGRGTAF QGDKLLYAVYRKLGVYEVEDQITAVRKFIEMGFIDE KRIAIWGWSYGGYVSSLALASGTGLFKCGIAVAPVSS WEYYASVYTERFMGLPTKDDNLEHYKNSTVMARAE YFRNVDYLLIHGTADDNVHFQNSAQIAKALVNAQV DFQAMWYSDQNHGLSGLSTNHLYTHMTHFLKQCFS LSDGKKKKKKGHHHHHH |
| 122 | mouse FAP | UniProt no. P97321 |

(continued)

| SEQ ID NO: | Name | Sequence |
|---|---|---|
| 123 | Murine FAP ectodomain+poly-lystag+his$_6$-tag | RPSRVYKPEGNTKRALTLKDILNGTFSYKTYFPNWIS EQEYLHQSEDDNIVFYNIETRESYIILSNSTMKSVNAT DYGLSPDRQFVYLESDYSKLWRYSYTATYYIYDLQN GEFVRGYELPRPIQYLCWSPVGSKLAYVYQNNIYLK QRPGDPPFQITYTGRENRIFNGIPDWVYEEEMLATKY ALWWSPDGKFLAYVEFNDSDIPIIAYSYYGDGQYPR TINIPYPKAGAKNPVVRVFIVDTTYPHHVGPMEVPVP EMIASSDYYFSWLTWVSSERVCLQWLKRVQNVSVL SICDFREDWHAWECPKNQEHVEESRTGWAGGFFVST PAFSQDATSYYKIFSDKDGYKHIHYIKDTVENAIQITS GKWEAIYIFRVTQDSLFYSSNEFEGYPGRRNIYRISIG

NSPPSKKCVTCHLRKERCQYYTASFSYKAKYYALVC YGPGLPISTLHDGRTDQEIQVLEENKELENSLRNIQLP KVEIKKLKDGGLTFWYKMILPPQFDRSKKYPLLIQVY GGPCSQSVKSVFAVNWITYLASKEGIVIALVDGRGTA FQGDKFLHAVYRKLGVYEVEDQLTAVRKFIEMGFID EERIAIWGWSYGGYVSSLALASGTGLFKCGIAVAPVS SWEYYASIYSERFMGLPTKDDNLEHYKNSTVMARA EYFRNVDYLLIHGTADDNVHFQNSAQIAKALVNAQV DFQAMWYSDQNHGILSGRSQNHLYTHMTHFLKQCF SLSDGKKKKKKGHHHHHH |
| 124 | Cynomolgus FAP ectodomain+poly-lys-tag+his$_6$-tag | RPPRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPFVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEDYPGRRNIYRISIG SYPPSKKCVTCHLRKERCQYYTASFSDYAKYYALVC YGPGIPISTLHDGRTDQEIKILEENKELENALKNIQLP KEEIKKLEVDEITLWYKMILPPQFDRSKKYPLLIQVY GGPCSQSVRSVFAVNWISYLASKEGMVIALVDGRGT AFQGDKLLYAVYRKLGVYEVEDQITAVRKFIEMGFI DEKRIAIWGWSYGGYVSSLALASGTGLFKCGIAVAP VSSWEYYASVYTERFMGLPTKDDNLEHYKNSTVMA RAEYFRNVDYLLIHGTADDNVHFQNSAQIAKALVNA QVDFQAMWYSDQNHGLSGLSTNHLYTHMTHFLKQ CFSLSDGKKKKKKGHHHHHH |

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the numbering systems according to Kabat (Kabat, E.A., et al., Sequences of Proteins of Immunological Interest, 5th ed., Public Health Service, National Institutes of Health, Bethesda, MD (1991)) as defined above.

## EXAMPLES

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM $KH_2PO_4/K_2HPO_4$, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

**Determination of binding and binding affinity of multispecific antibodies to the respective antigens using surface plasmon resonance (SPR) (BIACORE)**

Binding of the generated antibodies to the respective antigens is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C). Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

**Example 1**

**Preparation, purification and characterization of 4-1BBL antigen binding molecules comprising at least one antigen binding domain capable of specific binding to a tumor-associated antigen**

CD19-targeted 4-1BB ligand trimer-containing Fc fusion antigen binding molecules were prepared as described in International Patent Appl. Publ. No. WO 2016/075278 A1.

In particular, the following molecules were made:

a) Monovalent CD19-targeted and untargeted 4-1BB ligand trimer-containing Fc fusion antigen binding molecules

A DNA sequence encoding a dimeric 4-1BB ligand fused to human CL domain was subcloned in frame with the human IgG1 heavy chain CH2 and CH3 domains on the knob (Merchant, Zhu et al. 1998). A polypeptide containing one ectodomain of the 4-1BB ligand was fused to the human IgG1-CH1 domain. In Construct 3.4, in order to improve correct pairing the following mutations were additionally introduced in the crossed CH-CL (charged variant). In the dimeric 4-1BB ligand fused to human CL, E123R and Q124K, in the monomeric 4-1BB ligand fused to human CH1, K147E and K213E.

The variable region of heavy and light chain DNA sequences encoding a binder specific for CD19, clone 8B8-018 or clone 8B8-2B11, were subcloned in frame with either the constant heavy chain of the hole or the constant light chain of human IgG1. The Pro329Gly, Leu234Ala and Leu235Ala mutations have been introduced in the constant region of the knob and hole heavy chains to abrogate binding to Fc gamma receptors according to the method described in WO 2012/130831. Combination of the dimeric ligand-Fc knob chain containing the S354C/T366W mutations, the monomeric CH1 fusion, the targeted anti-CD19-Fc hole chain containing the Y349C/T366S/L368A/Y407V mutations and the anti-CD19 light chain allows generation of a heterodimer, which includes an assembled trimeric 4-1BB ligand and a CD19 binding Fab (**Figures 1A and 1B**). An untargeted version has been prepared accordingly by replacing the CD19 binder by germline DP47 (**Figure 1C**).

**Table 1: Monovalent CD19-4-1BBL Constructs used in the experiments**

| | Example in WO 2016/075278 | composed of |
|---|---|---|
| mono CD19(018)-4-1BBL (Charged variant) | Example 7.1.6 (Construct 3.4) | SEQ ID NO:33, SEQ ID NO:34 SEQ ID NO:41 and SEQ ID NO:42 |
| mono CD19(2B11)-4-1BBL (Charged variant) | Example 7.2.6 (Construct 4.4) | SEQ ID NO:47, SEQ ID NO:48 SEQ ID NO:41 and SEQ ID NO:42 |
| mono CD19(2B11)-4-1BBL | Example 7.2.7 (Construct 4.5) | SEQ ID NO:47, SEQ ID NO:48 SEQ ID NO:43 and SEQ ID NO:44 |
| mono untargeted DP47-4-1BBL | Example 7.3.12 (Control D) | SEQ ID NO:56, SEQ ID NO:57 SEQ ID NO:43 and SEQ ID NO:44 |

b) Bivalent CD19-targeted and untargeted 4-1BB ligand trimer-containing Fc fusion antigen binding molecules

The DNA sequences encoding the heavy and light chain variable regions of heavy and light chain specific a binder specific for CD19, clone 8B8-018 or clone 8B8-2B11, were subcloned in frame with either the constant heavy chain of the hole, the knob or the constant light chain of human IgG1. The Pro329Gly, Leu234Ala and Leu235Ala mutations were introduced in the constant region of the knob and hole heavy chains to abrogate binding to Fc gamma receptors according to the method described in WO 2012/130831. Furthermore, a polypeptide comprising two ectodomains of 4-1BB ligand was fused to the C-terminus of human IgG1 Fc hole chain and a polypeptide comprising one ectodomain of 4-1BB ligand was fused to the C-terminus of human IgG1 Fc knob chain. Combination of the anti-CD19 huIgG1 hole dimeric ligand heavy chain containing the Y349C/T366S/L368A/Y407V mutations, the anti-CD19 huIgG1 knob monomeric ligand heavy chain containing the S354C/T366W mutations and the anti-CD19 light chains allowed generation of a heterodimer, which included an assembled trimeric 4-1BB ligand and two CD19 binding Fabs (**Figure 1D**). An untargeted version has been prepared accordingly by replacing the CD19 binder by germline DP47 (**Figure 1E**).

**Table 2: Bivalent CD19-4-1BBL Constructs used in the experiments**

|  | Example in WO 2016/075278 | composed of |
|---|---|---|
| bi CD19(018)-4-1BBL | Example 7.1.8 (Construct 3.6) | 2 x SEQ ID NO:34, SEQ ID NO:45 and SEQ ID NO: 46 |
| bi CD19(2B11)-4-1BBL | Example 7.2.8 (Construct 4.6) | 2 x SEQ ID NO:48 SEQ ID NO:51 and SEQ ID NO: 52 |
| bi untargeted DP47-4-1BBL | Example 7.3.12 (Control C) | 2 x SEQ ID NO:57 SEQ ID NO:58 and SEQ ID NO:59 |

The production and characterization of the CD19-targeted and untargeted 4-1BB ligand trimer-containing Fc fusion antigen binding molecules is described in detail in WO 2016/075278, Example 7.4 and Examples 8 to 11, respectively.

FAP-targeted 4-1BB ligand trimer-containing Fc fusion antigen binding molecules were also prepared as described in International Patent Appl. Publ. No. WO 2016/075278 A1.

In particular, the following molecules were made:

c) Monovalent FAP-targeted 4-1BB ligand trimer-containing Fc fusion antigen binding molecules

The variable region of heavy and light chain DNA sequences encoding a binder specific for FAP, clone 28H1 or clone 4B9, were subcloned in frame with either the constant heavy chain of the hole or the constant light chain of human IgG1. The Pro329Gly, Leu234Ala and Leu235Ala mutations have been introduced in the constant region of the knob and hole heavy chains to abrogate binding to Fc gamma receptors according to the method described in WO 2012/130831. Combination of the dimeric ligand-Fc knob chain containing the S354C/T366W mutations, the monomeric CH1 fusion, the targeted anti-FAP-Fc hole chain containing the Y349C/T366S/L368A/Y407V mutations and the anti-FAP light chain allows generation of a heterodimer, which includes an assembled trimeric 4-1BB ligand and a FAP binding Fab (in analogy to Fig. 1A).

**Table 3: Monovalent FAP-4-1BBL Constructs**

|  | Example in WO 2016/075278 | composed of |
|---|---|---|
| mono FAP(4B9)-4-1BBL (Charged variant) | Example 2.1.4 (Construct 2.4) | SEQ ID NO: 112, SEQ ID NO:113 SEQ ID NO:41 and SEQ ID NO:42 |
| mono FAP(28H1)-4-1BBL | Example 1.1 (Construct 1.2) | SEQ ID NO: 114, SEQ ID NO: 115 SEQ ID NO:43 and SEQ ID NO:44 |

d) Bivalent FAP-targeted 4-1BB ligand trimer-containing Fc fusion antigen binding molecules

The DNA sequences encoding the heavy and light chain variable regions of heavy and light chain specific a binder specific for FAP, clone 28H1 or clone 4B9, were subcloned in frame with either the constant heavy chain of the hole, the knob or the constant light chain of human IgG1. The Pro329Gly, Leu234Ala and Leu235Ala mutations were introduced in the constant region of the knob and hole heavy chains to abrogate binding to Fc gamma receptors according to the method described in WO 2012/130831. Furthermore, a polypeptide comprising two ectodomains of 4-1BB ligand was fused to the C-terminus of human IgG1 Fc hole chain and a polypeptide comprising one ectodomain of 4-1BB ligand was fused to the C-terminus of human IgG1 Fc knob chain. Combination of the anti-FAP huIgG1 hole dimeric ligand heavy chain containing the Y349C/T366S/L368A/Y407V mutations, the anti-FAP huIgG1 knob monomeric ligand heavy chain containing the S354C/T366W mutations and the anti-FAP light chains allowed generation of a heterodimer, which included an assembled trimeric 4-1BB ligand and two FAP binding Fabs (**Figure 1D**).

**Table 4: Bivalent FAP-4-1BBL Constructs**

|  | Example in WO 2016/075278 | composed of |
|---|---|---|
| bi FAP(4B9)-4-1BBL | Example 2.1.3 (Construct 2.3) | 2 x SEQ ID NO:113, SEQ ID NO: 116 and SEQ ID NO: 117 |
| bi FAP(28H1)-4-1BBL | Example 1.1 (Construct 1.5) | 2 x SEQ ID NO:115<br>SEQ ID NO: 118 and SEQ ID NO:119 |

The production and characterization of the FAP-targeted 4-1BB ligand trimer-containing Fc fusion antigen binding molecules is described in detail in WO 2016/075278, Examples 1 to 6, respectively.

**Example 2**

**Preparation, purification and characterization of monovalent CD19-mu4-1BBL antigen binding molecule (hybrid surrogate)**

A CD19-targeted murine 4-1BB ligand trimer-containing Fc fusion antigen binding molecule was prepared as described in International Patent Appl. Publ. No. WO 2016/075278 A1. The DNA sequence encoding part of the ectodomain (amino acid 104-309, including the C160S mutation) of mouse 4-1BB ligand was synthetized according to the Q3U1Z9-1 sequence of Uniprot database.

A DNA sequence encoding the dimeric murine 4-1BB ligand fused to human CL domain was subcloned in frame with the human IgG1 heavy chain CH2 and CH3 domains on the knob (Merchant, Zhu et al. 1998). A polypeptide containing one ectodomain of the murine 4-1BB ligand was fused to the human IgG1-CH1 domain. The variable region of heavy and light chain DNA sequences encoding a binder specific for CD19, clone 8B8-018, were subcloned in frame with either the constant heavy chain of the hole or the constant light chain of human IgG1. The Pro329Gly, Leu234Ala and Leu235Ala mutations have been introduced in the constant region of the knob and hole heavy chains to abrogate binding to Fc gamma receptors according to the method described in WO 2012/130831. Combination of the dimeric ligand-Fc knob chain containing the S354C/T366W mutations, the monomeric CH1 fusion, the targeted anti-CD19-Fc hole chain containing the Y349C/T366S/L368A/Y407V mutations and the anti-CD19 light chain allows generation of a heterodimer, which includes an assembled trimeric murine 4-1BB ligand and a CD19 binding Fab (**Figure 1F**).

The amino acid sequences for the hybrid surrogate CD19-mu4-1BBL can be found in **Table 5.**

**Table 5: Amino acid sequences of mature hybrid surrogate CD19-mu4-1BBL**

| SEQ ID NO: | Description | Sequence |
|---|---|---|
| 60 | di-mu4-1BBL-CL Fc knob chain | RTEPRPALTITTSPNLGTRENNADQVTPVSHIGCPNTTQQGS PVFAKLLAKNQASLSNTTLNWHSQDGAGSSYLSQGLRYEE DKKELVVDSPGLYYVFLELKLSPTFTNTGHKVQGWVSLVL QAKPQVDDFDNLALTVELFPCSMENKLVDRSWSQLLLLK AGHRLSVGLRAYLHGAQDAYRDWELSYPNTTSFGLFLVK PDNPWEGGGGSGGGGSRTEPRPALTITTSPNLGTRENNAD QVTPVSHIGCPNTTQQGSPVFAKLLAKNQASLSNTTLNWH SQDGAGSSYLSQGLRYEEDKKELVVDSPGLYYVFLELKLS PTFTNTGHKVQGWVSLVLQAKPQVDDFDNLALTVELFPCS MENKLVDRSWSQLLLLKAGHRLSVGLRAYLHGAQDAYR DWELSYPNTTSFGLFLVKPDNPWEGGGGSGGGGSRTVAA PSVFIFPPSDRKLKSGTASVVCLLNNFYPREAKVQWKVDN ALQSGNSQESVTEQDSKDSTYSLSSTLTLSKADYEKHKVY ACEVTHQGLSSPVTKSFNRGECDKTHTCPPCPAPEAAGGPS VFLFPPKPKDTLMISRTPEVTCVVVDVSHEDPEVKFNWYV DGVEVHNAKTKPREEQYNSTYRVVSVLTVLHQDWLNGKE YKCKVSNKALGAPIEKTISKAKGQPREPQVYTLPPCRDELT KNQVSLWCLVKGFYPSDIAVEWESNGQPENNYKTTPPVLD SDGSFFLYSKLTVDKSRWQQGNVFSCSVMHEALHNHYTQ KSLSLSPGK |
| 61 | mono-mu4-1BBL-CH1 chain | RTEPRPALTITTSPNLGTRENNADQVTPVSHIGCPNTTQQGS PVFAKLLAKNQASLSNTTLNWHSQDGAGSSYLSQGLRYEE DKKELVVDSPGLYYVFLELKLSPTFTNTGHKVQGWVSLVL QAKPQVDDFDNLALTVELFPCSMENKLVDRSWSQLLLLK AGHRLSVGLRAYLHGAQDAYRDWELSYPNTTSFGLFLVK PDNPWEGGGGSGGGGSASTKGPSVFPLAPSSKSTSGGTAA LGCLVEDYFPEPVTVSWNSGALTSGVHTFPAVLQSSGLYS LSSVVTVPSSSLGTQTYICNVNHKPSNTKVDEKVEPKSC |
| 33 | anti-CD19(8B8-018) Fc hole chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGK |
| 34 | anti-CD19(8B8-018) light chain | DIVMTQTPLSLSVTPGQPASISCKSSQSLENPNGNTYLNWY LQKPGQSPQLLIYRVSKRFSGVPDRFSGSGSGTDFTLKISRV EAEDVGVYYCLQLTHVPYTFGQGTKLEIKRTVAAPSVFIFP PSDEQLKSGTASVVCLLNNFYPREAKVQWKVDNALQSGN SQESVTEQDSKDSTYSLSSTLTLSKADYEKHKVYACEVTH QGLSSPVTKSFNRGEC |

The hybrid surrogate CD19-mu4-1BBL was produced by co-transfecting CHO-K1 cells growing in suspension with the mammalian expression vectors using eviFect (Evitria AG). The cells were transfected with the corresponding expression vectors in a 1:1:1:1 ratio ("vector Fc-hole heavy chain": "vector CD19 light chain": "vector 4-1BBL Fc-knob heavy chain": "vector mu4-1BBL light chain"). For transfection CHO-K1 cells are cultivated in suspension serum free in eviMake (Evitria AG) culture medium. After 7 days at 37 °C in an incubator with a 5 % $CO_2$ atmosphere, cultivation supernatant is collected for purification by centrifugation and the solution is sterile filtered (0.22 mm filter) and kept at 4°C. Secreted proteins were purified from cell culture supernatants by affinity chromatography using Protein A, followed by size exclusion chromatography. For affinity chromatography, the supernatant was loaded on a Protein A MabSelect-Sure column (GE Healthcare) equilibrated with 20 mM sodium phosphate, 20 mM sodium citrate pH 7.5. Unbound protein was removed by washing with 20 mM sodium phosphate, 20 mM sodium citrate containing buffer (pH 7.5). The bound protein was eluted using a linear pH-gradient of sodium chloride of 20 mM sodium citrate, 100 mM NaCl, 100 mM Glycine, 0.01% Tween20 pH 3.0. The column was then washed with 20 mM sodium citrate, 100 mM NaCl, 100 mM glycine, 0.01% Tween20 pH 3.0. The pH of collected fractions was adjusted by adding 1/40 (v/v) of 2M Tris, pH8.0. The protein was concentrated and filtered prior to loading on a HiLoad Superdex column (GE Healthcare) equilibrated with 20 mM Histidine, 140 mM NaCl, 0.01% Tween20 pH6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM $K_2HPO_4$, 125 mM NaCl, 200mM L-arginine monohydrocloride, 0.02 % (w/v) $NaN_3$, pH 6.7 running buffer at 25°C.

**Table 6:** Biochemical analysis of hybrid surrogate CD19-mu4-1BBL

| Molecule | Monomer [%] | Yield [mg/l] | CE-SDS (non-red) |
|---|---|---|---|
| CD19-mu4-1BBL | 99 | 3.5 | 92 |

Functional characterization of hybrid surrogate CD19-mu4-1BBL by surface plasmon resonance:

The capacity of binding simultaneously murine 4-1BB Fc(kih) and human CD19 was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). Biotinylated murine 4-1BB Fc(kih) was directly coupled to a flow cell of a streptavidin (SA) sensor chip. Immobilization levels up to 600 resonance units (RU) were used. The CD19-targeted mu4-1BBL construct was passed at a concentration range of 200 nM with a flow of 30 $\mu$L/minute through the flow cells over 90 seconds and dissociation was set to zero sec. Monomeric human CD19-Fc(kih) was injected as second analyte with a flow of 30 $\mu$L/minute through the flow cells over 90 seconds at a concentration of 500 nM (**Figure 2A**). The dissociation was monitored for 120 sec. Bulk refractive index differences were corrected for by subtracting the response obtained in a reference flow cell, where no protein was immobilized. As can be seen in the graph of **Figure 2B**, the hybrid surrogate CD19-mu4-1BBL can bind simultaneously murine 4-1BB and human CD19.

**Example 3**

**Combination therapy of CD19-4-1BBL and rituximab or obinutuzumab *in vitro***

*Activation of NK cells*

CD19-4-1BBL is active when it can cross-link on CD19 expressing NHL cells. Antibodies such as rituximab or obinutuzumab (Gazyva) bind to CD20 on NHL cells and can thereby activate NK cells to mediate ADCC. Activated NK cells express 4-1BB. Our hypothesis is that CD19-4-1BBL can synergize with anti-CD20 ADCC mediating antibodies for NK cell activation. To test this, we used 3 different NHL cell lines, e.g. WSU-DLCL2 (CD19+CD20high), SU-DHL-8 (CD19+CD20low), and Naml-6 (CD19+CD20low). To measure the biological activity of activated NK cells, we incubated the respective NHL cells with purified PBMCs from fresh healthy blood at a ration of 1:25 for 24 hours, in the presence of dose-titrated rituximab or obinutuzumab alone, or adding dose-titrated CD19-4-1BBL on top of the $EC_{50}$ concentration of rituximab or obinutuzumab, in IMDM medium (Gibco, Cat No. 31980-048) +10% FBS (Gibco, Cat No. 16140-071) and 1% penicillin-Streptomycin (Gibco, Cat No. 15070-063). The supernatants were collected for the measurement of IFN-$\gamma$ by ELISA (DuoSet Human IFN-$\gamma$ ELISA kit, R&D Systems, Cat No. DY285). **Figure 3** shows that in all three NHL cell lines tested, CD19-4-1BBL could boost the effector function of NK cells for IFN-γ production that were activated by rituximab or obinutuzumab (Gazyva). On CD20^low cells such as SU-DHL-8 and Nalm-6, the combination with obinutuzumab induced higher IFN-γ production than the combination with rituximab.

To confirm that CD19-4-1BBL is only able to activate NK cells after the addition of ADCC mediating antibodies, we measured the activation markers such as CD25 on NK cells (gated on CD3⁻CD56^dim). Consistent with the IFN-γ production, CD19-4-1BBL could only boost the activation of NK cells by upregulating CD25 that were already activated by rituximab or obinutuzumab (**Figure 4**). In contrast, T cells (both CD4 and CD8) showed no activation in terms of CD25 or CD69 in this co-culture (data not shown).

We also studied whether the targeted CD19-4-1BBL is superior to the 4-1BB agonistic antibody urelumab in synergizing the ADCC function of NK cells with obinutuzumab. In a similar experiment as we did above, we observed that only the combination of CD19-4-1BBL with obinutuzumab boosted NK cell activation (CD25 upregulation), but not the combination of urelumab with obinutuzumab (**Figure 5**).

**Example 4**

**Combination therapy of hybrid CD19-4-1BBL and rituximab *in vivo***

The first proof on concept for the combination of hybrid CD19-4-1BBL (monovalent CD19-mu4-1BBL) and rituximab was carried out in tumor-bearing huCD16tgScid mice.

WSU-DLCL2 cells (human diffuse large B cell lymphoma) were originally obtained from ECACC (European Collection of Cell Culture) and after expansion deposited in the internal cell bank of Roche Glycart AG. Cells were cultured in RPMI containing 10% FCS and 1x Glutamax. The cells were cultured at 37 °C in a water-saturated atmosphere at 5 % $CO_2$. $1.5 \times 10^6$ cells per animal were injected s.c. into the right flank of the animals in RPMI cell culture medium (Gibco) and GFR matrigel (1:1, total volume of 100ul) at a viability of > 95.0%.

Human FcγRIIIa (CD16) transgenic SCID mice (which express both murine FcγRIV-positive macrophages and human FcγRIIIa-positive transgenic murine NK cells as effectors) in the age of 8-10 weeks at start of the experiment (bred at Charles River, France) were maintained under specific-pathogen-free condition with daily cycles of 12 h light / 12 h darkness according to committed guidelines (GV-Solas; Felasa; TierschG). The experimental study protocol was reviewed and approved by local government. After arrival, animals were maintained for one week to get accustomed to the new environment and for observation. Continuous health monitoring was carried out on a regular basis.

According to the protocol (Figure 6), female huCD16TgScid mice were injected with tumor cells s.c. as described above and treated once weekly with the compounds or PBS (Vehicle) when tumor size reached appr. 200 mm³ (day 13). All mice were injected i.v. with 200 μl of the appropriate solution once per week. To obtain the proper amount of compounds per 200 μl, the stock solutions (Table 5) were diluted with PBS when necessary. For combination therapy (Group D, Figure 6) with rituximab and hybrid CD19-4-1BBL construct, therapies were injected with a gap of one day. Tumor growth was measured twice weekly using a caliper and tumor volume was calculated as followed:

$$T_v\text{: } (W^2/2) \text{ x } L$$

(W: Width, L: Length)

The study was terminated and all mice were sacrificed after eight injections of the compounds (day 64 after tumor cell injection) and tumors were explanted and weighted. Figures 7A and 7B show the tumor growth kinetics (Mean +/- SEM) in all treatment groups as well as the tumor weights at study termination. Monotherapy of hybrid CD19-4-1BBL did not reveal any tumor growth inhibition. Monotherapy of rituximab induced slower tumor growth compared to vehicle. The combination of rituximab and hybrid CD19-4-1BBL however, induced statistically significant tumor growth inhibition and revealed significant lower tumor weight at study termination as compared to all other treatment groups.

**Table 7: Compositions used in the *in vivo* experiment**

| Compound | Formulation buffer | Concentration (mg/mL) |
|---|---|---|
| hybrid CD19-4-1BBL | 20 mM Histidine, 140 mM NaCl, pH6.0 | 4.11 (= stock solution) |
| Rituximab | 20mM Histidine, 140mM NaCl, pH6.0 | 10 (= stock solution) |

**Example 5**

**Combination therapy of monovalent CD19(2B11)-4-1BBL and obinutuzumab (Gazyva) *in vivo***

The proof on concept for the combination of mono CD19(2B11)-4-1BBL and GAZYVA was carried out in fully humanized NSG mice.

WSU-DLCL2 cells (human diffuse large B cell lymphoma) were originally obtained from ECACC (European Collection of Cell Culture) and after expansion deposited in the Roche Glycart internal cell bank. Cells were cultured in RPMI containing 10% FCS and 1x Glutamax. The cells were cultured at 37 °C in a water-saturated atmosphere at 5 % $CO_2$. $1.5 \times 10^6$ cells (in vitro passage 18) per animal were injected s.c. into the right flank of the animals in RPMI cell culture medium (Gibco) and GFR matrigel (1:1, total volume of 100 $\mu$l) at a viability of > 95.0%.

Female NOD/Shi-scid/IL-2Rγnull (NSG) mice at the age of 4-5 weeks at start of the experiment (bred at Taconic, Denmark) were maintained under specific-pathogen-free condition with daily cycles of 12 h light / 12 h darkness according to committed guidelines (GV-Solas; Felasa; TierschG). The experimental study protocol was reviewed and approved by local government (ZH193/2014). After arrival, animals were maintained for one week to get accustomed to the new environment and for observation. Continuous health monitoring was carried out on a regular basis.

According to the protocol (**Figure 8**), female NSG mice were injected i.p. with 15 mg/kg of Busulfan followed one day later by an i.v. injection of $1 \times 10^5$ human hematopoietic stem cells isolated from cord blood. At week 14-16 after stem cell injection mice were bled sublingual and blood was analyzed by flow cytometry for successful humanization. Efficiently engrafted mice were randomized according to their human T cell frequencies into the different treatment groups (Figure 8, n: 10/group). At that time, mice were injected with tumor cells s.c. as described above and treated once weekly with the compounds or PBS (Vehicle) when tumor size reached appr. 450 mm$^3$ (day 11). All mice were injected i.v. with 200 $\mu$l of the appropriate solution. To obtain the proper amount of compounds per 200 $\mu$l, the stock solutions (Table 6) were diluted with PBS when necessary. For combination therapies (Group D, Figure 8) with GAZYVA, the CD19-4-1BBL construct was injected concomitant. Tumor growth was measured twice weekly using a caliper and tumor volume was calculated as followed:

$$T_v: (W^2/2) \times L$$

(W: Width, L: Length)

**Figure 9** shows the tumor growth kinetics (Mean +/- SEM) in all treatment groups. No monotherapeutic effects of CD19-4-1BBL could be detected. Obinutuzumab, as a single agent, induced little tumor growth inhibition, however, the combination with CD19-4-1BBL induced complete tumor regression in all animals (Figure 9).

**Table 8: Compositions used in this experiment**

| Compound | Formulation buffer | Concentration (mg/mL) |
|---|---|---|
| mono CD19 (2B11)-4-1BBL | 20mM Histidine, 140mM NaCl, pH6.0 | 5.16 (= stock solution) |
| obinutuzumab (Gazyva) | 20mM Histidine, 140mM NaCl, pH6.0 | 25 (= stock solution) |

**Claims**

1. A 4-1BB (CD137) agonist for use in a method for treating a B-cell proliferative disorder, wherein the 4-1BB agonist is used in combination and concomitant with an anti-CD20 antibody selected from rituximab or obinutuzumab, wherein:

   (a) said B-cell proliferative disorder is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL); and
   (b) said 4-1BB agonist is an antigen binding molecule comprising

      (i) one antigen binding domain capable of specific binding to CD19,
      (ii) a first and a second polypeptide that are linked to each other by a disulfide bond, and
      (iii) an IgG1 Fc domain comprising the amino acid substitutions L234A, L235A and P329G (EU numbering);

and

(c) said first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other by a peptide linker and said second polypeptide comprises one ectodomain of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8.

2. The 4-1BB agonist for use in a method of claim 1, wherein the 4-1BB agonist and the anti-CD20 antibody are administered together in a single composition or administered separately in two different compositions.

3. The 4-1BB agonist for use in a method of claims 1 or 2, wherein the ectodomains of 4-1BBL comprise the amino acid sequence of SEQ ID NO:1 or SEQ ID NO:5.

4. The 4-1BB agonist for use in a method of any one of claims 1 to 3, wherein the antigen binding domain capable of specific binding to CD19 comprises

(a) a heavy chain variable region ($V_H$CD19) comprising (i) CDR-H1 comprising the amino acid sequence of SEQ ID NO:9, (ii) CDR-H2 comprising the amino acid sequence of SEQ ID NO:10, and (iii) CDR-H3 comprising the amino acid sequence of SEQ ID NO:11, and a light chain variable region ($V_L$CD19) comprising (iv) CDR-L1 comprising the amino acid sequence of SEQ ID NO:12, (v) CDR-L2 comprising the amino acid sequence of SEQ ID NO:13, and (vi) CDR-L3 comprising the amino acid sequence of SEQ ID NO:14, or
(b) a heavy chain variable region ($V_H$CD19) comprising (i) CDR-H1 comprising the amino acid sequence of SEQ ID NO:15, (ii) CDR-H2 comprising the amino acid sequence of SEQ ID NO:16, and (iii) CDR-H3 comprising the amino acid sequence of SEQ ID NO:17, and a light chain variable region ($V_L$CD19) comprising (iv) CDR-L1 comprising the amino acid sequence of SEQ ID NO:18, (v) CDR-L2 comprising the amino acid sequence of SEQ ID NO:19, and (vi) CDR-L3 comprising the amino acid sequence of SEQ ID NO:20.

5. The 4-1BB agonist for use in a method of any one of claims 1 to 4, wherein the antigen binding domain capable of specific binding to CD19 comprises a heavy chain variable region ($V_H$CD19) comprising an amino acid sequence of SEQ ID NO:21 and a light chain variable region ($V_L$CD19) comprising an amino acid sequence of SEQ ID NO:22 or wherein the antigen binding domain capable of specific binding to CD19 comprises a heavy chain variable region ($V_H$CD19) comprising an amino acid sequence of SEQ ID NO:23 and a light chain variable region ($V_L$CD19) comprising an amino acid sequence of SEQ ID NO:24.

6. The 4-1BB agonist for use in a method of claim 5, wherein

(a) the antigen binding domain capable of specific binding to CD19 is a Fab, and
(b) said first polypeptide as defined in claim 1b(ii) comprises the amino acid sequence selected from the group consisting of SEQ ID NO:25, SEQ ID NO:26, SEQ ID NO:27, SEQ ID NO:28, SEQ ID NO:29, SEQ ID NO:30, SEQ ID NO:31 and SEQ ID NO:32.

7. The 4-1BB agonist for use in a method of any one of claims 1 to 6, wherein the 4-1BB agonist is an antigen binding molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:41 and a second light chain comprising the amino acid sequence of SEQ ID NO:42.

8. The 4-1BB agonist for use in a method of any one of claims 1 to 7, wherein the combination is administered concomitant at intervals from about one week to three weeks.

9. The 4-1BB agonist for use in a method of any one of claims 1 to 8, wherein a pretreatment with an Type II anti-CD20 antibody, preferably obinutuzumab, is performed prior to the combination treatment, wherein the period of time between the pretreatment and the combination treatment is sufficient for the reduction of B-cells in the individual in response to the Type II anti-CD20 antibody, preferably obinutuzumab.

10. A pharmaceutical composition comprising

- a 4-1BB agonist as defined in claim 1, and
- an anti-CD20 antibody selected from rituximab or obinutuzumab,

wherein the 4-1BB agonist and the anti-CD20 antibody are administered together in a single composition.

11. The pharmaceutical composition of claim 10 for use as medicament.

12. The pharmaceutical composition of claims 10 or 11 for use in the treatment of a B-cell proliferative disorder selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).

13. The pharmaceutical composition of claim 10, or the composition for the use of claim 11 or claim 12, wherein the 4-1BB agonist is an antigen binding molecule comprising a first heavy chain comprising the amino acid sequence of SEQ ID NO:47, a first light chain comprising the amino acid sequence of SEQ ID NO:48, a second heavy chain comprising the amino acid sequence of SEQ ID NO:41 and a second light chain comprising the amino acid sequence of SEQ ID NO:42.

**Patentansprüche**

1. 4-1BB-(CD137)-Agonist zur Verwendung in einem Verfahren zum Behandeln einer B-Zellproliferationsstörung, wobei der 4-1BB-Agonist in Kombination und begleitend mit einem Anti-CD20-Antikörper, ausgewählt aus Rituximab oder Obinutuzumab, verwendet wird, wobei:

   (a) die B-Zellproliferationsstörung aus der Gruppe ausgewählt ist, die aus Non-Hodgkin-Lymphom (NHL), akuter lymphatischer Leukämie (ALL), chronischer lymphatischer Leukämie (CLL), diffusem großzelligem B-Zell-Lymphom (DLBCL), Follikellymphom (FL), Mantelzelllymphom (MCL), Marginalzonenlymphom (MZL), multiplem Myelom (MM) und Hodgkin-Lymphom (HL) besteht; und
   (b) der 4-1BB-Agonist ein antigenbindendes Molekül ist, umfassend

      (i) eine antigenbindende Domäne, die spezifisch an CD19 binden kann,
      (ii) ein erstes und ein zweites Polypeptid, die über eine Disulfidbindung aneinander gebunden sind, und
      (iii) eine IgG1-Fc-Domäne, die die Aminosäuresubstitutionen L234A, L235A und P329G (EU-Nummerierung) umfasst; und

   (c) das erste Polypeptid zwei Ektodomänen von 4-1BBL umfasst, die die Aminosäuresequenz umfassen, die aus der Gruppe ausgewählt ist, die aus SEQ ID NO: 1, SEQ ID NO:2, SEQ ID NO:3, SEQ ID NO:4, SEQ ID NO:5, SEQ ID NOA, SEQ ID NO:7 und SEQ ID NO:8 besteht, die über einen Peptid-Linker miteinander verbunden sind, und das zweite Polypeptid eine Ektodomäne von 4-1BBL umfasst, die die Aminosäuresequenz umfasst, die aus der Gruppe ausgewählt ist, die aus SEQ ID NO:1, SEQ ID NO:2, SEQ ID NO:3, SEQ ID NO:4, SEQ ID NO:5, SEQ ID NOA, SEQ ID NO:7 und SEQ ID NO:8 besteht.

2. 4-1BB-Agonist zur Verwendung in einem Verfahren nach Anspruch 1, wobei der 4-1BB-Agonist und der Anti-CD20-Antikörper zusammen in einer einzigen Zusammensetzung verabreicht werden oder separat in zwei verschiedenen Zusammensetzungen verabreicht werden.

3. 4-1BB-Agonist zur Verwendung in einem Verfahren nach den Ansprüchen 1 oder 2, wobei die Ektodomänen von 4-1BBL die Aminosäuresequenz von SEQ ID NO:1 oder SEQ ID NO:5 umfassen.

4. 4-1BB-Agonist zur Verwendung in einem Verfahren nach einem der Ansprüche 1 bis 3, wobei die antigenbindende Domäne, die spezifisch an CD19 binden kann,

   (a) eine variable Region der schweren Kette ($V_H$CD19), umfassend (i) CDR-H1, umfassend die Aminosäuresequenz von SEQ ID NO:9, (ii) CDR-H2, umfassend die Aminosäuresequenz von SEQ ID NO:10, und (iii) CDR-H3, umfassend die Aminosäuresequenz von SEQ ID NO:11, und eine variable Region der leichten Kette ($V_L$CD19), umfassend (iv) CDR-L1, umfassend die Aminosäuresequenz von SEQ ID NO:12, (v) CDR-L2, umfassend die Aminosäuresequenz von SEQ ID NO:13, und (vi) CDR-L3, umfassend die Aminosäuresequenz von SEQ ID NO:14, oder

(b) eine variable Region der schweren Kette (V$_H$CD19), umfassend (i) CDR-H1, umfassend die Aminosäuresequenz von SEQ ID NO:15, (ii) CDR-H2, umfassend die Aminosäuresequenz von SEQ ID NO:16, und (iii) CDR-H3, umfassend die Aminosäuresequenz von SEQ ID NO:17, und eine variable Region der leichten Kette (V$_L$CD19), umfassend (iv) CDR-L1, umfassend die Aminosäuresequenz von SEQ ID NO:18, (v) CDR-L2, umfassend die Aminosäuresequenz von SEQ ID NO:19, und (vi) CDR-L3, umfassend die Aminosäuresequenz von SEQ ID NO:20, umfasst.

5. 4-1BB-Agonist zur Verwendung in einem Verfahren nach einem der Ansprüche 1 bis 4, wobei die antigenbindende Domäne, die spezifisch an CD19 binden kann, eine variable Region der schweren Kette (V$_H$CD19), umfassend eine Aminosäuresequenz von SEQ ID NO:21, und eine variable Region der leichten Kette (V$_L$CD19), umfassend eine Aminosäuresequenz von SEQ ID NO:22, umfasst oder wobei die antigenbindende Domäne, die spezifisch an CD19 binden kann, eine variable Region der schweren Kette (V$_H$CD19), umfassend eine Aminosäuresequenz von SEQ ID NO:23, und eine variable Region der leichten Kette (V$_L$CD19), umfassend eine Aminosäuresequenz von SEQ ID NO:24, umfasst.

6. 4-1BB-Agonist zur Verwendung in einem Verfahren nach Anspruch 5, wobei

(a) die antigenbindende Domäne, die spezifisch an CD19 binden kann, ein Fab ist und
(b) das wie in Anspruch 1b(ii) definierte erste Polypeptid die Aminosäuresequenz umfasst, die aus der Gruppe ausgewählt ist, die aus SEQ ID NO:25, SEQ ID NO:26, SEQ ID NO:27, SEQ ID NO:28, SEQ ID NO:29, SEQ ID NO:30, SEQ ID NO:31 und SEQ ID NO:32 besteht.

7. 4-1BB-Agonist zur Verwendung in einem Verfahren nach einem der Ansprüche 1 bis 6, wobei der 4-1BB-Agonist ein antigenbindendes Molekül ist, das eine erste schwere Kette, umfassend die Aminosäuresequenz von SEQ ID NO:47, eine erste leichte Kette, umfassend die Aminosäuresequenz von SEQ ID NO:48, eine zweite schwere Kette, umfassend die Aminosäuresequenz von SEQ ID NO:41, und eine zweite leichte Kette, umfassend die Aminosäuresequenz von SEQ ID NO:42, umfasst.

8. 4-1BB-Agonist zur Verwendung in einem Verfahren nach einem der Ansprüche 1 bis 7, wobei die Kombination begleitend in Intervallen von etwa einer Woche bis drei Wochen verabreicht wird.

9. 4-1BB-Agonist zur Verwendung in einem Verfahren nach einem der Ansprüche 1 bis 8, wobei eine Vorbehandlung mit einem Typ-II-Anti-CD20-Antikörper, vorzugsweise Obinutuzumab, vor der Kombinationsbehandlung durchgeführt wird, wobei der Zeitraum zwischen der Vorbehandlung und der Kombinationsbehandlung ausreicht, um als Reaktion auf den Typ-II-Anti-CD20-Antikörper, vorzugsweise Obinutuzumab, B-Zellen in dem Individuum zu reduzieren.

10. Pharmazeutische Zusammensetzung, umfassend

- einen 4-1BB-Agonisten, wie in Anspruch 1 definiert, und
- einen Anti-CD20-Antikörper, ausgewählt aus Rituximab oder Obinutuzumab,

wobei der 4-1BB-Agonist und der Anti-CD20-Antikörper zusammen in einer einzigen Zusammensetzung verabreicht werden.

11. Pharmazeutische Zusammensetzung nach Anspruch 10 zur Verwendung als ein Medikament.

12. Pharmazeutische Zusammensetzung nach den Ansprüchen 10 oder 11 zur Verwendung bei der Behandlung einer B-Zellproliferationsstörung, die aus der Gruppe ausgewählt ist, die aus Non-Hodgkin-Lymphom (NHL), akuter lymphatischer Leukämie (ALL), chronischer lymphatischer Leukämie (CLL), diffusem großzelligem B-Zell-Lymphom (DLBCL), Follikellymphom (FL), Mantelzellymphom (MCL), Marginalzonenlymphom (MZL), multiplem Myelom (MM) und Hodgkin-Lymphom (HL) besteht.

13. Pharmazeutische Zusammensetzung nach Anspruch 10 oder Zusammensetzung für die Verwendung nach Anspruch 11 oder Anspruch 12, wobei der 4-1BB-Agonist ein antigenbindendes Molekül ist, das eine erste schwere Kette, umfassend die Aminosäuresequenz von SEQ ID NO:47, eine erste leichte Kette, umfassend die Aminosäuresequenz von SEQ ID NO:48, eine zweite schwere Kette, umfassend die Aminosäuresequenz von SEQ ID NO:41, und eine zweite leichte Kette, umfassend die Aminosäuresequenz von SEQ ID NO:42, umfasst.

**Revendications**

1. Agoniste de 4-1BB (CD137) pour une utilisation dans une méthode de traitement d'un trouble prolifératif des lymphocytes B, dans lequel l'agoniste de 4-1BB est utilisé en combinaison et de manière concomitante avec un anticorps anti-CD20 choisi parmi le rituximab ou l'obinutuzumab,
   dans lequel :

   (a) ledit trouble prolifératif des lymphocytes B est choisi dans le groupe constitué par le lymphome non hodgkinien (LNH), la leucémie lymphocytaire aiguë (LLA), la leucémie lymphocytaire chronique (LLC), le lymphome diffus à grandes cellules B (LDGCB), le lymphome folliculaire (LF), le lymphome à cellules du manteau (LCM), le lymphome de la zone marginale (LZM), le myélome multiple (MM) et le lymphome hodgkinien (LH) ; et
   (b) ledit agoniste de 4-1BB est une molécule de liaison à l'antigène comprenant

      (i) un domaine de liaison à l'antigène capable de se lier spécifiquement à CD19,
      (ii) un premier et un second polypeptide qui sont liés l'un à l'autre par une liaison disulfure, et
      (iii) un domaine Fc d'IgG1 comprenant les substitutions d'acides aminés L234A, L235A et P329G (numérotation EU) ; et

   (c) ledit premier polypeptide comprend deux ectodomaines de 4-1BBL comprenant la séquence d'acides aminés choisie dans le groupe constitué par la SEQ ID NO : 1, la SEQ ID NO : 2, la SEQ ID NO : 3, la SEQ ID NO : 4, la SEQ ID NO : 5, la SEQ ID NO : 6, la SEQ ID NO : 7 et la SEQ ID NO : 8 qui sont reliés l'un à l'autre par un lieur peptidique et ledit second polypeptide comprend un ectodomaine de 4-1BBL comprenant la séquence d'acides aminés choisie dans le groupe constitué par la SEQ ID NO : 1, la SEQ ID NO : 2, la SEQ ID NO : 3, la SEQ ID NO : 4, la SEQ ID NO : 5, la SEQ ID NO : 6, la SEQ ID NO : 7 et la SEQ ID NO : 8.

2. Agoniste de 4-1BB pour une utilisation dans une méthode selon la revendication 1, dans lequel l'agoniste de 4-1BB et l'anticorps anti-CD20 sont administrés conjointement dans une composition unique ou administrés séparément dans deux compositions différentes.

3. Agoniste de 4-1BB pour une utilisation dans une méthode selon les revendications 1 ou 2, dans lequel les ectodomaines de 4-1BBL comprennent la séquence d'acides aminés de SEQ ID NO : 1 ou SEQ ID NO : 5.

4. Agoniste de 4-1BB pour une utilisation dans une méthode selon l'une quelconque des revendications 1 à 3, dans lequel le domaine de liaison à l'antigène capable de se lier spécifiquement à CD 19 comprend

   (a) une région variable de chaîne lourde ($V_H$CD19) comprenant (i) une CDR-H1 comprenant la séquence d'acides aminés de SEQ ID NO : 9, (ii) une CDR-H2 comprenant la séquence d'acides aminés de SEQ ID NO : 10, et (iii) une CDR-H3 comprenant la séquence d'acides aminés de SEQ ID NO : 11, et une région variable de chaîne légère ($V_L$CD19) comprenant (iv) une CDR-L1 comprenant la séquence d'acides aminés de SEQ ID NO : 12, (v) une CDR-L2 comprenant la séquence d'acides aminés de SEQ ID NO : 13, et (vi) une CDR-L3 comprenant la séquence d'acides aminés de SEQ ID NO : 14, ou
   (b) une région variable de chaîne lourde ($V_H$CD19) comprenant (i) une CDR-H1 comprenant la séquence d'acides aminés de SEQ ID NO : 15, (ii) une CDR-H2 comprenant la séquence d'acides aminés de SEQ ID NO : 16, et (iii) une CDR-H3 comprenant la séquence d'acides aminés de SEQ ID NO : 17, et une région variable de chaîne légère ($V_L$CD19) comprenant (iv) une CDR-L1 comprenant la séquence d'acides aminés de SEQ ID NO : 18, (v) une CDR-L2 comprenant la séquence d'acides aminés de SEQ ID NO : 19, et (vi) une CDR-L3 comprenant la séquence d'acides aminés de SEQ ID NO : 20.

5. Agoniste de 4-1BB pour une utilisation dans une méthode selon l'une quelconque des revendications 1 à 4, dans lequel le domaine de liaison à l'antigène capable de se lier spécifiquement à CD19 comprend une région variable de chaîne lourde ($V_H$CD19) comprenant une séquence d'acides aminés de SEQ ID NO : 21 et une région variable de chaîne légère ($V_L$CD19) comprenant une séquence d'acides aminés de SEQ ID NO : 22 ou dans lequel le domaine de liaison à l'antigène capable de se lier spécifiquement à CD19 comprend une région variable de chaîne lourde ($V_H$CD19) comprenant une séquence d'acides aminés de SEQ ID NO : 23 et une région variable de chaîne légère ($V_L$CD19) comprenant une séquence d'acides aminés de SEQ ID NO : 24.

**6.** Agoniste de 4-1BB pour une utilisation dans une méthode selon la revendication 5, dans lequel

(a) le domaine de liaison à l'antigène capable de se lier spécifiquement à CD19 est un Fab, et
(b) ledit premier polypeptide tel que défini dans la revendication 1b(ii) comprend la séquence d'acides aminés choisie dans le groupe constitué par la SEQ ID NO : 25, la SEQ ID NO : 26, la SEQ ID NO : 27, la SEQ ID NO : 28, la SEQ ID NO : 29, la SEQ ID NO : 30, la SEQ ID NO : 31 et la SEQ ID NO : 32.

**7.** Agoniste de 4-1BB pour une utilisation dans une méthode selon l'une quelconque des revendications 1 à 6, dans lequel l'agoniste de 4-1BB est une molécule de liaison à l'antigène comprenant une première chaîne lourde comprenant la séquence d'acides aminés de SEQ ID NO : 47, une première chaîne légère comprenant la séquence d'acides aminés de SEQ ID NO : 48, une seconde chaîne lourde comprenant la séquence d'acides aminés de SEQ ID NO : 41 et une seconde chaîne légère comprenant la séquence d'acides aminés de SEQ ID NO : 42.

**8.** Agoniste de 4-1BB pour une utilisation dans une méthode selon l'une quelconque des revendications 1 à 7, dans lequel la combinaison est administrée de manière concomitante à des intervalles d'environ une semaine à trois semaines.

**9.** Agoniste de 4-1BB pour une utilisation dans une méthode selon l'une quelconque des revendications 1 à 8, dans lequel un prétraitement avec un anticorps anti-CD20 de type II, de préférence l'obinutuzumab, est effectué avant le traitement avec la combinaison, dans lequel la période de temps entre le prétraitement et le traitement avec la combinaison est suffisante pour la réduction des lymphocytes B chez l'individu en réponse à l'anticorps anti-CD20 de type II, de préférence l'obinutuzumab.

**10.** Composition pharmaceutique comprenant

- un agoniste de 4-1BB tel que défini dans la revendication 1, et
- un anticorps anti-CD20 choisi parmi le rituximab ou l'obinutuzumab,

dans laquelle l'agoniste de 4-1BB et l'anticorps anti-CD20 sont administrés conjointement dans une composition unique.

**11.** Composition pharmaceutique selon la revendication 10 pour une utilisation comme médicament.

**12.** Composition pharmaceutique selon les revendications 10 ou 11 pour une utilisation dans le traitement d'un trouble prolifératif des lymphocytes B choisi dans le groupe constitué par le lymphome non hodgkinien (LNH), la leucémie lymphocytaire aiguë (LLA), la leucémie lymphocytaire chronique (LLC), le lymphome diffus à grandes cellules B (LDGCB), le lymphome folliculaire (LF), le lymphome à cellules du manteau (LCM), le lymphome de la zone marginale (LZM), le myélome multiple (MM) et le lymphome hodgkinien (LH).

**13.** Composition pharmaceutique selon la revendication 10, ou composition pour une utilisation selon la revendication 11 ou la revendication 12, dans laquelle l'agoniste de 4-1BB est une molécule de liaison à l'antigène comprenant une première chaîne lourde comprenant la séquence d'acides aminés de SEQ ID NO : 47, une première chaîne légère comprenant la séquence d'acides aminés de SEQ ID NO : 48, une seconde chaîne lourde comprenant la séquence d'acides aminés de SEQ ID NO : 41 et une seconde chaîne légère comprenant la séquence d'acides aminés de SEQ ID NO : 42.

Fig. 1

Fig. 2

(2A)

huCD19-Fc (kih)
(Analyte 2)

Bispecific
construct
(Analyte 1)

mu4-1BB
Fc kih B

(2B)

# Fig. 3

**(3A)**

**(3B)**

WSU-DLCL2

SU-DHL-8

-•- Rituximab
-★- Rituximab + CD19-4-1BBL
-△- Gazyva
-✳- Gazyva + CD19-4-1BBL

-•- Rituximab
-★- Rituximab + CD19-4-1BBL
-△- Gazyva
-✳- Gazyva + CD19-4-1BBL

EP 3 765 489 B1

# Fig. 3

## (3C)

Nalm-6

EP 3 765 489 B1

# Fig. 4

**(4A)**

WSU-DLCL2

**(4B)**

SU-DHL-8

- Rituximab
- Rituximab + CD19-4-1BBL
- Gazyva
- Gazyva + CD19-4-1BBL

- Rituximab
- Rituximab + CD19-4-1BBL
- Gazyva
- Gazyva + CD19-4-1BBL

EP 3 765 489 B1

# Fig. 4

## (4C)

Nalm-6

- ● Rituximab
- ★ Rituximab + CD19-4-1BBL
- △ Gazyva
- ✳ Gazyva + CD19-4-1BBL

# Fig. 5

## (5A)

## (5B)

Fig. 5

**(5C)**

Nalm-6

EP 3 765 489 B1

# Fig. 6

**huCD16Tg Scid mice**

**d0**
**Injection of cancer cells**
*(WSU-DLCL2: 1.5x106; sc.)*

**d13  d14**
**Therapy injection**
(d13: Rituximab;
d14: 4-1BBL molecule)

**d20  d21**

**Day 67**
**Termination and ex**
**vivo analysis**

| Group | No. of animals | Compound | Dose (mg/kg) | Route of administration (therapy) | No. of treatments |
|---|---|---|---|---|---|
| A | 10 | Vehicle | -- | i.v. | 8 (once weekly) |
| B | 10 | Rituximab | 10 | i.v. | 8 (once weekly) |
| C | 10 | hybrid mono CD19-mu4-1BBL | 3 | i.v. | 8 (once weekly) |
| D | 10 | Rituximab + hybrid mono CD19-mu4-1BBL | 10 / 3 | i.v. | 8 (once weekly) |

EP 3 765 489 B1

Fig. 7

(7A)

(7B)

# Fig. 8

| | d0 | d1 | week 14-16 | d0 | d11 | Day 49 |
|---|---|---|---|---|---|---|
| | Busulfan injection | HSC injection | Screening in blood | Injection of cancer cells (WSU-DLCL2: 1.5x106; sc.) | Therapy injection (tumor: ~450mm3) | Termination and ex vivo analysis |

| Group | No. of animals | Compound | Dose (mg/kg) | Route of administration (therapy) | No. of treatments |
|---|---|---|---|---|---|
| A | 10 | Vehicle | - | i.v. | 6 (once per week) |
| B | 10 | mono CD19 (2B11)-4-1BBL | 3 | i.v. | 6 (once per week) |
| C | 10 | GAZYVA | 10 | i.v. | 6 (once per week) |
| D | 10 | GAZYVA + mono CD19 (2B11)-4-1BBL | 10 /3 | i.v. | 6 (once per week) |

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010010051 A
- WO 2016075278 A
- WO 9316185 A
- US 5571894 A
- US 5587458 A
- US 5869046 A
- EP 404097 A
- WO 199301161 A
- US 6248516 B1
- US 7250297 B1
- US 20070224633 A
- US 20040132028 A1
- WO 2005044859 A
- WO 2004035607 A
- WO 2005103081 A
- WO 2004056312 A
- US 5736137 A, Andersen
- WO 2007031875 A
- US 5821333 A
- US 5731168 A
- US 7695936 B
- US 20030157108 A, Presta, L.
- US 20040093621 A
- WO 2003011878 A, Jean-Mairet
- US 6602684 B, Umana
- US 20050123546 A, Umana
- WO 199730087 A, Patel
- WO 199858964 A, Raju, S
- WO 199922764 A, Raju, S
- US 5500362 A
- WO 5821337 A
- WO 2006029879 A
- WO 2005100402 A
- WO 2013120929 A1
- US 6737056 B
- US 7332581 B
- WO 2012130831 A
- US 6194551 B
- WO 9951642 A
- US 20050014934 A, Hinton
- US 7371826 B
- WO 2014177460 A1
- US 5648260 A
- US 5624821 A
- WO 9429351 A
- US 7521541 B
- WO 2012130831 A1
- US 5821337 A
- WO 2009089004 A
- WO 2009080252 A
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- WO 2012020006 A
- US 20040132066 A
- WO 2016075278 A1
- US 20050260186 A
- US 20060104968 A
- US 6267958 B
- US 6171586 B
- WO 2006044908 A

**Non-patent literature cited in the description**

- **MAUDE et al.** *N Engl J Med,* 2014, vol. 371, 1507-1517
- **OAK ; BARTLETT.** *Expert Opin Investig Drugs,* 2015, vol. 24, 715-724
- **BARGOU et al.** *Science,* 2008, vol. 321, 974-977
- **KLINGER et al.** *Blood,* 2012, vol. 119 (26), 6226-6233
- **KWON ; WEISSMAN.** *Proc Natl Acad Sci USA,* 1989, vol. 86, 1963-1967
- **VINAY ; KWON.** *Cell Mol Immunol.,* 2011, vol. 8, 281-284
- **DIEHL et al.** *J Immunol,* 2002, vol. 168, 3755-3762
- **ZHANG et al.** *J Immunol,* 2010, vol. 184, 787-795

- **GOODWIN et al.** *Eur J Immunol.,* 1993, vol. 23, 2631-2641
- **SHAO ; SCHWARZ.** *J Leukoc Biol,* 2011, vol. 89, 21-29
- **BARTKOWIAK ; CURRAN.** *Front Oncol,* 2015, vol. 5, 117
- **SNELL et al.** *Immunol Rev,* 2011, vol. 244, 197-217
- **MELERO et al.** *Nature Med,* 1997, vol. 3, 682-685
- **LI ; RAVETCH.** *Science,* 2011, vol. 333, 1030-1034
- **DUBROT et al.** *Cancer Immunol Immunother,* 2010, vol. 59, 1223-1233
- **SIMEONE ; ASCIERTO.** *J Immunotoxicol,* 2012, vol. 9, 241-247
- **HORNIG et al.** *J Immunother,* 2012, vol. 35, 418-429
- **MÜLLER et al.** *J Immunother.,* 2008, vol. 31, 714-722
- **ZHANG et al.** *Clin Cancer Res,* 2007, vol. 13, 2758-2767
- **LIU et al.** *J. Immunother.,* 2010, vol. 33, 500-509
- **ROBERT.** *NEJM,* 2017, vol. 377 (14), 1331-1344
- **KARMALI.** *Annals Onc.,* 2018, vol. 29 (2), 332-340
- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **HOUSTON, J.S.** *Methods in Enzymol.,* 1991, vol. 203, 46-96
- *Curr Opin Chem Biol,* 2009, vol. 13, 245-255
- **STUMPP et al.** Darpins: A new generation of protein therapeutics. *Drug Discovery Today,* 2008, vol. 13, 695-701
- *Biochim Biophys Acta,* 2000, vol. 1482, 337-350
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- **LILJEBLAD et al.** *Glyco J,* 2000, vol. 17, 323-329
- **HEELEY.** *Endocr Res,* 2002, vol. 28, 217-229
- **CRAGG et al.** *Blood,* 2004, vol. 103, 2738-2743
- **CRAGG et al.** *Blood,* 2003, vol. 101, 1045-1052
- **KLEIN et al.** *mAbs,* 2013, vol. 5, 22-33
- **INN.** *WHO Drug Information,* 2012, vol. 26 (4), 453
- **INN.** *WHO Drug Information,* 2009, vol. 23 (2), 176
- **REFF, M.E.** *Blood,* 1994, vol. 83, 435-445
- **POPPEMA, S ; VISSER, L.** *Biotest Bulletin,* 1987, vol. 3, 131-139
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **KABAT et al.** Sequences of Proteins of Immunological Interest. *U.S. Dept. of Health and Human Services,* 1983
- **CHOTHIA et al.** *J. Mol. Biol.,* 1987, vol. 196, 901-917
- **KABAT et al.** Sequence of Proteins of Immunological Interest. *U.S. Dept. of Health and Human Services,* 1983
- **ALMAGRO ; FRANSSON.** *Front. Biosci.,* 2008, vol. 13, 1619-1633
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **BOWIE, J. U. et al.** *Science,* 1990, vol. 247, 1306-10
- **CUNNINGHAM ; WELLS.** *Science,* 1989, vol. 244, 1081-1085
- **WRIGHT et al.** *TIBTECH,* 1997, vol. 15, 26-32
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DALL'ACQUA, W.F. et al.** *J. Biol. Chem.,* 2006, vol. 281, 23514-23524

- **GREVYS, A et al.** *J. Immunol.,* 2015, vol. 194, 5497-5508
- **DUNCAN ; WINTER.** *Nature,* 1988, vol. 322, 738-40
- **KAM, N.W. et al.** *Proc. Natl. Acad. Sci. USA,* 2005, vol. 102, 11600-11605
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202, 163
- **CRAGG ; GLENNIE.** *Blood,* 2004, vol. 103, 2738-2743
- **SCHAEFER, W. et al.** *PNAS,* 2011, vol. 108, 11187-1191
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Remington's Pharmaceutical Sciences. Mack Printing Company, 1990
- **SUNTHARALINGAM et al.** *N Engl J Med,* 2006, vol. 355, 1018-1028
- **KABAT, E.A et al.** Sequences of Proteins of Immunological Interest. Public Health Service, National Institutes of Health, 1991
- **KABAT, E.A. et al.** Sequences of Proteins of Immunological Interest. Public Health Service, National Institutes of Health, 1991
- **SAMBROOK et al.** Molecular cloning: A laboratory manual. Cold Spring Harbor Laboratory Press, 1989
- **KABAT, E.A. et al.** Sequences of Proteins of Immunological Interest. 1991
- **BONIFACINO, J.S. ; DASSO, M ; HARFORD, J.B.** Current Protocols in Cell Biology. Lippincott-Schwartz, 2000